# EUROPEAN PATENT SPECIFICATION

(11) **EP 1 891 244 B1**
(45) Date of publication and mention of the grant of the patent: **06.10.2010**
(21) Application number: 06849755.1
(22) Date of filing: 12.04.2006
(51) Int. Cl.: C12Q 1/68, C12Q 1/70

(54) **COMPOSITIONS FOR USE IN IDENTIFICATION OF ADENOVIRUSES**
ZUSAMMENSETZUNGEN FÜR DIE IDENTIFIZIERUNG VON ADENOVIREN
COMPOSITIONS A UTILISER DANS L IDENTIFICATION D ADENOVIRUS

(30) Priority: 13.04.2005 US 671003 P
(43) Date of publication of application: 27.02.2008
(73) Proprietor: Ibis Biosciences, Inc., 2251 Faraday Ave. Carlsbad, CA 92008 (US)
(72) Inventor: HALL, Thomas, A., Oceanside, CA 92056 (US); SAMPATH, Rangarajan, San Diego, CA 92129 (US); BLYN, Lawrence, Mission Viejo, CA 92692 (US)
(74) Representative: Hallybone, Huw George
(86) International application number: PCT/US2006/014178
(87) International publication number: WO 2007/086904

(56) References cited:
- WO-A-96/29431
- WO-A-2004/060278
- YAO Z P ET AL: "Mass Spectrometry-Based Proteolytic Mapping for Rapid Virus Identification" ANALYTICAL CHEMISTRY, AMERICAN CHEMICAL SOCIETY. COLUMBUS, US, vol. 74, no. 11, 1 June 2002 (2002-06-01), pages 2529-2534, XP002980424 ISSN: 0003-2700
- BLYN L B ET AL.: "Rapid detection and molecular serotyping of adenovirus by use of PCR followed by electrospray ionization mass spectrometry" JOURNAL OF CLINICAL MICROBIOLOGY, vol. 46, no. 2, February 2008 (2008-02), pages 644-651,

## Description

### FIELD OF THE INVENTION

The present invention provides compositions, kits and methods as defined in the claims for rapid identification and quantification of adenoviruses by molecular mass and base composition analysis.

### BACKGROUND OF THE INVENTION

First isolated in 1953 by investigators attempting to establish cell-lines from adenoidal tissue of children removed during tonsillectomy and from military recruits with febrile illness, adenoviruses are a frequent cause of acute upper respiratory tract infections. Adenoviruses are widespread in nature, infecting birds, many mammals and man. There are 2 genera, Aviadenovirus (avian) and Mastadenovirus (mammalian). There are several subgroups of mammalian adenoviruses including: Subgroup A (serotypes 12, 18 and 31), Subgroup B (serotypes 3, 7, 11, 14, 21, 34 and 35), Subgroup C (serotypes 1, 2, 5 and 6), Subgroup D (serotypes 8-10,13,15, 17, 19,20, 22-30, 32, 33 and 36-39), Subgroup E (serotype 4), and Subgroups F-G (serotypes 40 and 41).

All Adenovirus particles are similar: non-enveloped, 60-90 nm diameter and have icosahedral symmetry, containing 252 capsomers: 240 "hexons" + 12 "pentons" at the vertices of the icosahedron (2-3-5 symmetry). Individual protomers can be isolated by progressive chemical disruption of purified virus particles. The hexons consist of a trimer of polypeptide II with a central pore; VI, VIII and IX are minor polypeptides also associated with the hexon, thought to be involved in stabilization and/or assembly of the particle. The pentons, which have a toxin-like activity, are more complex; the base consists of a pentamer of peptide III, 5 molecules of IIIa are also associated with the penton base.

The adenoviral genome consists of linear, non-segmented double-stranded DNA, 30-38 kbp (with size varying among subgroups) which has the theoretical capacity to encode 30-40 genes. The genomic structure (as determined by cross-hybridization and restriction mapping) is used to assign adenoviruses to subgroups.

Certain types of adenovirus are commonly associated with particular clinical syndromes including: Acute Respiratory Illness, Pharyngitis, Gastroenteritis, Conjunctivitis, Pneumonia, Keratoconjunctivitis, Acute Haemorrhagic Cystitis, and Hepatitis. Most Adenovirus infections involve either the respiratory or gastrointestinal tracts or the eye. Adenovirus infections are very common, most are asymptomatic. Virus can be isolated from the majority of tonsils/adenoids surgically removed, indicating latent infections. It is not known how long the virus can persist in the body, or whether it is capable of reactivation after long periods, causing disease. Adenoviruses are difficult to isolate and populations tend to be heterogeneous among the cells of an infected individual. It is known that virus is reactivated during events of immunosuppression.

The present invention provides, *inter alia,* methods of identifying viruses of the Adenoviridae family. Also provided are oligonucleotide primers, compositions and kits containing the oligonucleotide primers, which define viral bioagent identifying amplicons and, upon amplification, produce corresponding amplification products whose molecular masses provide the means to identify viruses of the Adenoviridae family at the sub-species level.

### SUMMARY OF THE INVENTION

The present invention provides compositions, kits and methods as defined in the claims for rapid identification and quantification of adenoviruses by molecular mass and base composition analysis.

One embodiment is a composition of is an oligonucleotide primer pair including an oligonucleotide primer 14 to 35 nucleobases in length having at least 70% sequence identity with SEQ ID NO: 26 and an oligonucleotide primer 14 to 35 nucleobases in length having at least 70% sequence identity with SEQ ID NO: 121, wherein said primer pair is capable of identifying subgroup and serotype members of the adenoviridae family.

Described herein is also an oligonucleotide primer 14 to 35 nucleobases in length having at least 70% sequence identity with SEQ ID NO: 61.

Described herein is also an oligonucleotide primer 14 to 35 nucleobases in length having at least 70% sequence identity with SEQ ID NO: 122.

Described herein is also a composition of is an oligonucleotide primer pair including an oligonucleotide primer 14 to 35 nucleobases in length having at least 70% sequence identity with SEQ ID NO: 61 and an oligonucleotide primer 14 to 35 nucleobases in length having at least 70% sequence identity with SEQ ID NO: 122.

Described herein is also an oligonucleotide primer 14 to 35 nucleobases in length having at least 70% sequence identity with SEQ ID NO: 38.

Described herein is also an oligonucleotide primer 14 to 35 nucleobases in length having at least 70% sequence identity with SEQ ID NO: 82.

Described herein is also a composition of is an oligonucleotide primer pair including an oligonucleotide primer 14 to 35 nucleobases in length having at least 70% sequence identity with SEQ ID NO: 38 and an oligonucleotide primer 14 to 35 nucleobases in length having at least 70% sequence identity with SEQ ID NO: 82.

Described herein is also an oligonucleotide primer 14 to 35 nucleobases in length having at least 70% sequence identity with SEQ ID NO: 63.

Described herein is also an oligonucleotide primer 14 to 35 nucleobases in length having at least 70% sequence identity with SEQ ID NO: 95.

Described herein is also a composition of is an oligonucleotide primer pair including an oligonucleotide primer 14 to 35 nucleobases in length having at least 70% sequence identity with SEQ ID NO: 63 and an oligonucleotide primer 14 to 35 nucleobases in length having at least 70% sequence identity with SEQ ID NO: 95.

Described herein is also an oligonucleotide primer 14 to 35 nucleobases in length having at least 70% sequence identity with SEQ ID NO: 19.

Described herein is also an oligonucleotide primer 14 to 35 nucleobases in length having at least 70% sequence identity with SEQ ID NO: 93.

Described herein is also a composition of is an oligonucleotide primer pair including an oligonucleotide primer 14 to 35 nucleobases in length having at least 70% sequence identity with SEQ ID NO: 19 and an oligonucleotide primer 14 to 35 nucleobases in length having at least 70% sequence identity with SEQ ID NO: 93.

Described herein is also an oligonucleotide primer 14 to 35 nucleobases in length having at least 70% sequence identity with SEQ ID NO: 54.

Described herein is also an oligonucleotide primer 14 to 35 nucleobases in length having at least 70% sequence identity with SEQ ID NO: 113.

Described herein is also a composition of is an oligonucleotide primer pair including an oligonucleotide primer 14 to 35 nucleobases in length having at least 70% sequence identity with SEQ ID NO: 54 and an oligonucleotide primer 14 to 35 nucleobases in length having at least 70% sequence identity with SEQ ID NO: 113.

Described herein is also an oligonucleotide primer 14 to 35 nucleobases in length having at least 70% sequence identity with SEQ ID NO: 36.

Described herein is also an oligonucleotide primer 14 to 35 nucleobases in length having at least 70% sequence identity with SEQ ID NO: 98.

Described herein is also a composition of is an oligonucleotide primer pair including an oligonucleotide primer 14 to 35 nucleobases in length having at least 70% sequence identity with SEQ ID NO: 36 and an oligonucleotide primer 14 to 35 nucleobases in length having at least 70% sequence identity with SEQ ID NO: 98.

Described herein is also an oligonucleotide primer 14 to 35 nucleobases in length having at least 70% sequence identity with SEQ ID NO: 16.

Described herein is also an oligonucleotide primer 14 to 35 nucleobases in length having at least 70% sequence identity with SEQ ID NO: 106.

Described herein is also a composition of is an oligonucleotide primer pair including an oligonucleotide primer 14 to 35 nucleobases in length having at least 70% sequence identity with SEQ ID NO: 16 and an oligonucleotide primer 14 to 35 nucleobases in length having at least 70% sequence identity with SEQ ID NO: 106.

In some embodiments, either or both of the primers of the primer pair contain at least one modified nucleobase such as 5-propynyluracil or 5-propynylcytosine for example.

In some embodiments, either or both of the primers of the primer pair comprises at least one universal nucleobase such as inosine for example.

In some embodiments, either or both of the primers of the primer pair comprises at least one non-templated T residue on the 5'-end.

In some embodiments, either or both of the primers of the primer pair comprises at least one non-template tag.

In some embodiments, either or both of the primers of the primer pair comprises at least one molecular mass modifying tag.

Some embodiments are kits comprising the composition of claim 1, further comprising one or more primer pair compositions wherein each member of the one or more primer pairs of the kit is of a length of 14 to 35 nucleobases and has 70% to 100% sequence identity with the corresponding member from the group of primer pairs represented by SEQ ID NOs: 61:122, 26:121, 38:82, 63:95, 19:93, 54:113, 36:98 and 16:106. Described herein is also other kits one or more of any of the primer pairs listed in Table 2.

Some embodiments are kits that contain a set of two general survey adenovirus primer pairs represented by primer pair compositions wherein each member of each pair of primers has 70% to 100% sequence identity with the corresponding member from the group of primer pairs represented by SEQ ID NOs: 61:122, 26:121.

Some embodiments of the kits contain at least one calibration polynucleotide for use in quantitiation of adenoviruses in a given sample, and also for use as a positive control for amplification.

Some embodiments of the kits contain at least one anion exchange functional group linked to a magnetic bead.

In some embodiments, the present invention provides primers and compositions comprising pairs of primers, and kits containing the same, and methods for use in identification of adenoviruses. The primers are designed to produce amplification products of DNA encoding genes that have conserved and variable regions across different subgroups and serotypes of adenoviruses.

In some embodiments, the present invention also provides methods for dentification of adenoviruses. Nucleic acid from the virus is amplified using the primers of claim 1 to obtain an amplification product. The molecular mass of the amplification product is measured. Optionally, the base composition of the amplification product is determined from the molecular mass. The molecular mass or base composition is compared with a plurality of molecular masses or base compositions of known analogous adenovirus identifying amplicons, wherein a match between the molecular mass or base composition and a member of the plurality of molecular masses or base compositions identifies the adenovirus. In some embodiments, the molecular mass is measured by mass spectrometry in a modality such as electrospray ionization (ESI) time of flight (TOF) mass spectrometry or ESI Fourier transform ion cyclotron resonance (FTICR) mass spectrometry, for example. Other mass spectrometry techniques can also be used to measure the molecular mass of adenovirus identifying amplicons.

In some embodiments, the present invention is also directed to a method for determining the presence or absence of an adenovirus in a sample. Nucleic acid from the sample is amplified using the composition of claim 1 to obtain an amplification product. The molecular mass of the amplification product is determined. Optionally, the base composition of the amplification product is determined from the molecular mass. The molecular mass or base composition of the amplification product is compared with the known molecular masses or base compositions of one or more known analogous adenovirus identifying amplicons, wherein a match between the molecular mass or base composition of the amplification product and the molecular mass or base composition of one or more known adenovirus identifying amplicons indicates the presence of the adenovirus in the sample. In some embodiments, the molecular mass is measured by mass spectrometry.

In some embodiments, the present invention also provides methods for determination of the quantity of an unknown adenovirus in a sample. The sample is contacted with the composition claim 1 and a known quantity of a calibration polynucleotide comprising a calibration sequence. Nucleic acid from the unknown adenovirus in the sample is concurrently amplified with the composition described above and nucleic acid from the calibration polynucleotide in the sample is concurrently amplified with the composition described above to obtain a first amplification product comprising an adenovirus identifying amplicon and a second amplification product comprising a calibration amplicon. The molecular masses and abundances for the adenovirus identifying amplicon and the calibration amplicon are determined. The adenovirus identifying amplicon is distinguished from the calibration amplicon based on molecular mass and comparison of adenovirus identifying amplicon abundance and calibration amplicon abundance indicates the quantity of adenovirus in the sample. In some embodiments, the base composition of the adenovirus identifying amplicon is determined.

In some embodiments, the present invention provides methods for detecting or quantifying adenoviruses by combining a nucleic acid amplification process with a mass determination process. In some embodiments, such methods identify or otherwise analyze the adenovirus by comparing mass information from an amplification product with a calibration or control product. Such methods can be carried out in a highly multiplexed and/or parallel manner allowing for the analysis of as many as 300 samples per 24 hours on a single mass measurement platform. The accuracy of the mass determination methods in some embodiments of the present invention permits allows for the ability to discriminate between different adenoviruses such as subgroups A, B, C, D, E, and F, as well as serotypes 3, 4, 7 and 21.

### BRIEF DESCRIPTION OF THE DRAWINGS

The foregoing summary of the invention, as well as the following detailed description of the invention, is better understood when read in conjunction with the accompanying drawings which are included by way of example and not by way of limitation.

**Figure 1**: process diagram illustrating a representative primer pair selection process.

**Figure 2**: process diagram illustrating an embodiment of the calibration method.

**Figure 4**: a series of mass spectra of bioagent identifying amplicons obtained by amplification of adenovirus serotypes 21, 12, 8, 7 and 4 with primer pair number 739.

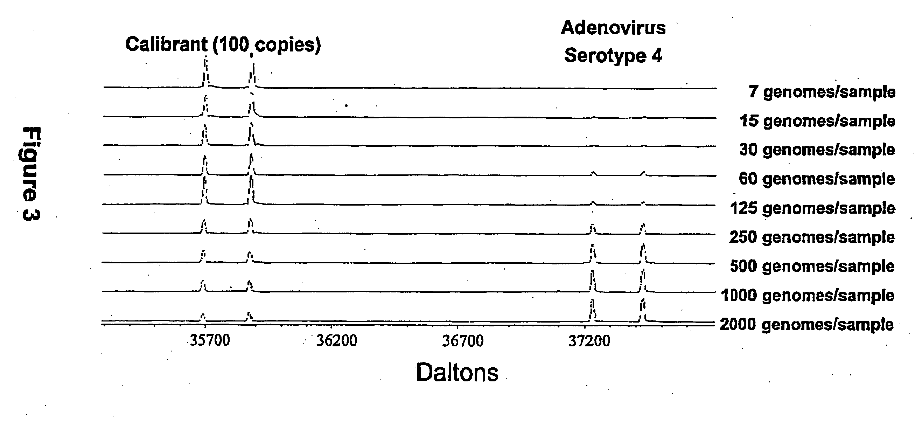

**Figure 3**: a series of mass spectra of amplification products corresponding to calibration amplicons and serotype 4 adenoviral bioagent identifying amplicons produced with primer pair number 769 (SEQ ID NOs: 26:121) with different quantities of genome copies per sample.

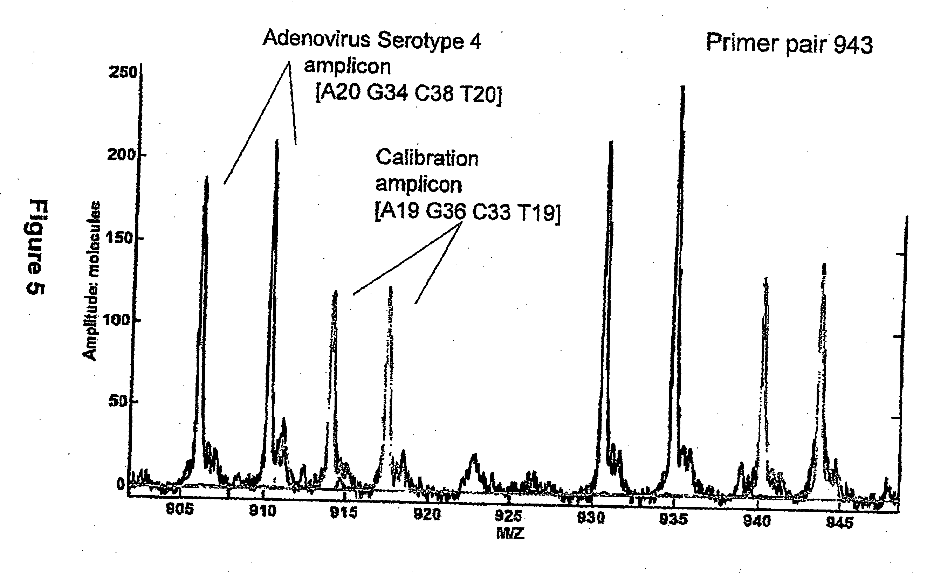

**Figure 5**: A representative mass spectrum of amplification products corresponding to adenovirus identifying amplicons and calibration amplicons obtained with primer pair number 943 (SEQ ID NOs: 61:122).

### DEFINITIONS

As used herein, the term "abundance" refers to an amount. The amount may be described in terms of concentration which are common in molecular biology such as "copy number," "pfu or plate-forming unit" which are well known to those with ordinary skill. Concentration may be relative to a known standard or may be absolute.

. As used herein the term "adenovirus" refers to a virus member of the family *Adenoviridae.* Adenoviruses are classified as group I under the Baltimore classification scheme. Adenoviruses are medium-sized (60-90 nm), non-enveloped icosahedral viruses containing double-stranded DNA. There are 51 immunologically distinct types (6 subgenera: A through F) that can cause human infections. Adenoviruses are unusually stable to chemical or physical agents and adverse pH conditions, allowing for prolonged survival outside of the body and water. Adenoviruses are spread via respiratory droplets.

As used herein, the term "amplifiable nucleic acid" is used in reference to nucleic acids that may be amplified by any amplification method. It is contemplated that "amplifiable nucleic acid" also comprises "sample template."

As used herein the term "amplification" refers to a special case of nucleic acid replication involving template specificity. It is to be contrasted with non-specific template replication (i.e., replication that is template-dependent but not dependent on a specific template). Template specificity is here distinguished from fidelity of replication (i.e., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out. Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Qß replicase, MDV-1 RNA is the specific template for the replicase (D.L. Kacian et al., Proc. Natl. Acad. Sci. USA 69:3038 [1972]). Other nucleic acid will not be replicated by this amplification enzyme. Similarly, in the case of T7 RNA polymerase, this amplification enzyme has a stringent specificity for its own promoters (Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (D.Y. Wu and R. B. Wallace, Genomics 4:560 [1989]). Finally, Taq and Pfu polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences (H.A. Erlich (ed.), PCR Technology, Stockton Press [1989]).

As used herein, the term "amplification reagents" refers to those reagents (deoxyribonucleotide triphosphates, buffer, etc.), needed for amplification, excluding primers, nucleic acid template, and the amplification enzyme. Typically, amplification reagents along with other reaction components are placed and contained in a reaction vessel (test tube, microwell, etc.).

As used herein, the term "analogous" when used in context of comparison of bioagent identifying amplicons indicates that the bioagent identifying amplicons being compared are produced with the same pair of primers. For example, bioagent identifying amplicon "A" and bioagent identifying amplicon "B", produced with the same pair of primers are analogous with respect to each other. Bioagent identifying amplicon "C", produced with a different pair of primers is not analogous to either bioagent identifying amplicon "A" or bioagent identifying amplicon "B".

As used herein, the term "anion exchange functional group" refers to a positively charged functional group capable of binding an anion through an electrostatic interaction. The most well known anion exchange functional groups are the amines, including primary, secondary, tertiary and quaternary amines.

The term "bacteria" or "bacterium" refers to any member of the groups of eubacteria and archaebacteria.

As used herein, a "base composition" is the exact number of each nucleobase (for example, A, T, C and G) in a segment of nucleic acid. For example, amplification of nucleic acid of Adenovirus Type 21 with primer pair number 739 produces an amplification product 139 nucleobases in length from nucleic acid of the hexon gene that has a base composition of A36 G31 C44 T28 (by convention - with reference to the sense strand of the amplification product). Because the molecular masses of each of the four natural nucleotides and chemical modifications thereof are known, a measured molecular mass can be deconvoluted to a list of possible base compositions. Identification of a base composition of a sense strand which is complementary to the corresponding antisense strand in terms of base composition provides a confirmation of the true base composition of an unknown amplification product. For example, the base composition of the antisense strand of the 139 nucleobase amplification product described above is A28 G44 C31 T36.

As used herein, a "base composition probability cloud" is a representation of the diversity in base composition resulting from a variation in sequence that occurs among different isolates of a given species. The "base composition probability cloud" represents the base composition constraints for each species and is typically visualized using a pseudo four-dimensional plot.

In the context of this invention, a "bioagent" is any organism, cell, or virus, living or dead, or a nucleic acid derived from such an organism, cell or virus. Examples of bioagents include, but are not limited, to cells, (including but not limited to human clinical samples, bacterial cells and other pathogens), viruses, fungi, protists, parasites, and pathogenicity markers (including but not limited to: pathogenicity islands, antibiotic resistance genes, virulence factors, toxin genes and other bioregulating compounds). Samples may be alive or dead or in a vegetative state (for example, vegetative bacteria or spores) and may be encapsulated or bioengineered. In the context of this invention, a "pathogen" is a bioagent which causes a disease or disorder.

As used herein, a "bioagent division" is defined as group of bioagents above the species level and includes but is not limited to, orders, families, classes, clades, genera or other such groupings of bioagents above the species level.

As used herein, the term "bioagent identifying amplicon" refers to a polynucleotide that is amplified from a bioagent in an amplification reaction and which 1) provides sufficient variability to distinguish among bioagents from whose nucleic acid the bioagent identifying amplicon is produced and 2) whose molecular mass is amenable to a rapid and convenient molecular mass determination modality such as mass spectrometry, for example.

As used herein, the term "biological product" refers to any product originating from an organism. Biological products are often products of processes of biotechnology. Examples of biological products include, but are not limited to: cultured cell lines, cellular components, antibodies, proteins and other cell-derived biomolecules, growth media, growth harvest fluids, natural products and biopharmaceutical products.

The terms "biowarfare agent" and "bioweapon" are synonymous and refer to a bacterium, virus, fungus or protozoan that could be deployed as a weapon to cause bodily harm to individuals. Military or terrorist groups may be implicated in deployment of biowarfare agents.

In context of this invention, the term "broad range survey primer pair" refers to a primer pair designed to produce bioagent identifying amplicons across different broad groupings of bioagents. For example, the ribosomal RNA-targeted primer pairs are broad range survey primer pairs which have the capability of producing bacterial bioagent identifying amplicons for essentially all known bacteria. With respect to broad range primer pairs employed for identification of viruses, a broad range survey primer pair for adenoviruses, such as primer pair number 615 (SEQ ID NOs: 45:101) for example, will produce an adenovirus identifying amplicon for essentially all known members of the *Adenoviridae* family.

The term "calibration amplicon" refers to a nucleic acid segment representing an amplification product obtained by amplification of a calibration sequence with a pair of primers designed to produce a bioagent identifying amplicon.

The term "calibration sequence" refers to a polynucleotide sequence to which a given pair of primers hybridizes for the purpose of producing an internal (i.e: included in the reaction) calibration standard amplification product for use in determining the quantity of a bioagent in a sample. The calibration sequence may be expressly added to an amplification reaction, or may already be present in the sample prior to analysis.

The term "clade primer pair" refers to a primer pair designed to produce bioagent identifying amplicons for species belonging to a clade group. A clade primer pair may also be considered as a "speciating" primer pair which is useful for distinguishing among closely related species.

The term "codon" refers to a set of three adjoined nucleotides (triplet) that codes for an amino acid or a termination signal.

In context of this invention, the term "codon base composition analysis," refers to determination of the base composition of an individual codon by obtaining a bioagent identifying amplicon that includes the codon. The bioagent identifying amplicon will at least include regions of the target nucleic acid sequence to which the primers hybridize for generation of the bioagent identifying amplicon as well as the codon being analyzed, located between the two primer hybridization regions.

As used herein, the terms "complementary" or "complementarity" are used in reference to polynucleotides (i.e., a sequence of nucleotides such as an oligonucleotide or a target nucleic acid) related by the base-pairing rules. For example, for the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids. Either term may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand.

The term "complement of a nucleic acid sequence" as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs. Where a first oligonucleotide is complementary to a region of a target nucleic acid and a second oligonucleotide has complementary to the same region (or a portion of this region) a "region of overlap" exists along the target nucleic acid. The degree of overlap will vary depending upon the extent of the complementarity

In context of this invention, the term "division-wide primer pair" refers to a primer pair designed to produce bioagent identifying amplicons within sections of a broader spectrum of bioagents For example, primer pair number 1113 (SEQ ID NOs: 63:95), a division-wide primer pair, is designed to produce adenovirus identifying amplicons for members of adenovirus subgroup A. Other division-wide primer pairs may be used to produce adenovirus identifying amplicons for other members of adenovirus subgroups including subgroups B, C, D, E and F.

As used herein, the term "concurrently amplifying" used with respect to more than one amplification reaction refers to the act of simultaneously amplifying more than one nucleic acid in a single reaction mixture.

As used herein, the term "drill-down primer pair" refers to a primer pair designed to produce bioagent identifying amplicons for identification of sub-species characteristics or confirmation of a species assignment. For example, primer pair number 200 (SEQ ID NOs: 1:64), a drill-down adenovirus primer pair, is designed to produce adenovirus identifying amplicons for adenovirus serotype 4. Other drill-down primer pairs may be used to produce adenovirus identifying amplicons for other adenovirus serotypes such as, for example, serotypes 3, 7, 16 and 21.

The term "duplex" refers to the state of nucleic acids in which the base portions of the nucleotides on one strand are bound through hydrogen bonding the their complementary bases arrayed on a second strand. The condition of being in a duplex form reflects on the state of the bases of a nucleic acid. By virtue of base pairing, the strands of nucleic acid also generally assume the tertiary structure of a double helix, having a major and a minor groove. The assumption of the helical form is implicit in the act of becoming duplexed.

As used herein, the term "etiology" refers to the causes or origins, of diseases or abnormal physiological conditions.

The term "gene" refers to a DNA sequence that comprises control and coding sequences necessary for the production of an RNA having a non-coding function (e.g., a ribosomal or transfer RNA), a polypeptide or a precursor. The RNA or polypeptide can be encoded by a full length coding sequence or by any portion of the coding sequence so long as the desired activity or function is retained.

The terms "homology," "homologous" and "sequence identity" refer to a degree of identity. There may be partial homology or complete homology. A partially homologous sequence is one that is less than 100% identical to another sequence. Determination of sequence identity is described in the following example: a primer 20 nucleobases in length which is otherwise identical to another 20 nucleobase primer but having two non-identical residues has 18 of 20 identical residues (18/20 = 0.9 or 90% sequence identity). In another example, a primer 15 nucleobases in length having all residues identical to a 15 nucleobase segment of a primer 20 nucleobases in length would have 15/20 = 0.75 or 75% sequence identity with the 20 nucleobase primer. In context of the present invention, sequence identity is meant to be properly determined when the query sequence and the subject sequence are both described and aligned in the 5' to 3' direction. Sequence alignment algorithms such as BLAST, will return results in two different alignment orientations. In the Plus/Plus orientation, both the query sequence and the subject sequence are aligned in the 5' to 3' direction. On the other hand, in the Plus/Minus orientation, the query sequence is in the 5' to 3' direction while the subject sequence is in the 3' to 5' direction. It should be understood that with respect to the primers of the present invention, sequence identity is properly determined when the alignment is designated as Plus/Plus. Sequence identity may also encompass alternate or modified nucleobases that perform in a functionally similar manner to the regular nucleobases adenine, thymine, guanine and cytosine with respect to hybridization and primer extension in amplification reactions. In a non-limiting example, if the 5-propynyl pyrimidines propyne C and/or propyne T replace one or more C or T residues in one primer which is otherwise identical to another primer in sequence and length, the two primers will have 100% sequence identity with each other. In another non-limiting example, Inosine (I) may be used as a replacement for G or T and effectively hybridize to C, A or U (uracil). Thus, if inosine replaces one or more C, A or U residues in one primer which is otherwise identical to another primer in sequence and length, the two primers will have 100% sequence identity with each other. Other such modified or universal bases may exist which would perform in a functionally similar manner for hybridization and amplification reactions and will be understood to fall within this definition of sequence identity.

As used herein, "housekeeping gene" refers to a gene encoding a protein or RNA involved in basic functions required for survival and reproduction of a bioagent. Housekeeping genes include, but are not limited to genes encoding RNA or proteins involved in translation, replication, recombination and repair, transcription, nucleotide metabolism, amino acid metabolism, lipid metabolism, energy generation, uptake, secretion and the like.

As used herein, the term "hybridization" is used in reference to the pairing of complementary nucleic acids. Hybridization and the strength of hybridization (i.e., the strength of the association between the nucleic acids) is influenced by such factors as the degree of complementary between the nucleic acids, stringency of the conditions involved, and the Tₘ of the formed hybrid. "Hybridization" methods involve the annealing of one nucleic acid to another, complementary nucleic acid, i.e., a nucleic acid having a complementary nucleotide sequence. The ability of two polymers of nucleic acid containing complementary sequences to find each other and anneal through base pairing interaction is a well-recognized phenomenon. The initial observations of the "hybridization" process by Marmur and Lane, Proc. Natl. Acad. Sci. USA 46:453 (1960) and Doty et al., Proc. Natl. Acad. Sci. USA 46:461 (1960) have been followed by the refinement of this process into an essential tool of modem biology.

The term *"in silico"* refers to processes taking place via computer calculations. For example, electronic PCR (ePCR) is a process analogous to ordinary PCR except that it is carried out using nucleic acid sequences and primer pair sequences stored on a computer formatted medium.

As used herein, "intelligent primers" are primers that are designed to bind to highly conserved sequence regions of a bioagent identifying amplicon that flank an intervening variable region and, upon amplification, yield amplification products which ideally provide enough variability to distinguish individual bioagents, and which are amenable to molecular mass analysis. By the term "highly conserved," it is meant that the sequence regions exhibit between about 80-100%, or between about 90-100%, or between about 95-100% identity among all, or at least 70%, at least 80%, at least 90%, at least 95%, or at least 99% of species or strains.

The "ligase chain reaction" (LCR; sometimes referred to as "Ligase Amplification Reaction" (LAR) described by Barany, Proc. Natl. Acad. Sci., 88:189 (1991); Barany, PCR Methods and Applic., 1:5 (1991); and Wu and Wallace, Genomics 4:560 (1989) has developed into a well-recognized alternative method for amplifying nucleic acids. In LCR, four oligonucleotides, two adjacent oligonucleotides which uniquely hybridize to one strand of target DNA, and a complementary set of adjacent oligonucleotides, that hybridize to the opposite strand are mixed and DNA ligase is added to the mixture. Provided that there is complete complementarity at the junction, ligase will covalently link each set of hybridized molecules. Importantly, in LCR, two probes are ligated together only when they base-pair with sequences in the target sample, without gaps or mismatches. Repeated cycles of denaturation, hybridization and ligation amplify a short segment of DNA. LCR has also been used in combination with PCR to achieve enhanced detection of single-base changes. However, because the four oligonucleotides used in this assay can pair to form two short ligatable fragments, there is the potential for the generation of target-independent background signal. The use of LCR for mutant screening is limited to the examination of specific nucleic acid positions.

The term "locked nucleic acid" or "LNA" refers to a nucleic acid analogue containing one or more 2'-O, 4'-C-methylene-β-D-ribofuranosyl nucleotide monomers in an RNA mimicking sugar conformation. LNA oligonucleotides display unprecedented hybridization affinity toward complementary single-stranded RNA and complementary single- or double-stranded DNA. LNA oligonucleotides induce A-type (RNA-like) duplex conformations.

As used herein, the term "mass-modifying tag" refers to any modification to a given nucleotide which results in an increase in mass relative to the analogous non-mass modified nucleotide. Mass-modifying tags can include heavy isotopes of one or more elements included in the nucleotide such as carbon-13 for example. Other possible modifications include addition of substituents such as iodine or bromine at the 5 position of the nucleobase for example.

The term "mass spectrometry" refers to measurement of the mass of atoms or molecules. The molecules are first converted to ions, which are separated using electric or magnetic fields according to the ratio of their mass to electric charge. The measured masses are used to identity the molecules.

The term "microorganism" as used herein means an organism too small to be observed with the unaided eye and includes, but is not limited to bacteria, virus, protozoans, fungi; and ciliates.

The term "multi-drug resistant" or multiple-drug resistant" refers to a microorganism which is resistant to more than one of the antibiotics or antimicrobial agents used in the treatment of said microorganism.

The term "multiplex PCR" refers to a PCR reaction where more than one primer set is included in the reaction pool allowing 2 or more different DNA targets to be amplified by PCR in a single reaction tube.

The term "non-template tag" refers to a stretch of at least three guanine or cytosine nucleobases of a primer used to produce a bioagent identifying amplicon which are not complementary to the template. A non-template tag is incorporated into a primer for the purpose of increasing the primer-duplex stability of later cycles of amplification by incorporation of extra G-C pairs which each have one additional hydrogen bond relative to an A-T pair.

The term "nucleic acid sequence" as used herein refers to the linear composition of the nucleic acid residues A, T, C or G or any modifications thereof, within an oligonucleotide, nucleotide or polynucleotide, and fragments or portions thereof, and to DNA or RNA of genomic or synthetic origin which may be single or double stranded, and represent the sense or antisense strand

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

The term "nucleotide analog" as used herein refers to modified or non-naturally occurring nucleotides such as 5-propynyl pyrimidines (i.e., 5-propynyl-dTTP and 5-propynyl-dTCP), 7-deaza purines (i.e., 7-deaza-dATP and 7-deaza-dGTP). Nucleotide analogs include base analogs and comprise modified forms of deoxyribonucleotides as well as ribonucleotides.

The term "oligonucleotide" as used herein is defined as a molecule comprising two or more deoxyribonucleotides or ribonucleotides, preferably at least 5 nucleotides, more preferably at least about 13 to 35 nucleotides. The exact size will depend on many factors, which in turn depend on the ultimate function or use of the oligonucleotide. The oligonucleotide may be generated in any manner, including chemical synthesis, DNA replication, reverse transcription, PCR, or a combination thereof. Because mononucleotides are reacted to make oligonucleotides in a manner such that the 5' phosphate of one mononucleotide pentose ring is attached to the 3' oxygen of its neighbor in one direction via a phosphodiester linkage, an end of an oligonucleotide is referred to as the "5'-end" if its 5' phosphate is not linked to the 3' oxygen of a mononucleotide pentose ring and as the "3'-end" if its 3' oxygen is not linked to a 5' phosphate of a subsequent mononucleotide pentose ring. As used herein, a nucleic acid sequence, even if internal to a larger oligonucleotide, also may be said to have 5' and 3' ends. A first region along a nucleic acid strand is said to be upstream of another region if the 3' end of the first region is before the 5' end of the second region when moving along a strand of nucleic acid in a 5' to 3' direction. All oligonucleotide primers disclosed herein are understood to be presented in the 5' to 3' direction when reading left to right. When two different, non-overlapping oligonucleotides anneal to different regions of the same linear complementary nucleic acid sequence, and the 3' end of one oligonucleotide points towards the 5' end of the other, the former may be called the "upstream" oligonucleotide and the latter the "downstream" oligonucleotide. Similarly, when two overlapping oligonucleotides are hybridized to the same linear complementary nucleic acid sequence, with the first oligonucleotide positioned such that its 5' end is upstream of the 5' end of the second oligonucleotide, and the 3' end of the first oligonucleotide is upstream of the 3' end of the second oligonucleotide, the first oligonucleotide may be called the "upstream" oligonucleotide and the second oligonucleotide may be called the "downstream" oligonucleotide.

In the context of this invention, a "pathogen" is a bioagent which causes a disease or disorder.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

The term "peptide nucleic acid" ("PNA") as used herein refers to a molecule comprising bases or base analogs such as would be found in natural nucleic acid, but attached to a peptide backbone rather than the sugar-phosphate backbone typical of nucleic acids. The attachment of the bases to the peptide is such as to allow the bases to base pair with complementary bases of nucleic acid in a manner similar to that of an oligonucleotide. These small molecules, also designated anti gene agents, stop transcript elongation by binding to their complementary strand of nucleic acid (Nielsen, et al. Anticancer Drug Des. 8:53 63).

The term "polymerase" refers to an enzyme having the ability to synthesize a complementary strand of nucleic acid from a starting template nucleic acid strand and free dNTPs.

As used herein, the term "polymerase chain reaction" ("PCR") refers to the method of K.B. Mullis U.S. Patent Nos. 4,683,195, 4,683,202, and 4,965,188, hereby incorporated by reference, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified." With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of 32P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

The term "polymerization means" or "polymerization agent" refers to any agent capable of facilitating the addition of nucleoside triphosphates to an oligonucleotide. Preferred polymerization means comprise DNA and RNA polymerases.

As used herein, the terms "pair of primers," or "primer pair" are synonymous. A primer pair is used for amplification of a nucleic acid sequence. A pair of primers comprises a forward primer and a reverse primer. The forward primer hybridizes to a sense strand of a target gene sequence to be amplified and primes synthesis of an antisense strand (complementary to the sense strand) using the target sequence as a template. A reverse primer hybridizes to the antisense strand of a target gene sequence to be amplified and primes synthesis of a sense strand (complementary to the antisense strand) using the target sequence as a template.

The primers are designed to bind to highly conserved sequence regions of a bioagent identifying amplicon that flank an intervening variable region and yield amplification products which ideally provide enough variability to distinguish each individual bioagent, and which are amenable to molecular mass analysis. In some embodiments, the highly conserved sequence regions exhibit between about 80-100%, or between about 90-100%, or between about 95-100% identity, or between about 99-100% identity. The molecular mass of a given amplification product provides a means of identifying the bioagent from which it was obtained, due to the variability of the variable region. Thus design of the primers requires selection of a variable region with appropriate variability to resolve the identity of a given bioagent. Bioagent identifying amplicons are ideally specific to the identity of the bioagent.

Properties of the primers may include any number of properties related to structure including, but not limited to: nucleobase length which may be contiguous (linked together) or non-contiguous (for example, two or more contiguous segments which are joined by a linker or loop moiety), modified or universal nucleobases (used for specific purposes such as for example, increasing hybridization affinity, preventing non-templated adenylation and modifying molecular mass) percent complementarity to a given target sequences.

Properties of the primers also include functional features including, but not limited to, orientation of hybridization (forward or reverse) relative to a nucleic acid template. The coding or sense strand is the strand to which the forward priming primer hybridizes (forward priming orientation) while the reverse priming primer hybridizes to the non-coding or antisense strand (reverse priming orientation). The functional properties of a given primer pair also include the generic template nucleic acid to which the primer pair hybridizes. For example, identification of bioagents can be accomplished at different levels using primers suited to resolution of each individual level of identification. Broad range survey primers are designed with the objective of identifying a bioagent as a member of a particular division (e.g., an order, family, genus or other such grouping of bioagents above the species level of bioagents). In some embodiments, broad range survey intelligent primers are capable of identification of bioagents at the species or sub-species level. Other primers may have the functionality of producing bioagent identifying amplicons for members of a given taxonomic genus, clade, species, sub-species or genotype (including genetic variants which may include presence of virulence genes or antibiotic resistance genes or mutations). Additional functional properties of primer pairs include the functionality of performing amplification either singly (single primer pair per amplification reaction vessel) or in a multiplex fashion (multiple primer pairs and multiple amplification reactions within a single reaction vessel).

As used herein, the terms "purified" or "substantially purified" refer to molecules, either nucleic or amino acid sequences, that are removed from their natural environment, isolated or separated, and are at least 60% free, preferably 75% free, and most preferably 90% free from other components with which they are naturally associated. An "isolated polynucleotide" or "isolated oligonucleotide" is therefore a substantially purified polynucleotide.

The term "reverse transcriptase" refers to an enzyme having the ability to transcribe DNA from an RNA template. This enzymatic activity is known as reverse transcriptase activity. Reverse transcriptase activity is desirable in order to obtain DNA from RNA viruses which can then be amplified and analyzed by the methods of the present invention.

The term "ribosomal RNA" or "rRNA" refers to the primary ribonucleic acid constituent of ribosomes. Ribosomes are the protein-manufacturing organelles of cells and exist in the cytoplasm. Ribosomal RNAs are transcribed from the DNA genes encoding them.

The term "sample" in the present specification and claims is used in its broadest sense. On the one hand it is meant to include a specimen or culture (e.g., microbiological cultures). On the other hand, it is meant to include both biological and environmental samples. A sample may include a specimen of synthetic origin. Biological samples may be animal, including human, fluid, solid (e.g., stool) or tissue, as well as liquid and solid food and feed products and ingredients such as dairy items, vegetables, meat and meat by-products, and waste. Biological samples may be obtained from all of the various families of domestic animals, as well as feral or wild animals, including, but not limited to, such animals as ungulates, bear, fish, lagamorphs, rodents, etc. Environmental samples include environmental material such as surface matter, soil, water, air and industrial samples, as well as samples obtained from food and dairy processing instruments, apparatus, equipment, utensils, disposable and non-disposable items. These examples are not to be construed as limiting the sample types applicable to the present invention. The term "source of target nucleic acid" refers to any sample that contains nucleic acids (RNA or DNA). Particularly preferred sources of target nucleic acids are biological samples including, but not limited to blood, saliva, cerebral spinal fluid, pleural fluid, milk, lymph, sputum and semen.

As used herein, the term "sample template" refers to nucleic acid originating from a sample that is analyzed for the presence of "target" (defined below). In contrast, "background template" is used in reference to nucleic acid other than sample template that may or may not be present in a sample. Background template is often a contaminant. It may be the result of carryover, or it may be due to the presence of nucleic acid contaminants sought to be purified away from the sample. For example, nucleic acids from organisms other than those to be detected may be present as background in a test sample.

A "segment" is defined herein as a region of nucleic acid within a target sequence.

The "self-sustained sequence replication reaction" (3SR) (Guatelli et al., Proc. Natl. Acad. Sci., 87:1874-1878 [1990], with an erratum at Proc. Natl. Acad. Sci., 87:7797 [1990]) is a transcription-based *in vitro* amplification system (Kwok et al., Proc. Natl. Acad. Sci., 86:1173-1177 [1989]) that can exponentially amplify RNA sequences at a uniform temperature. The amplified RNA can then be utilized for mutation detection (Fahy et al., PCR Meth. Appl., 1:25-33 [1991]). In this method, an oligonucleotide primer is used to add a phage RNA polymerase promoter to the 5' end of the sequence of interest. In a cocktail of enzymes and substrates that includes a second primer, reverse transcriptase, RNase H, RNA polymerase and ribo- and deoxyribonucleoside triphosphates, the target sequence undergoes repeated rounds of transcription, cDNA synthesis and second-strand synthesis to amplify the area of interest. The use of 3SR to detect mutations is kinetically limited to screening small segments of DNA (e.g., 200-300 base pairs).

As used herein, the term ""sequence alignment"" refers to a listing of multiple DNA or amino acid sequences and aligns them to highlight their similarities. The listings can be made using bioinformatics computer programs.

In context of this invention, the term "speciating primer pair" refers to a primer pair designed to produce a bioagent identifying amplicon with the diagnostic capability of identifying species members of a group of genera or a particular genus of bioagents. Primer pair number 769 (SEQ ID NOs: 26:121), for example, is a speciating primer pair used to identify subgroup and serotype members of the *Adenoviridae* family.

As used herein, a "sub-species characteristic" is a genetic characteristic that provides the means to distinguish two members of the same bioagent species. For example, one viral strain could be distinguished from another viral strain of the same species by possessing a genetic change (e.g., for example, a nucleotide deletion, addition or substitution) in one of the viral genes, such as the RNA-dependent RNA polymerase. Sub-species characteristics are responsible for the phenotypic differences among the different serotypes of adenoviruses.

As used herein, the term "target," refers to a nucleic acid sequence or structure to be detected or characterized. Thus, the "target" is sought to be sorted out from other nucleic acid sequences and contains a sequence that has at least partial complementarity with an oligonucleotide primer. The target nucleic acid may comprise single- or double-stranded DNA or RNA. A "segment" is defined as a region of nucleic acid within the target sequence.

The term "template" refers to a strand of nucleic acid on which a complementary copy is built from nucleoside triphosphates through the activity of a template-dependent nucleic acid polymerase. Within a duplex the template strand is, by convention, depicted and described as the "bottom" strand. Similarly, the non-template strand is often depicted and described as the "top" strand.

As used herein, the term "Tₘ" is used in reference to the "melting temperature." The melting temperature is the temperature at which a population of double-stranded nucleic acid molecules becomes half dissociated into single strands. Several equations for calculating the Tₘ of nucleic acids are well known in the art. As indicated by standard references, a simple estimate of the Tₘ value may be calculated by the equation: Tₘ=81.5+0.41 (% G+C), when a nucleic acid is in aqueous solution at 1 M NaCl (see e.g., Anderson and Young, Quantitative Filter Hybridization, in Nucleic Acid Hybridization (1985). Other references (e.g., Allawi, H. T. & SantaLucia, J., Jr. Thermodynamics and NMR of internal G.T mismatches in DNA. Biochemistry 36, 10581-94 (1997) include more sophisticated computations which take structural and environmental, as well as sequence characteristics into account for the calculation of Tₘ.

The term "triangulation genotyping analysis" refers to a method of genotyping a bioagent by measurement of molecular masses or base compositions of amplification products, corresponding to bioagent identifying amplicons, obtained by amplification of regions of more than one gene. In this sense, the term "triangulation" refers to a method of establishing the accuracy of information by comparing three or more types of independent points of view bearing on the same findings. Triangulation genotyping analysis carried out with a plurality of triangulation genotyping analysis primers yields a plurality of base compositions that then provide a pattern or "barcode" from which a species type can be assigned. The species type may represent a previously known sub-species or strain, or may be a previously unknown strain having a specific and previously unobserved base composition barcode indicating the existence of a previously unknown genotype.

As used herein, the term "triangulation genotyping analysis primer pair" is a primer pair designed to produce bioagent identifying amplicons for determining species types in a triangulation genotyping analysis.

The employment of more than one bioagent identifying amplicon for identification of a bioagent is herein referred to as "triangulation identification." Triangulation identification is pursued by analyzing a plurality of bioagent identifying amplicons produced with different primer pairs. This process is used to reduce false negative and false positive signals, and enable reconstruction of the origin of hybrid or otherwise engineered bioagents. For example, identification of the three part toxin genes typical of *B. anthracis* (Bowen et al., J. Appl. Microbiol., 1999, 87, 270-278) in the absence of the expected signatures from the *B. anthracis* genome would suggest a genetic engineering event.

In the context of this invention, the term "unknown bioagent" may mean either: (i) a bioagent whose existence is known (such as the well known bacterial species *Staphylococcus aureus* for example but which is not known to be in a sample to be analyzed, or (ii) a bioagent whose existence is not known (for example, the SARS coronavirus was unknown prior to April 2003). For example, if the method for identification of coronaviruses disclosed in commonly owned U.S. Patent Serial No. 10/829,826 was to be employed prior to April 2003 to identify the SARS coronavirus in a clinical sample, both meanings of "unknown" bioagent are applicable since the SARS coronavirus was unknown to science prior to April, 2003 and since it was not known what bioagent (in this case a coronavirus) was present in the sample. On the other hand, if the method of U.S. Patent Serial No. 10/829,826 was to be employed subsequent to April 2003 to identify the SARS coronavirus in a clinical sample, only the first meaning (i) of "unknown" bioagent would apply since the SARS coronavirus became known to science subsequent to April 2003 and since it was not known what bioagent was present in the sample.

The term "variable sequence" as used herein refers to differences in nucleic acid sequence between two nucleic acids. For example, the genes of two different bacterial species may vary in sequence by the presence of single base substitutions and/or deletions or insertions of one or more nucleotides. These two forms of the structural gene are said to vary in sequence from one another. In the context of the present invention, "viral nucleic acid" includes, but is not limited to, DNA, RNA, or DNA that has been obtained from viral RNA, such as, for example, by performing a reverse transcription reaction. Viral RNA can either be single-stranded (of positive or negative polarity) or double-stranded.

The term "virus" refers to obligate, ultramicroscopic, parasites that are incapable of autonomous replication (i.e., replication requires the use of the host cell's machinery). Viruses can survive outside of a host cell but cannon replicate.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. In contrast, the term "modified", "mutant" or "polymorphic" refers to a gene or gene product that displays modifications in sequence and or functional properties (i.e., altered characteristics) when compared to the wild-type gene or gene product. It is noted that naturally-occurring mutants can be isolated; these are identified by the fact that they have altered characteristics when compared to the wild-type gene or gene product.

As used herein, a "wobble base" is a variation in a codon found at the third nucleotide position of a DNA triplet. Variations in conserved regions of sequence are often found at the third nucleotide position due to redundancy in the amino acid code.

### DETAILED DESCRIPTION OF EMBODIMENTS

### A. Bioagent Identifying Amplicons

The present invention provides methods for detection and identification of unknown bioagents using bioagent identifying amplicons. Primers are selected to hybridize to conserved sequence regions of nucleic acids derived from a bioagent, and which bracket variable sequence regions to yield a bioagent identifying amplicon, which can be amplified and which is amenable to molecular mass determination. The molecular mass then provides a means to uniquely identify the bioagent without a requirement for prior knowledge of the possible identity of the bioagent. The molecular mass or corresponding base composition signature of the amplification product is then matched against a database of molecular masses or base composition signatures. A match is obtained when an experimentally-determined molecular mass or base composition of an analyzed amplification product is compared with known molecular masses or base compositions of known bioagent identifying amplicons and the experimentally determined molecular mass or base composition is the same as the molecular mass or base composition of one of the known bioagent identifying amplicons. Alternatively, the experimentally-determined molecular mass or base composition may be within experimental error of the molecular mass or base composition of a known bioagent identifying amplicon and still be classified as a match. In some cases, the match may also be classified using a probability of match model such as the models described in U.S. Serial No. 11/073,362. Furthermore, the method can be applied to rapid parallel multiplex analyses, the results of which can be employed in a triangulation identification strategy. The present method provides rapid throughput and does not require nucleic acid sequencing of the amplified target sequence for bioagent detection and identification.

Despite enormous biological diversity, all forms of life on earth share sets of essential, common features in their genomes. Since genetic data provide the underlying basis for identification of bioagents by the methods of the present invention, it is necessary to select segments of nucleic acids which ideally provide enough variability to distinguish each individual bioagent and whose molecular mass is amenable to molecular mass determination.

Unlike bacterial genomes, which exhibit conservation of numerous genes (i.e. housekeeping genes) across all organisms, viruses do not share a gene that is essential and conserved among all virus families. Therefore, viral identification is achieved within smaller groups of related viruses, such as members of a particular virus family or genus. For example, RNA-dependent RNA polymerase is present in all single-stranded RNA viruses and can be used for broad priming as well as resolution within the virus family.

In some embodiments of the present invention, at least one viral nucleic acid segment is amplified in the process of identifying the bioagent. Thus, the nucleic acid segments that can be amplified by the primers disclosed herein and that provide enough variability to distinguish each individual bioagent and whose molecular masses are amenable to molecular mass determination are herein described as bioagent identifying amplicons.

In some embodiments of the present invention, bioagent identifying amplicons comprise from about 45 to about 150 nucleobases (i.e. from about 45 to about 200 linked nucleosides), although both longer and short regions may be used. One of ordinary skill in the art will appreciate that the invention embodies compounds of 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150 nucleobases in length, or any range therewithin.

It is the combination of the portions of the bioagent nucleic acid segment to which the primers hybridize (hybridization sites) and the variable region between the primer hybridization sites that comprises the bioagent identifying amplicon.

In some embodiments, bioagent identifying amplicons amenable to molecular mass determination which are produced by the primers described herein are either of a length, size or mass compatible with the particular mode of molecular mass determination or compatible with a means of providing a predictable fragmentation pattern in order to obtain predictable fragments of a length compatible with the particular mode of molecular mass determination. Such means of providing a predictable fragmentation pattern of an amplification product include, but are not limited to, cleavage with chemical reagents, restriction enzymes or cleavage primers, for example. Thus, in some embodiments, bioagent identifying amplicons are larger than 150 nucleobases and are amenable to molecular mass determination following restriction digestion. Methods of using restriction enzymes and cleavage primers are well known to those with ordinary skill in the art.

In some embodiments, amplification products corresponding to bioagent identifying amplicons are obtained using the polymerase chain reaction (PCR) that is a routine method to those with ordinary skill in the molecular biology arts. Other amplification methods may be used such as ligase chain reaction (LCR), low-stringency single primer PCR, and multiple strand displacement amplification (MDA). These methods are also known to those with ordinary skill.

### B. Primers and Primer Pairs

Described herein are primers designed to bind to conserved sequence regions of a bioagent identifying amplicon that flank an intervening variable region and yield amplification products which provide variability sufficient to distinguish each individual bioagent, and which are amenable to molecular mass analysis. In some embodiments, the highly conserved sequence regions exhibit between about 80-100%, or between about 90-100%, or between about 95-100% identity, or between about 99-100% identity. The molecular mass of a given amplification product provides a means of identifying the bioagent from which it was obtained, due to the variability of the variable region. Thus, design of the primers involves selection of a variable region with sufficient variability to resolve the identity of a given bioagent. In some embodiments, bioagent identifying amplicons are specific to the identity of the bioagent.

In some embodiments, identification of bioagents is accomplished at different levels using primers suited to resolution of each individual level of identification. Broad range survey primers are designed with the objective of identifying a bioagent as a member of a particular division (e.g., an order, family, genus or other such grouping of bioagents above the species level of bioagents). In some embodiments, broad range survey intelligent primers are capable of identification of bioagents at the species or sub-species level.

In some embodiments, drill-down primers are designed with the objective of identifying a bioagent at the sub-species level (including strains, subtypes, variants and isolates) based on sub-species characteristics which may, for example, include single nucleotide polymorphisms (SNPs), variable number tandem repeats (VNTRs), deletions, drug resistance mutations or any other modification of a nucleic acid sequence of a bioagent relative to other members of a species having different sub-species characteristics. Drill-down intelligent primers are not always required for identification at the sub-species level because broad range survey intelligent primers may, in some cases provide sufficient identification resolution to accomplishing this identification objective.

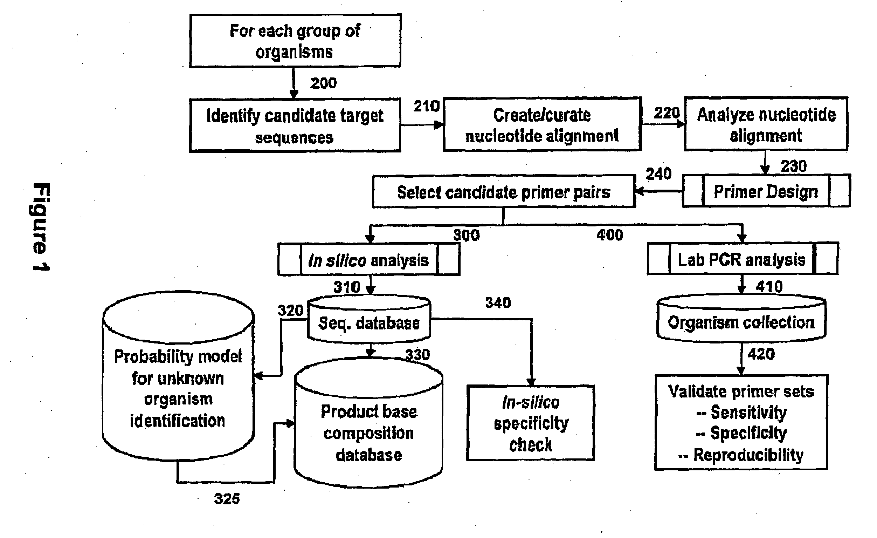

A representative process flow diagram used for primer selection and validation process is outlined in Figure 1. For each group of organisms, candidate target sequences are identified **(200)** from which nucleotide alignments are created **(210)** and analyzed **(220).** Primers are then designed by selecting appropriate priming regions **(230)** to facilitate the selection of candidate primer pairs **(240).** The primer pairs are then subjected to *in silico* analysis by electronic PCR (ePCR) **(300)** wherein bioagent identifying amplicons are obtained from sequence databases such as GenBank or other sequence collections **(310)** and checked for specificity *in silico* **(320).** Bioagent identifying amplicons obtained from GenBank sequences **(310)** can also be analyzed by a probability model which predicts the capability of a given amplicon to identify unknown bioagents such that the base compositions of amplicons with favorable probability scores are then stored in a base composition database **(325).** Alternatively, base compositions of the bioagent identifying amplicons obtained from the primers and GenBank sequences can be directly entered into the base composition database **(330).** Candidate primer pairs **(240)** are validated by testing their ability to hybridize to target nucleic acid by an in vitro amplification by a method such as PCR analysis **(400)** of nucleic acid from a collection of organisms **(410).** Amplification products thus obtained are analyzed by gel electrophoresis or by mass spectrometry to confirm the sensitivity, specificity and reproducibility of the primers used to obtain the amplification products **(420).**

Many of the important pathogens, including the organisms of greatest concern as biowarfare agents, have been completely sequenced. This effort has greatly facilitated the design of primers for the detection of unknown bioagents. The combination of broad-range priming with division-wide and drill-down priming has been used very successfully in several applications of the technology, including environmental surveillance for biowarfare threat agents and clinical sample analysis for medically important pathogens.

Synthesis of primers is well known and routine in the art. The primers may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed.

In some embodiments primers are employed as compositions for use in methods for identification of viral bioagents as follows: a primer pair composition is contacted with nucleic acid (such as, for example, DNA from a DNA virus, or DNA reverse transcribed from the RNA of an RNA virus) of an unknown viral bioagent. The nucleic acid is then amplified by a nucleic acid amplification technique, such as PCR for example, to obtain an amplification product that represents a bioagent identifying amplicon. The molecular mass of each strand of the double-stranded amplification product is determined by a molecular mass measurement technique such as mass spectrometry for example, wherein the two strands of the double-stranded amplification product are separated during the ionization process. In some embodiments, the mass spectrometry is electrospray Fourier transform ion cyclotron resonance mass spectrometry (ESI-FTICR-MS) or electrospray time of flight mass spectrometry (ESI-TOF-MS). A list of possible base compositions can be generated for the molecular mass value obtained for each strand and the choice of the correct base composition from the list is facilitated by matching the base composition of one strand with a complementary base composition of the other strand. The molecular mass or base composition thus determined is then compared with a database of molecular masses or base compositions of analogous bioagent identifying amplicons for known viral bioagents. A match between the molecular mass or base composition of the amplification product and the molecular mass or base composition of an analogous bioagent identifying amplicon for a known viral bioagent indicates the identity of the unknown bioagent. In some embodiments, the primer pair used is one of the primer pairs of Table 2. In some embodiments, the method is repeated using one or more different primer pairs to resolve possible ambiguities in the identification process or to improve the confidence level for the identification assignment.

In some embodiments, a bioagent identifying amplicon may be produced using only a single primer (either the forward or reverse primer of any given primer pair), provided an appropriate amplification method is chosen, such as, for example, low stringency single primer PCR (LSSP-PCR). Adaptation of this amplification method in order to produce bioagent identifying amplicons can be accomplished by one with ordinary skill in the art without undue experimentation.

In some embodiments, the oligonucleotide primers are broad range survey primers which hybridize to conserved regions of nucleic acid encoding the hexon gene of all (or between 80% and 100%, between 85% and 100%, between 90% and 100% or between 95% and 100%) known adenoviruses and produce adenovirus identifying amplicons.

In some cases, the molecular mass or base composition of a viral bioagent identifying amplicon defined by a broad range survey primer pair does not provide enough resolution to unambiguously identify a viral bioagent at or below the species level. These cases benefit from further analysis of one or more viral bioagent identifying amplicons generated from at least one additional broad range survey primer pair or from at least one additional division-wide primer pair. The employment of more than one bioagent identifying amplicon for identification of a bioagent is herein referred to as triangulation identification.

In other embodiments, the oligonucleotide primers are division-wide primers which hybridize to nucleic acid encoding genes of species within a genus of viruses. In other embodiments, the oligonucleotide primers are drill-down primers which enable the identification of sub-species, characteristics. Drill down primers provide the functionality of producing bioagent identifying amplicons for drill-down analyses such as strain typing when contacted with nucleic acid under amplification conditions. Identification of such sub-species characteristics is often critical for determining proper clinical treatment of viral infections. In some embodiments, sub-species characteristics are identified using only broad range survey primers and division-wide and drill-down primers are not used.

In some embodiments, the primers used for amplification hybridize to and amplify genomic DNA, DNA of bacterial plasmids, DNA of DNA viruses or DNA reverse transcribed from RNA of an RNA virus.

In some embodiments, the primers used for amplification hybridize directly to viral RNA and act as reverse transcription primers for obtaining DNA from direct amplification of viral RNA. Methods of amplifying RNA to produce cDNA using reverse transcriptase are well known to those with ordinary skill in the art and can be routinely established without undue experimentation.

In some embodiments, various computer software programs may be used to aid in design of primers for amplification reactions such as *Primer Premier 5* (Premier Biosoft, Palo Alto, CA) or *OLIGO* Primer Analysis Software (Molecular Biology Insights, Cascade, CO). These programs allow the user to input desired hybridization conditions such as melting temperature of a primer-template duplex for example. In some embodiments, an *in silico* PCR search algorithm, such as (ePCR) is used to analyze primer specificity across a plurality of template sequences which can be readily obtained from public sequence databases such as GenBank for example. An existing RNA structure search algorithm (Macke et al., Nucl. Acids Res., 2001, 29, 4724-4735, has been modified to include PCR parameters such as hybridization conditions, mismatches, and thermodynamic calculations (SantaLucia, Proc. Natl. Acad. Sci. U.S.A., 1998, 95, 1460-1465).
This also provides information on primer specificity of the selected primer pairs. In some embodiments, the hybridization conditions applied to the algorithm can limit the results of primer specificity obtained from the algorithm. In some embodiments, the melting temperature threshold for the primer template duplex is specified to be 35 °C or a higher temperature. In some embodiments the number of acceptable mismatches is specified to be seven mismatches or less. In some embodiments, the buffer components and concentrations and primer concentrations may be specified and incorporated into the algorithm, for example, an appropriate primer concentration is about 250 nM and appropriate buffer components are 50 mM sodium or potassium and 1.5 mM Mg²⁺.

One with ordinary skill in the art of design of amplification primers will recognize that a given primer need not hybridize with 100% complementarity in order to effectively prime the synthesis of a complementary nucleic acid strand in an amplification reaction. Moreover, a primer may hybridize over one or more segments such that intervening or adjacent segments are not involved in the hybridization event. (e.g., for example, a loop structure or a hairpin structure). The primers of the present invention may comprise at least 70%, at least 75%, at least 80%, at least 85%, at least 90%, at least 95% or at least 99% sequence identity with any of the primers listed in Table 2. Thus, in some embodiments of the present invention, an extent of variation of 70% to 100%, or any range therewithin, of the sequence identity is possible relative to the specific primer sequences disclosed herein. Determination of sequence identity is described in the following example: a primer 20 nucleobases in length which is identical to another 20 nucleobase primer having two non-identical residues has 18 of 20 identical residues (18/20 = 0.9 or 90% sequence identity). In another example, a primer 15 nucleobases in length having all residues identical to a 15 nucleobase segment of primer 20 nucleobases in length would have 15/20 = 0.75 or 75% sequence identity with the 20 nucleobase primer.

Percent homology, sequence identity or complementarity, can be determined by, for example, the Gap program (Wisconsin Sequence Analysis Package, Version 8 for UNIX, Genetics Computer Group, University Research Park, Madison WI), using default settings, which uses the algorithm of Smith and Waterman (Adv. Appl. Math., 1981, 2, 482-489). In some embodiments, complementarity of primers with respect to the conserved priming regions of viral nucleic acid is between about 70% and about 75% 80%. In other embodiments, homology, sequence identity or complementarity, is between about 75% and about 80%. In yet other embodiments, homology, sequence identity or complementarity, is at least 85%, at least 90%, at least 92%, at least 94%, at least 95%, at least 96%, at least 97%, at least 98%, at least 99% or is 100%.

In some embodiments, the primers described herein comprise at least 70%, at least 75%, at least 80%, at least 85%, at least 90%, at least 92%, at least 94%, at least 95%, at least 96%, at least 98%, or at least 99%, or 100% (or any range therewithin) sequence identity with the primer sequences specifically disclosed herein.

One with ordinary skill is able to calculate percent sequence identity or percent sequence homology and able to determine, without undue experimentation, the effects of variation of primer sequence identity on the function of the primer in its role in priming synthesis of a complementary strand of nucleic acid for production of an amplification product of a corresponding bioagent identifying amplicon.

In one embodiment, the primers are at least 13 nucleobases in length. In another embodiment, the primers are less than 36 nucleobases in length.

In some embodiments of the present invention, the oligonucleotide primers are 13 to 35 nucleobases in length (13 to 35 linked nucleotide residues). These embodiments comprise oligonucleotide primers 13, 14, 15, 16, 17, 18, 19, 20, 21, 22, 23, 24, 25, 26, 27, 28, 29, 30, 31, 32, 33, 34 or 35 nucleobases in length, or any range therewithin. The present invention contemplates using both longer and shorter primers. Furthermore, the primers may also be linked to one or more other desired moieties, including, but not limited to, affinity groups, ligands, regions of nucleic acid that are not complementary to the nucleic acid to be amplified, labels, etc. Primers may also form hairpin structures. For example, hairpin primers may be used to amplify short target nucleic acid molecules. The presence of the hairpin may stabilize the amplification complex (see e.g., TAQMAN MicroRNA Assays, Applied Biosystems, Foster City, California).

In some embodiments, any oligonucleotide primer pair may have one or both primers with less then 70% sequence homology with a corresponding member of any of the primer pairs of Table 2 if the primer pair has the capability of producing an amplification product corresponding to a bioagent identifying amplicon. In other embodiments, any oligonucleotide primer pair may have one or both primers with a length greater than 35 nucleobases if the primer pair has the capability of producing an amplification product corresponding to a bioagent identifying amplicon.

In some embodiments, the function of a given primer may be substituted by a combination of two or more primers segments that hybridize adjacent to each other or that are linked by a nucleic acid loop structure or linker which allows a polymerase to extend the two or more primers in an amplification reaction.

In some embodiments, the primer pairs used for obtaining bioagent identifying amplicons are the primer pairs of Table 2. In other embodiments, other combinations of primer pairs are possible by combining certain members of the forward primers with certain members of the reverse primers. An example can be seen in Table 2 for two primer pair combinations of forward primer HEX_HAD_-6_18_F (SEQ ID NO: 47), with the reverse primers HEX_HAD_86_105_2_R (SEQ ID NO: 123), HEX_HAD_61_84_R, or (SEQ ID NO: 81). Arriving at a favorable alternate combination of primers in a primer pair depends upon the properties of the primer pair, most notably the size of the bioagent identifying amplicon that would be produced by the primer pair, which should be between about 45 to about 150 nucleobases in length. Alternatively, a bioagent identifying amplicon longer than 150 nucleobases in length could be cleaved into smaller segments by cleavage reagents such as chemical reagents, or restriction enzymes, for example.

In some embodiments, the primers are configured to amplify nucleic acid of a bioagent to produce amplification products that can be measured by mass spectrometry and from whose molecular masses candidate base compositions can be readily calculated.

In some embodiments, any given primer comprises a modification comprising the addition of a non-templated T residue to the 5' end of the primer (i.e., the added T residue does not necessarily hybridize to the nucleic acid being amplified). The addition of a non-templated T residue has an effect of minimizing the addition of non-templated adenosine residues as a result of the non-specific enzyme activity of *Taq* polymerase (Magnuson et al., Biotechniques, 1996, 21, 700-709), an occurrence which may lead to ambiguous results arising from molecular mass analysis.

In some embodiments of the present invention, primers may contain one or more universal bases. Because any variation (due to codon wobble in the 3^{rd} position) in the conserved regions among species is likely to occur in the third position of a DNA (or RNA) triplet, oligonucleotide primers can be designed such that the nucleotide corresponding to this position is a base which can bind to more than one nucleotide, referred to herein as a "universal nucleobase." For example, under this "wobble" pairing, inosine (I) binds to U, C or A; guanine (G) binds to U or C, and uridine (U) binds to U or C. Other examples of universal nucleobases include nitroindoles such as 5-nitroindole or 3-nitropyrrole (Loakes et al., Nucleosides and Nucleotides, 1995, 14, 1001-1003), the degenerate nucleotides d**P** or d**K** (Hill *et al.*), an acyclic nucleoside analog containing 5-nitroindazole (Van Aerschot et al., Nucleosides and Nucleotides, 1995, 14, 1053-1056) or the purine analog 1-(2-deoxy-β-D-ribofuranosyl)-imidazole-4-carboxamide (Sala et al., Nucl. Acids Res., 1996, 24, 3302-3306).

In some embodiments, to compensate for the somewhat weaker binding by the wobble base, the oligonucleotide primers are designed such that the first and second positions of each triplet are occupied by nucleotide analogs that bind with greater affinity than the unmodified nucleotide. Examples of these analogs include, but are not limited to, 2,6-diaminopurine which binds to thymine, 5-propynyluracil (also known as propynylated thymine) which binds to adenine and 5-propynylcytosine and phenoxazines, including G-clamp, which binds to G. Propynylated pyrimidines are described in U.S. Patent Nos. 5,645,985, 5,830,653 and 5,484,908, each of which is commonly owned and incorporated herein by reference in its entirety. Propynylated primers are described in U.S Pre-Grant Publication No. 2003-0170682, which is also commonly owned. Phenoxazines are described in U.S. Patent Nos. 5,502,177, 5,763,588, and 6,005,096. G-clamps are described in U.S. Patent Nos. 6,007,992 and 6,028,183, each of which is incorporated herein by reference in its entirety.

In some embodiments, for broad priming of rapidly evolving RNA viruses, primer hybridization is enhanced using primers containing 5-propynyl deoxy-cytidine and deoxy-thymidine nucleotides. These modified primers offer increased affinity and base pairing selectivity.

In some embodiments, non-template primer tags are used to increase the melting temperature (Tₘ) of a primer-template duplex in order to improve amplification efficiency. A non-template tag is at least three consecutive A or T nucleotide residues on a primer which are not complementary to the template. In any given non-template tag, A can be replaced by C or G and T can also be replaced by C or G. Although Watson-Crick hybridization is not expected to occur for a non-template tag relative to the template, the extra hydrogen bond in a G-C pair relative to an A-T pair confers increased stability of the primer-template duplex and improves amplification efficiency for subsequent cycles of amplification when the primers hybridize to strands synthesized in previous cycles.

In other embodiments, propynylated tags may be used in a manner similar to that of the non-template tag, wherein two or more 5-propynylcytidine or 5-propynyluridine residues replace template matching residues on a primer. In other embodiments, a primer contains a modified internucleoside linkage such as a phosphorothioate linkage, for example.

In some embodiments, the primers contain mass-modifying tags. Reducing the total number of possible base compositions of a nucleic acid of specific molecular weight provides a means of avoiding a persistent source of ambiguity in determination of base composition of amplification products. Addition of mass-modifying tags to certain nucleobases of a given primer will result in simplification of *de novo* determination of base composition of a given bioagent identifying amplicon from its molecular mass.

In some embodiments of the present invention, the mass modified nucleobase comprises one or more of the following: for example, 7-deaza-2'-deoxyadenosine-5-triphosphate, 5-iodo-2'-deoxyuridine-5'-triphosphate, 5-bromo-2'-deoxyuridine-5'-triphosphate, 5-bromo-2'-deoxycyfidine-5'-triphosphate, 5-iodo-2'-deoxycytidine-5'-triphosphate, 5-hydroxy-2'-deoxyuridine-5'-triphosphate, 4-thiothymidine-5'-triphosphate, 5-aza-2'-deoxyuridine-5'-triphosphate, 5-fluoro-2'-deoxyuridine-5'-triphosphate, 06-methyl-2'-deoxyguanosine-5'-triphosphate, N2-methyl-2'-deoxyguanosine-5'-triphosphate, 8-oxo-2'-deoxyguanosine-5'-triphosphate or thiothymidine-5'-triphosphate. In some embodiments, the mass-modified nucleobase comprises ¹⁵N or ¹³C or both ¹⁵N and ¹³C.

In some embodiments, multiplex amplification is performed where multiple bioagent identifying amplicons are amplified with a plurality of primer pairs. The advantages of multiplexing are that fewer reaction containers (for example, wells of a 96- or 384-well plate) are needed for each molecular mass measurement, providing time, resource and cost savings because additional bioagent identification data can be obtained within a single analysis. Multiplex amplification methods are well known to those with ordinary skill and can be developed without undue experimentation. However, in some embodiments, one useful and non-obvious step in selecting a plurality candidate bioagent identifying amplicons for multiplex amplification is to ensure that each strand of each amplification product will be sufficiently different in molecular mass that mass spectral signals will not overlap and lead to ambiguous analysis results. In some embodiments, a 10 Da difference in mass of two strands of one or more amplification products is sufficient to avoid overlap of mass spectral peaks.

In some embodiments, as an alternative to multiplex amplification, single amplification reactions can be pooled before analysis by mass spectrometry. In these embodiments, as for multiplex amplification embodiments, it is useful to select a plurality of candidate bioagent identifying amplicons to ensure that each strand of each amplification product will be sufficiently different in molecular mass that mass spectral signals will not overlap and lead to ambiguous analysis results.

### C Determination of Molecular Mass of Bioagent Identifying Amplicons

In some embodiments, the molecular mass of a given bioagent identifying amplicon is determined by mass spectrometry. Mass spectrometry has several advantages, not the least of which is high bandwidth characterized by the ability to separate (and isolate) many molecular peaks across a broad range of mass to charge ratio (m/z). Thus mass spectrometry is intrinsically a parallel detection scheme without the need for radioactive or fluorescent labels, since every amplification product is identified by its molecular mass. The current state of the art in mass spectrometry is such that less than femtomole quantities of material can be readily analyzed to afford information about the molecular contents of the sample. An accurate assessment of the molecular mass of the material can be quickly obtained, irrespective of whether the molecular weight of the sample is several hundred, or in excess of one hundred thousand atomic mass units (amu) or Daltons.

In some embodiments, intact molecular ions are generated from amplification products using one of a variety of ionization techniques to convert the sample to gas phase. These ionization methods include, but are not limited to, electrospray ionization (ES), matrix-assisted laser desorption ionization (MALDI) and fast atom bombardment (FAB). Upon ionization, several peaks are observed from one sample due to the formation of ions with different charges. Averaging the multiple readings of molecular mass obtained from a single mass spectrum affords an estimate of molecular mass of the bioagent identifying amplicon. Electrospray ionization mass spectrometry (ESI-MS) is particularly useful for very high molecular weight polymers such as proteins and nucleic acids having molecular weights greater than 10 kDa, since it yields a distribution of multiply-charged molecules of the sample without causing a significant amount of fragmentation.

The mass detectors used in the methods of the present invention include, but are not limited to, Fourier transform ion cyclotron resonance mass spectrometry (FT-ICR-MS), time of flight (TOF), ion trap, quadrupole, magnetic sector, Q-TOF, and triple quadrupole.

### D. Base Compositions of Bioagent Identifying Amplicons

Although the molecular mass of amplification products obtained using intelligent primers provides a means for identification of bioagents, conversion of molecular mass data to a base composition signature is useful for certain analyses. As used herein, "base composition" is the exact number of each nucleobase (A, T, C and G) determined from the molecular mass of a bioagent identifying amplicon. In some embodiments, a base composition provides an index of a specific organism. Base compositions can be calculated from known sequences of known bioagent identifying amplicons and can be experimentally determined by measuring the molecular mass of a given bioagent identifying amplicon, followed by determination of all possible base compositions which are consistent with the measured molecular mass within acceptable experimental error. The following example illustrates determination of base composition from an experimentally obtained molecular mass of a 46-mer amplification product originating at position 1337 of the 16S rRNA of *Bacillus anthracis.* The forward and reverse strands of the amplification product have measured molecular masses of 14208 and 14079 Da, respectively. The possible base compositions derived from the molecular masses of the forward and reverse strands for the *B. anthracis* products are listed in Table 1.

**Table 1**

| **Possible Base Compositions for *B. anthracis* 46mer Amplification Product** | | | | | |
|---|---|---|---|---|---|
| **Calc. Mass Forward Strand** | **Mass Error Forward Strand** | **Base Composition of Forward Strand** | **Calc. Mass Reverse Strand** | **Mass Error Reverse Strand** | **Base Compositions of Reverse Strand** |
| 14208.2935 | 0.079520 | A1 G17 C10 T18 | 14079.2624 | 0.080600 | A0 G14 C13 T19 |
| 14208.3160 | 0.056980 | A1 G20 C15 T10 | 14079.2849 | 0.058060 | A0 G17 C18 T11 |
| 14208.3386 | 0.034440 | A1 G23 C20 T2 | 14079.3075 | 0.035520 | A0 G20 C23 T3 |
| 14208.3074 | 0.065560 | A6 G11 C3 T26 | 14079.2538 | 0.089180 | A5 G5 C1 T35 |
| 14208.3300 | 0.043020 | A6 G14 C8 T18 | 14079.2764 | 0.066640 | A5 G8 C6 T27 |
| 14208.3525 | 0.020480 | A6 G17 C13 T10 | 14079.2989 | 0.044100 | A5 G11 C11 T19 |
| 14208.3751 | 0.002060 | A6 G20 C18 T2 | 14079.3214 | 0.021560 | A5 G14 C16 T11 |
| 14208.3439 | 0.029060 | A11 G8 C1 T26 | 14079.3440 | 0.000980 | A5 G17 C21 T3 |
| 14208.3665 | 0.006520 | A11 G11 C6 T18 | 14079.3129 | 0.030140 | A10 G5 C4 T27 |
| **14208.3890** | **0.016020** | **A11 G14 C11 T10** | 14079.3354 | 0.007600 | A10 G8 C9 T19 |
| 14208.4116 | 0.038560 | A11 G17 C16 T2 | **14079.3579** | **0.014940** | **A10 G11 C14 T11** |
| 14208.4030 | 0.029980 | A16 G8 C4 T18 | 14079.3805 | 0.037480 | A10 G14 C19 T3 |
| 14208.4255 | 0.052520 | A16 G11 C9 T10 | 14079.3494 | 0.006360 | A15 G2 C2 T27 |
| 14208.4481 | 0.075060 | A16 G14 C14 T2 | 14079.3719 | 0.028900 | A15 G5 C7 T19 |
| 14208.4395 | 0.066480 | A21 G5 C2 T18 | 14079.3944 | 0.051440 | A15 G8 C12 T11 |
| 14208.4620 | 0.089020 | A21 G8 C7 T10 | 14079.4170 | 0.073980 | A15 G11 C17 T3 |
| - | - | - | 14079.4084 | 0.065400 | A20 G2 C5 T19 |
| - | - | - | 14079.4309 | 0.087940 | A20 G5 C10 T13 |

Among the 16 possible base compositions for the forward strand and the 18 possible base compositions for the reverse strand that were calculated, only one pair (shown in bold) are complementary base compositions, which indicates the true base composition of the amplification product. It should be recognized that this logic is applicable for determination of base compositions of any bioagent identifying amplicon, regardless of the class of bioagent from which the corresponding amplification product was obtained.

In some embodiments, assignment of previously unobserved base compositions (also known as "true unknown base compositions") to a given phylogeny can be accomplished via the use of pattern classifier model algorithms. Base compositions, like sequences, vary slightly from strain to strain within species, for example. In some embodiments, the pattern classifier model is the mutational probability model. On other embodiments, the pattern classifier is the polytope model. The mutational probability model and polytope model are both commonly owned and described in U.S. Patent application Serial No. 11/073,362.

In one embodiment, it is possible to manage this diversity by building "base composition probability clouds" around the composition constraints for each species. This permits identification of organisms in a fashion similar to sequence analysis. A "pseudo four-dimensional plot" can be used to visualize the concept of base composition probability clouds. Optimal primer design requires optimal choice of bioagent identifying amplicons and maximizes the separation between the base composition signatures of individual bioagents. Areas where clouds overlap indicate regions that may result in a misclassification, a problem which is overcome by a triangulation identification process using bioagent identifying amplicons not affected by overlap of base composition probability clouds.

In some embodiments, base composition probability clouds provide the means for screening potential primer pairs in order to avoid potential misclassifications of base compositions. In other embodiments, base composition probability clouds provide the means for predicting the identity of a bioagent whose assigned base composition was not previously observed and/or indexed in a bioagent identifying amplicon base composition database due to evolutionary transitions in its nucleic acid sequence. Thus, in contrast to probe-based techniques, mass spectrometry determination of base composition does not require prior knowledge of the composition or sequence in order to make the measurement.

The present invention provides bioagent classifying information similar to DNA sequencing and phylogenetic analysis at a level sufficient to identify a given bioagent. Furthermore, the process of determination of a previously unknown base composition for a given bioagent (for example, in a case where sequence information is unavailable) has downstream utility by providing additional bioagent indexing information with which to populate base composition databases. The process of future bioagent identification is thus greatly improved as more BCS indexes become available in base composition databases.

### E. Triangulation Identification

In some cases, a molecular mass of a single bioagent identifying amplicon alone does not provide enough resolution to unambiguously identify a given bioagent. The employment of more than one bioagent identifying amplicon for identification of a bioagent is herein referred to as "triangulation identification." Triangulation identification is pursued by determining the molecular masses of a plurality of bioagent identifying amplicons selected within a plurality of housekeeping genes. This process is used to reduce false negative and false positive signals, and enable reconstruction of the origin of hybrid or otherwise engineered bioagents. For example, identification of the three part toxin genes typical of B. *anthracis* (Bowen et al., J. Appl. Microbiol., 1999, 87, 270-278) in the absence of the expected signatures from the *B. anthracis* genome would suggest a genetic engineering event.

In some embodiments, the triangulation identification process can be pursued by characterization of bioagent identifying amplicons in a massively parallel fashion using the polymerase chain reaction (PCR), such as multiplex PCR where multiple primers are employed in the same amplification reaction mixture, or PCR in multi-well plate format wherein a different and unique pair of primers is used in multiple wells containing otherwise identical reaction mixtures. Such multiplex and multi-well PCR methods are well known to those with ordinary skill in the arts of rapid throughput amplification of nucleic acids. In other related embodiments, one PCR reaction per well or container may be carried out, followed by an amplicon pooling step wherein the amplification products of different wells are combined in a single well or container which is then subjected to molecular mass analysis. The combination of pooled amplicons can be chosen such that the expected ranges of molecular masses of individual amplicons are not overlapping and thus will not complicate identification of signals.

### F. Codon Base Composition Analysis

In some embodiments of the present invention, one or more nucleotide substitutions within a codon of a gene of an infectious organism confer drug resistance upon an organism which can be determined by codon base composition analysis. The organism can be a bacterium, virus, fungus or protozoan.

In some embodiments, the amplification product containing the codon being analyzed is of a length of about 35 to about 200 nucleobases. The primers employed in obtaining the amplification product can hybridize to upstream and downstream sequences directly adjacent to the codon, or can hybridize to upstream and downstream sequences one or more sequence positions away from the codon. The primers may have between about 70% to 100% sequence complementarity with the sequence of the gene containing the codon being analyzed.

In some embodiments, the codon base composition analysis is undertaken

In some embodiments, the codon analysis is undertaken for the purpose of investigating genetic disease in an individual. In other embodiments, the codon analysis is undertaken for the purpose of investigating a drug resistance mutation or any other deleterious mutation in an infectious organism such as a bacterium, virus, fungus or protozoan. In some embodiments, the virus is an adenovirus identified in a biological product.

In some embodiments, the molecular mass of an amplification product containing the codon being analyzed is measured by mass spectrometry. The mass spectrometry can be either electrospray (ESI) mass spectrometry or matrix-assisted laser desorption ionization (MALDI) mass spectrometry. Time-of-flight (TOF) is an example of one mode of mass spectrometry compatible with the analyses of the present invention.

The methods of the present invention can also be employed to determine the relative abundance of drug resistant strains of the organism being analyzed. Relative abundances can be calculated from amplitudes of mass spectral signals with relation to internal calibrants. In some embodiments, known quantities of internal amplification calibrants can be included in the amplification reactions and abundances of analyte amplification product estimated in relation to the known quantities of the calibrants.

In some embodiments, upon identification of one or more drug-resistant strains of an infectious organism infecting an individual, one or more alternative treatments can be devised to treat the individual.

### G. Determination of the Quantity of a Bioagent

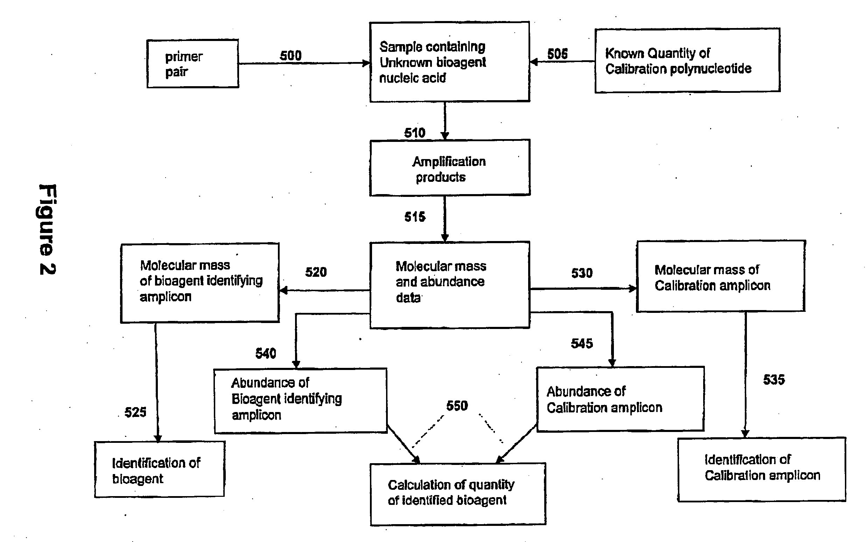

In some embodiments, the identity and quantity of an unknown bioagent can be determined using the process illustrated in Figure 2. Primers **(500)** and a known quantity of a calibration polynucleotide **(505)** are added to a sample containing nucleic acid of an unknown bioagent. The total nucleic acid in the sample is then subjected to an amplification reaction **(510)** to obtain amplification products. The molecular masses of amplification products are determined **(515)** from which are obtained molecular mass and abundance data. The molecular mass of the bioagent identifying amplicon **(520)** provides the means for its identification **(525)** and the molecular mass of the calibration amplicon obtained from the calibration polynucleotide **(530)** provides the means for its identification **(535).** The abundance data of the bioagent identifying amplicon is recorded **(540)** and the abundance data for the calibration data is recorded **(545),** both of which are used in a calculation **(550)** which determines the quantity of unknown bioagent in the sample.

A sample comprising an unknown bioagent is contacted with a pair of primers that provide the means for amplification of nucleic acid from the bioagent, and a known quantity of a polynucleotide that comprises a calibration sequence. The nucleic acids of the bioagent and of the calibration sequence are amplified and the rate of amplification is reasonably assumed to be similar for the nucleic acid of the bioagent and of the calibration sequence. The amplification reaction then produces two amplification products: a bioagent identifying amplicon and a calibration amplicon. The bioagent identifying amplicon and the calibration amplicon should be distinguishable by molecular mass while being amplified at essentially the same rate. Effecting differential molecular masses can be accomplished by choosing as a calibration sequence, a representative bioagent identifying amplicon (from a specific species of bioagent) and performing, for example, a 2-8 nucleobase deletion or insertion within the variable region between the two priming sites. The amplified sample containing the bioagent identifying amplicon and the calibration amplicon is then subjected to molecular mass analysis by mass spectrometry, for example. The resulting molecular mass analysis of the nucleic acid of the bioagent and of the calibration sequence provides molecular mass data and abundance data for the nucleic acid of the bioagent and of the calibration sequence. The molecular mass data obtained for the nucleic acid of the bioagent enables identification of the unknown bioagent and the abundance data enables calculation of the quantity of the bioagent, based on the knowledge of the quantity of calibration polynucleotide contacted with the sample.

In some embodiments, construction of a standard curve where the amount of calibration polynucleotide spiked into the sample is varied provides additional resolution and improved confidence for the determination of the quantity of bioagent in the sample. The use of standard curves for analytical determination of molecular quantities is well known to one with ordinary skill and can be performed without undue experimentation.

In some embodiments, multiplex amplification is performed where multiple bioagent identifying amplicons are amplified with multiple primer pairs which also amplify the corresponding standard calibration sequences. In this or other embodiments, the standard calibration sequences are optionally included within a single vector which functions as the calibration polynucleotide. Multiplex amplification methods are well known to those with ordinary skill and can be performed without undue experimentation.

In some embodiments, the calibrant polynucleotide is used as an internal positive control to confirm that amplification conditions and subsequent analysis steps are successful in producing a measurable amplicon. Even in the absence of copies of the genome of a bioagent, the calibration polynucleotide should give rise to a calibration amplicon. Failure to produce a measurable calibration amplicon indicates a failure of amplification or subsequent analysis step such as amplicon purification or molecular mass determination. Reaching a conclusion that such failures have occurred is in itself, a useful event.

In some embodiments, the calibration sequence is comprised of DNA. In some embodiments, the calibration sequence is comprised of RNA.

In some embodiments, the calibration sequence is inserted into a vector that itself functions as the calibration polynucleotide. In some embodiments, more than one calibration sequence is inserted into the vector that functions as the calibration polynucleotide. Such a calibration polynucleotide is herein termed a "combination calibration polynucleotide." The process of inserting polynucleotides into vectors is routine to those skilled in the art and can be accomplished without undue experimentation. Thus, it should be recognized that the calibration method should not be limited to the embodiments described herein. The calibration method can be applied for determination of the quantity of any bioagent identifying amplicon when an appropriate standard calibrant polynucleotide sequence is designed and used. The process of choosing an appropriate vector for insertion of a calibrant is also a routine operation that can be accomplished by one with ordinary skill without undue experimentation.

### H. Identification of Adenoviruses

In other embodiments of the present invention, the primer pairs produce bioagent identifying amplicons within stable and highly conserved regions of adenoviruses. The advantage to characterization of an amplicon defined by priming regions that fall within a highly conserved region is that there is a low probability that the region will evolve past the point of primer recognition, in which case, the primer hybridization of the amplification step would fail. Such a primer set is thus useful as a broad range survey-type primer. In another embodiment of the present invention, the intelligent primers produce bioagent identifying amplicons in a region which evolves more quickly than the stable region described above. The advantage of characterization bioagent identifying amplicon corresponding to an evolving genomic region is that it is useful for distinguishing emerging strain variants.

The present invention also has significant advantages as a platform for identification of diseases caused by emerging viruses such as, for example, members of the Adenoviridae family. The present invention eliminates the need for prior knowledge of bioagent sequence to generate hybridization probes. Thus, in another embodiment, the present invention provides a means of determining the etiology of a virus infection when the process of identification of viruses is carried out in a clinical setting and, even when the virus is a new species never observed before. This is possible because the methods are not confounded by naturally occurring evolutionary variations (a major concern for characterization of viruses which evolve rapidly) occurring in the sequence acting as the template for production of the bioagent identifying amplicon. Measurement of molecular mass and determination of base composition is accomplished in an unbiased manner without sequence prejudice.

Another embodiment of the present invention also provides a means of tracking the spread of adenovirus when a plurality of samples obtained from different locations are analyzed by the methods described above in an epidemiological setting. In one embodiment, a plurality of samples from a plurality of different locations is analyzed with primer pairs which produce bioagent identifying amplicons, a subset of which contains a specific adenovirus. The corresponding locations of the members of the adenovirus-containing subset indicate the spread of the specific virus to the corresponding locations.

### I. Kits

The present invention also provides kits according to the claims for carrying out the methods described herein. In some embodiments, the kit may comprise a sufficient quantity of one or more primer pairs to perform an amplification reaction on a target polynucleotide from a bioagent to form a bioagent identifying amplicon. In some embodiments, the kit may comprise from one to fifty primer pairs, from one to twenty primer pairs, from one to ten primer pairs, or from two to five primer pairs. In some embodiments, the kit may comprise one or more primer pairs recited in Table 2.

In some embodiments, the kit comprises one or more broad range survey primer(s), division wide primer(s), or drill-down primer(s), or any combination thereof. If a given problem involves identification of a specific bioagent, the solution to the problem may require the selection of a particular combination of primers to provide the solution to the problem. A kit may be designed so as to comprise particular primer pairs for identification of a particular bioagent. A drill-down kit may be used, for example, to distinguish different sub-species types of adenoviruses or genetically engineered adenoviruses. In some embodiments, the primer pair components of any of these kits may be additionally combined to comprise additional combinations of broad range survey primers and division-wide primers so as to be able to identify the adenovirus.

In some embodiments, the kit contains standardized calibration polynucleotides for use as internal amplification calibrants. Internal calibrants are described in commonly owned U.S. Patent Application Serial No: 60/545,425.

In some embodiments, the kit comprises a sufficient quantity of reverse transcriptase (if an RNA virus is to be identified for example), a DNA polymerase, suitable nucleoside triphosphates (including alternative dNTPs such as inosine or modified dNTPs such as the 5-propynyl pyrimidines or any dNTP containing molecular mass-modifying tags such as those described above), a DNA ligase, and/or reaction buffer, or any combination thereof, for the amplification processes described above. A kit may further include instructions pertinent for the particular embodiment of the kit, such instructions describing the primer pairs and amplification conditions for operation of the method. A kit may also comprise amplification reaction containers such as microcentrifuge tubes and the like. A kit may also comprise reagents or other materials for isolating bioagent nucleic acid or bioagent identifying amplicons from amplification, including, for example, detergents, solvents, or ion exchange resins which may be linked to magnetic beads. A kit may also comprise a table of measured or calculated molecular masses and/or base compositions of bioagents using the primer pairs of the kit.

In some embodiments, the kit includes a computer program stored on a computer formatted medium (such as a compact disk or portable USB disk drive, for example) comprising instructions which direct a processor to analyze data obtained from the use of the primer pairs of the present invention. The instructions of the software transform data related to amplification products into a molecular mass or base composition which is a useful concrete and tangible result used in identification and/or classification of bioagents. In some embodiments, the kits of the present invention contain all of the reagents sufficient to carry out one or more of the methods described herein.

While the present invention has been described with specificity in accordance with certain of its embodiments, the following examples serve only to illustrate the invention and are not intended to limit the same. In order that the invention disclosed herein may be more efficiently understood, examples are provided below. It should be understood that these examples are for illustrative purposes only and are not to be construed as limiting the invention in any manner.

### EXAMPLES

### Example 1: Design and Validation of Primers that Define Bioagent Identifying Amplicons for Adenoviruses

### A. General Process of Primer Design

For design of primers that define adenovirus identifying amplicons, a series of adenovirus genome segment sequences were obtained, aligned and scanned for regions where pairs of PCR primers would amplify products of about 45 to about 150 nucleotides in length and distinguish subgroups and/or individual serotypes from each other by their molecular masses or base compositions. A typical process shown in Figure 1 is employed for this type of analysis.

A database of expected base compositions for each primer region was generated using an *in si*/*ico* PCR search algorithm, such as (ePCR). An existing RNA structure search algorithm (Macke et al., Nucl. Acids Res., 2001, 29, 4724-4735) has been modified to include PCR parameters such as hybridization conditions, mismatches, and thermodynamic calculations (SantaLucia, Proc. Natl. Acad. Sci. U.S.A., 1998, 95, 1460-1465). This also provides information on primer specificity of the selected primer pairs.

### Example 2: Selection of Primers that Define Bioagent Identifying Amplicons for Identification of Adenoviruses

Initial primer design began with the design of primer pairs to produce bioagent identifying amplicons representing segments of the adenoviral hexon gene. These primer pairs were designed to perform a variety of tasks ranging from the general detection of all adenovirus strains to the identification of specific serotypes. Because, in some embodiments, base composition is the final analysis product, one primer pair can be used to identify many serotypes provided that the amplified region has sufficient variation (one base change or more). At the conclusion of the testing phase, a two primer pair test set was selected. These 2 primer pairs (primer pair nos: 943 (SEQ ID NOs: 61:122) and 769 (SEQ ID NOs: 26:121) produce amplicons whose base compositions specifically demonstrate the presence of adenovirus and, in most cases, are simultaneously diagnostic for the serotype of the adenovirus species present. In cases where the two primer pairs cannot specifically identify the serotype of the adenovirus present, other primers can be used to determine the information, such as, for example, any or all of primer pair numbers 1113 (SEQ ID NOs: 3 8:82), 1117 (SEQ ID NOs: 63:95), 1119 (SEQ ID NOs: 19:93), 1121 (SEQ ID NOs: 54:113), 1124 (SEQ ID NOs: 36:98), and 1126 (SEQ ID NOs: 16:106).

Table 2 represents a collection of primers (sorted by primer pair number) designed to identify adenoviruses using the methods described herein. Tp = 5-propynyluracil; Cp = 5-propynylcytosine. The primer pair number is an in-house database index number. Primer sites were identified on essential adenoviral genes, such as, for example, the hexon gene. The forward or reverse primer name shown in Table 2 indicates the gene region of the viral genome to which the primer hybridizes relative to a reference sequence. In Table 2, for example, the forward primer name HEX_HAD4_1442_1466_F indicates that the forward primer (_F) hybridizes to residues 1442-1466 of an adenovirus reference sequence represented by GenBank Accession No. X84646. GenBank Accession Numbers for reference sequences of the various serotypes of adenoviruses are shown in Table 3 (below) which is sorted according to primer pair number. In some cases, the reference sequences are extractions from adenovirus genomic sequences. One with ordinary skill knows how to obtain individual gene sequences or portions thereof from genomic sequences present in GenBank.

**Table 2: Primer Pairs for Identification of Adenoviruses**

| **Primer Pair Number** | **Forward Primer Name** | **Forward Sequence** | **Forward SEQ ID NO:** | **Reverse Primer Name** | **Reverse Sequence** | **Reverse SEQ ID NO:** |
|---|---|---|---|---|---|---|
| 194 | HEX_HAD7+4+21_934_9 53_F | AGACCCAATTACATTGGCTT | 2 | HEX_HAD7+4+21_976_995_R | CCAGTGCTGTTGTAGTACAT | 68 |
| 195 | HEX_HAD7+4+21_976_9 95_F | ATGTACTACAACAGTACTGG | 5 | REX_HAD7+4+21_1031_1050_R | CAAGTCAACCACAGCATTCA | 66 |
| 196 | HEX_HAD7+4+21_970_9 89_F | GGGCTTATGTACTACAACAG | 11 | HEX_HAD7+4+21_1039_1059_R | TCTGTCTTGCAAGTCAACCAC | 104 |
| 197 | HEX_HAD7+3_771_791_ F | GGAATTTTTTGATGGTAGAGA | 10 | HEX_HAD7+3_809_827_R | TAAAGCACAATTTCAGGCG | 76 |
| 198 | HEX_HAD4+16_746_765 _F | TAGATCTGGCTTTCTTTGAC | 22 | HEX_HAD4+16_828_848_R | ATATGAGTATCTGGAGTCTGC | 65 |
| 199 | HEX_HAD7_509_529_F | GGAAAGACATTACTGCAGACA | 9 | HEX_HAD7_559_578_R | CCAACTTGAGGCTCTGGCTG | 67 |
| 200 | HEX_HAD4_1216_1234_ F | ACAGACACTTACCAGGGTG | 1 | HEX_HAD4_1270_1289_R | ACTGTGGTGTCATCTTTGTC | 64 |
| 201 | HEX_HAD21_515_536_F | TCACTAAAGACAAAGGTCTTCC | 27 | HEX_HAD21_547_567_R | GGCTTCGCCGTCTGTAATTTC | 73 |
| 202 | HEX_HAD_1342_1362_M 1_F | CGGATCCAAGCTAATCTTTGG | 7 | HEX_HAD_1446_1469_M1_R | GGTATGTACTCATAGGTGTTGGTG | 75 |
| 203 | HEX_HAD7+4+21_934_9 53P_F | AGACpCpCAATTpACpATpTGGCTT | 2 | HEX_HAD7+4+21_976_995P_R | CpCpAGTGCTGTpTpGTAGTACAT | 70 |
| 204 | HEX_HAD7+4+21_976_9 95P_F | ATpGTpACTpACAACAGTACpTpGG | 5 | HEX_HAD7+4+21_1031_1050P_ R | CAAGTpCpAACCACAGCATpTpCA | 66 |
| 205 | HEX_HAD7+4+21_970_9 89P_F | GGGCpTpTATpGTpACTACAACpAG | 11 | HEX_HAD7+4+21_1039_1059P_ R | TCTGTpCpTTGCAAGTpCpAACCAC | 104 |
| 206 | HEX_RAD7+3_771_791P_F | GGAATTpTpTpTpTGATGGTAGAGA | 10 | HEX_HAD7+3_809_827P_R | TAAAGCACAATpTpTpCpAGGCG | 76 |
| 207 | HEX_HAD4+16_746_765 P_F | TAGATCTGGCTpTpTpCpTTTGAC | 22 | HEX_HAD4+16_828_848P_R | ATATGAGTATpCpTpGGAGTpCpTGC | 65 |
| 208 | HEX_HAD_1342_1362P_ M1_F | CGGATpCCAAGCpTAATCpTpTTGG | 7 | HEX_HAD_1446_1469P_M1_R | GGTATGTACTCATAGGTGTpTpGGTG | 75 |
| 214 | HEX_HAD2_772_795_F | CAATCCGTTCTGGTTCCGGATGAA | 6 | HEX_HAD2_842_865_R | CTTGCCGGTCGTTCAAAGAGGTAG | 72 |
| 216 | HEX_HAD7+4+21_73_90 _F | AGTCCGGGTCTGGTGCAG | 3 | HEX_HAD7+4+21_163_179_R | CGGTCGGTGGTCACATC | 69 |
| 217 | HEX_HAD7+4+21_1_18_ F | ATGGCCACCCCATCGATG | 4 | HEX_HAD7+4+21_36_54_R | CTGTCCGGCGATGTGCATG | 71 |
| 218 | HEX_HAD7+4+21-1612_ 1634_F | GGTCGTTATGTGCCTTTCCACAT | 12 | HEX_HAD7+4+21_1694_1718_R | TCCTTTCTGAAGTTCCACTCATAGG | 99 |
| 613 | HEX_HAD_-3_17_F | GATATGGCCACCCCATCGAT | 8 | HEX_HAD_80_97_R | GGGCGAACTGCACCAGAC | 74 |
| 614 | HEX_HAD_-4_17_F | TGATATGGCCACCCCATCGAT | 45 | HEX_HAD_80_98_R | TGGGCGAACTGCACCAGAC | 119 |
| 615 | HEX_HAD_-4_17P_F | TGATATGGCCACCCpCpATCGAT | 45 | HEX_HAD_82_99P_R | TCGGGCGAACTGCACpCpAG | 102 |
| 616 | HEX_HAD_-4_18P_F | TGATATGGCCACCCpCpATCGATG | 46 | HEX_HAD_86_101P_R | TCGCGGGCpGAACTGCpA | 100 |
| 617 | HEX_HAD_24_45P_F | TCAATGGGCATACpATGCpACATC | 24 | HEX_HAD_82_98P_R | TGGGCGAACTGCACpCpAG | 118 |
| 618 | HEX_HAD_1620_1643_F | TGTGCCTTTCCACATACAGGTGCC | 58 | HEX_HAD_1702_1727_R | TTGTTCACATCCTTGCTGAAGTTCCA | 128 |
| 619 | HEX_HADA_537_556_F | TCAGCCAGAGCCGCAAGTAG | 28 | HEX_HADA_635_654_R | TGCGTAGGAGCCATAGCACG | 112 |
| 620 | HEX_HADB_658_677P_F | TCATGCTACGGGTpCpTTTTGC | 29 | HEX_HADB_760_786P_R | | 96 |
| 621 | HEX_HADC_630_650P_F | TACCTTTCAACCTGAACpCpTCA | 18 | HEX_HADC_724_744P_R | TGAACpCpGTAGCATGGTTTCAT | 105 |
| 622 | HEX_HADD_695_713P_F | TCAACATGGGTGTGCpTpGGC | 23 | HEX_HADD_745_767P_R | TCGGTATTTCpTpGTCTTGCAAGTC | 103 |
| 623 | HEX_HADE_1214_1232P _F | TGACAGACACpTpTpACCAGGG | 42 | HEX_HADE_1318_1339P_R | TGATTpTpCpCATGGCAAAAGGATT | 108 |
| 624 | HEX_HADF_799_815P_F | TAAGCGCGCpCpGATACCCA | 16 | HEX_HADF_895_919P_R | TGAAGTTGTCpCpCpTAAAACCAATGTA | 106 |
| 625 | HEX_HAD4_741_765P_F | | 43 | HEX_HAD4_828_849P_R | TATATGAGTpATpCTpGGAGTCTGC | 87 |
| 626 | HEX_HAD4_1215_1234P _F | TACAGACACpTpTpACCAGGGTG | 17 | HEX_HAD4_1270_1290P_R | TACTGTGGTGTCATpCpTpTTGTC | 80 |
| 627 | HEX_HAD7_770_791P_F | TGGAATpTpTpTTCGATGGTAGAGA | 53 | HEX_HAD7_809_828P_R | TTAAAGCACAATpTpTpCAGGCG | 126 |
| 628 | HEX_HAD7_507_529P_F | TTGGAAAGACATpTpACTGCAGACA | 62 | HEX_HAD7_559_578P_R | TCAACTpTpGAGGCTCTGGCTG | 88 |
| 629 | HEX_HAD21_510_536P_ F | | 44 | HEX_HAD21_547_567P_R | TGCTTCGCCGTpCpTGTAATTTC | 114 |
| 630 | HEX_HAD7_930_953P_F | TAACAGACCpCAATTACpATTGGCTT | 15 | HEX_HAD7_976_997P_R | TGCCpAGTGCTGTTGTAGTACpAT | 111 |
| 631 | HEX_HAD7_972_996P_F | | 39 | HEX_HAD7_1031_1052P_R | TpGCAAGTpCAACCACAGCATTCA | 125 |
| 632 | HEX_HAD7_966_989P_F | TGTCGGGCTTATCTpACTpACAACAG | 57 | HAD7_1039_1059P_R | TCTGTCTTpGCAAGTpCAACCAC | 104 |
| 638 | HEX_HAD7_756_784P.1 _F | | 59 | HEX_HAD7_817_846P.1_R | | 77 |
| 639 | HEX_HAD7_650_677P.1 _F | | 56 | HEX_HAD7_760_787P.1_R | | 79 |
| 640 | HEX_HAD7_1197_1223P .1_F | | 33 | HEX_HAD7_1301_1330P.1_R | | 89 |
| 641 | HEX_HAD_1620_1643_F | TGTGCCTTTCCACATACAGGTGCC | 58 | HEX_HAD_1702_1734_R | | 85 |
| 707 | HEX_HAD_-4_17_2_F | TGATATGGCCACCCCATCGAT | 45 | HEX_HAD_82_99_R | TCGGGCGAACTGCACCAG | 102 |
| 708 | HEX_HAD_24_45_F | TCAATGGGCATACATGCACATC | 24 | HEX_HAD_82_98_R | TGGGCGAACTGCACCAG | 118 |
| 709 | HEX_HADB_658_677_F | TCATGCTACGGGTCTTTTGC | 29 | HEX_HADB_760_786_R | TCCATCGAAAAATTCCATGTCAATATC | 94 |
| 710 | HEX_HADC_630_650_F | TACCTTTCAACCTGAACCTCA | 18 | HEX_HADC_724_744_R | TGAACCGTAGCATGGTTTCAT | 105 |
| 711 | HEX_HADD_695_713_F | TCAACATGGGTGTGCTGGC | 23 | HEX_HADD_745_767_R | TCGGTATTTCTGTCTTGCAAGTC | 103 |
| 712 | HEX_HADE_1214_1232_ F | TGACAGACACTTACCAGGG | 42 | HEX_HADE_1318_1339_R | TGATTTCCATGGCAAAAGGATT | 109 |
| 714 | HEX_HAD7_930_953_F | TAACAGACCCAATTACATTGGCTT | 15 | HEX_HAD7_976_997_R | TGCCAGTGCTGTTGTAGTACAT | 111 |
| 715 | HEX_HAD7_972_996_F | TCTTATGTACTACAACAGCACTGGA | 39 | HEX_HAD7_1031_1052_R | TGCAAGTCAACCACAGCATTCA | 110 |
| 716 | HEX_HAD7_966_989_F | TGTCGGGCTTATGTACTACAACAG | 57 | HEX_HAD7_1039_1059_R | TCTGTCTTGCAAGTCAACCAC | 104 |
| 717 | HEX_HAD7_756_784.1_ F | | 59 | HEX_HAD7_817_846.1_R | | 78 |
| 718 | HEX_HAD7_650_677.1_ F | | 56 | HEX_HAD7_760_787.1_R | TACTATCAAAGAAAGCCAGGTCTATATC | 79 |
| 719 | HEX_HAD7_1197_1223. 1_F | | 34 | HEX_HAD7_1301_1330.1_R | | 89 |
| 720 | HEX_HAD7_971_995P_F | | 51 | HEX_HAD7_1031_1052P_R | TpGCAAGTpCAACCACAGCATTCA | 125 |
| 721 | HEX_HAD7_971_995_F | TGCTTATGTACTACAACAGCACTGG | 50 | HEX_HAD7_1031_1052_R | TGCAAGTCAACCACAGCATTCA | 110 |
| 739 | HEX_HAD4_1438_1466_ F | | 30 | HEX_HAD4_1558_1576_R | TCGCGTTGCGGTGGTGGTT | 101 |
| 740 | HEX_HAD4_1446_1469_ F | TAACACCTACGAGTACATGAACGG | 14 | HEX_HAD4_1552_1571_R | TTGCGGTGGTGGTTGAAGGG | 127 |
| 741 | HEX_HAD4_1013_1037_ F | TGAATGCTGTGGTTGACTTGCAAGA | 40 | HEX_HAD4_1106_1130_R | TAGCTGTCCACCGCCTGATTCCACA | 84 |
| 742 | HEX_HAD4_1013_1041_ F | | 41 | HEX_HAD4_1106_1130_2_R | TAGCTGTCCACAGCCTGATTCCACA | 83 |
| 743 | HEX_HAD4_961_985_F | TACTACAACAGCACTGGCAATATGG | 20 | HEX_HAD4_1021_1045_R | TGTTTCTGTCTTGCAAGTCAACCAC | 124 |
| 768 | HEX_HAD4_1442_1465_ F | TCACCAACACCTACGAGTACATGA | 25 | HEX_HAD4_1538_1562_R | TGGTTGAAGGGATTTACGTTGTCCA | 120 |
| 769 | HEX_HAD4_1442_1466_ F | TCACCAACACCTACGAGTACATGAA | 26 | HEX_HAD4_1537_1562_R | TGGTTGAAGGGATTTACGTTGTCCAT | 121 |
| 901 | HEX_HAD7_1197_1223P .1_F | | 33 | HEX_HAD7_1301_1330P.1_R | | 89 122 |
| 943 | HEX_HAD_-7_17_F | TTGCAAGATGGCCACCCCATCGAT | 61 | HEX_HAD_86_105_R | TGTGGCGCGGGCGAACTGCA | 116 |
| 944 | HEX_HAD_20_45_F | TGCCCCAATGGGCATACATGCACATC | 49 | HEX_HAD_86_103_R | TGGCGCGGGCGAACTGCA | 122 |
| 945 | HEX_HAD_20_45_F | TGCCCCAATGGGCATACATGCACATC | 47 | HEX_HAD_86_105_R | TGTGGCGCGGGCGAACTGCA | 123 |
| 946 | HEX_HAD_-6_18_F | TGCAAGATGGCCACCCCATCGATG | 47 | HEX_HAD_86_105_2_R | TGTTGCGCGGGCGAACTGCA | 123 |
| 947 | HEX_HAD_-6_18_F | TGCAAGATGGCCACCCCATCGATG | 47 | HEX_HAD_61_84_R | TAGACCCGGACTCAGGTACTCCGA | 81 |
| 948 | HEX_HAD_22_45_F | TCCCAATGGGCATACATGCACATC | 31 | HEX_HAD_8__99_R | TCGGGCGAACTGCACCAG | 102 |
| 1113 | HEX_HADA_532_556_F | TCTTATCAGCCAGAGCCGCAAGTAG | 38 | HEX_HADA_635_655_R | TAGCGTAGGAGCCATAGCACG | 82 |
| 1114 | HEX_HADA_1989 2018_ F | | 32 | HEX_HADA_2060_2086_R | TGGAGGAGTCAAACATGATTGACACCT | 115 |
| 1115 | HEX_HADA_2055 2084_ F | | 13 | HEX_HADA_2129_2156_R | TCCCCATCCACAGAACGCTTTATTTCAA | 97 |
| 1116 | HEX_HADB_656_677_F | TGCCATGCTACGGGTCTTTTGC | 48 | HEX_HADB_760_790_R | | 92 |
| 1117 | HEX_HADB_1633_1660_ F | | 63 | HEX_HADB_1702_1730_R | | 95 |
| 1118 | HEX_HADC_1783_1810_ F | | 52 | HEX_HADC_1900_1919_R | TAGGCGGTGTTGTGGGCCAT | 86 |
| 1119 | HEX_HADC_1537_1562_ F | TACGACTACATGAACAAGCGAGTGGT | 19 | HEX_HADC_1642_1662_R | TCCAGCATTGCGGTGGTGGTT | 93 |
| 1120 | HEX_HADC_1436_1463_F | | 35 | HEX_HADC_1552_1570_R | TGGGAGCCACCACTCGCTT | 117 |
| 1121 | HEX_HADD_693_713_F | TGGCAACATGGGTGTGCTGGC | 54 | HEX_HADD_745_770_R | TGCTCGGTATTTCTGTCTTGCAAGTC | 113 |
| 1122 | HEX_HADD_621_646_F | TTCCATGCCCAACAGACCCAACTACA | 60 | HEX_HADD_697_719_R | TGACCAGCCAGCACACCCATGTT | 107 |
| 1123 | HEX_HADE_1208_1232_ F | TGGGATTGACAGATACTTACCAGGG | 55 | HEX_HADE_1318_1339_2_R | TGATTTCCATGGCAAAAGGATT | 109 |
| 1124 | HEX_HADE_665_689_F | TCGCCAAGCCTACCAACAAAGAAGG | 36 | HEX_HADE_752_779_R | TCCGTAGTTTTGCTGTCAAAGAAAGCCA | 98 |
| 1125 | HEX_HADE_599_625_F | | 21 | HEX_HADE_667_692_R | TCACCTTCTTTGTTGGTAGGCTTGGC | 90 |
| 1126 | HEX_HADF_799_815_F | TAAGCGCCCGATACCCA | 16 | HEX_HADF_895_919_R | TGAAGTTGTCCCTAAAACCAATGTA | 106 |
| 1127 | HEX_HADF_1847_1869_F | TCTACCTCTGCGCTGCAAACATG | 37 | HEX_HADF_1908_1934_R | TCAGCCCAATTTCGCGAAGGAATAGAA | 91 |

**Table 3: Reference Sequence Details for Primer Pair Name Coordinates**

| **Primer Pair No.** | **Reference Sequence Adenovirus Type** | **Source for Reference Sequence (GenBank Accession No.)** | **Reference SEQ ID NO:** | **Specificity (Adenovirus Groups and Types)** |
|---|---|---|---|---|
| 194 | 7 | Z48571 | 129 | Types 7, 4 and 21 |
| 195 | 7 | Z48571 | 129 | Types 7, 4 and 21 |
| 196 | 7 | Z48571 | 129 | Types 7, 4 and 21 |
| 197 | 7 | Z48571 | 129 | Types 7 and 3 |
| 198 | 4 | X84646 | 130 | Types 4 and 16 |
| 199 | 7 | Z48571 | 129 | Type 7 |
| 200 | 4 | X84646 | 130 | Type 4 |
| 201 | 21 | AY008279 | 131 | Type 21 |
| 202 | 7 | Z48571 | 129 | All |
| 203 | 7 | Z48571 | 129 | Types 7, 4 and 21 |
| 204 | 7 | Z48571 | 129 | Types 7, 4 and 21 |
| 205 | 7 | Z48571 | 129 | Types 7, 4 and 21 |
| 206 | 7 | Z48571 | 129 | Types 7 and 3 |
| 207 | 4 | X84646 | 130 | Types 4 and 16 |
| 208 | 7 | Z48571 | 129 | All |
| 214 | 2 | AJ278924 | 132 | Type 2 |
| 216 | 7 | Z48571 | 129 | Types 7, 4 and 21 |
| 217 | 7 | Z48571 | 129 | Types 7, 4 and 21 |
| 218 | 7 | Z48571 | 129 | Types 7, 4 and 21 |
| 613 | 7 | Z48571 | 129 | All |
| 614 | 7 | Z48571 | 129 | All |
| 615 | 7 | Z48571 | 129 | All |
| 616 | 7 | Z48571 | 129 | All |
| 617 | 7 | Z48571 | 129 | All |
| 618 | 7 | Z48571 | 129 | All |
| 619 | 12 | X73487 | 133 | Adenovirus A |
| 620 | 7 | Z48571 | 129 | Adenovirus B |
| 621 | 1 | AF534906 | 134 | Adenovirus C |
| 622 | 8 | AB090341 | 135 | Adenovirus D |
| 623 | 4 | X84646 | 130 | Adenovirus E |
| 624 | 40 | L19443 | 136 | Adenovirus F |
| 625 | 4 | X84646 | 130 | Types 4 and 16 |
| 626 | 4 | X84646 | 130 | Type 4 |
| 627 | 7 | Z48571 | 129 | Types 3 and 7 |
| 628 | 7 | Z48571 | 129 | Type 21 |
| 629 | 21 | AY008279 | 131 | Type 21 |
| 630 | 7 | Z48571 | 129 | Types 3, 4, 7 and 21 |
| 631 | 7 | Z48571 | 129 | Types 3, 4, 7 and 21 |
| 632 | 7 | Z48571 | 129 | Types 3, 4, 7 and 21 |
| 638 | 7 | Z48571 | 129 | Types 3, 4, 7 and 21 |
| 639 | 7 | Z48571 | 129 | Types 3, 4, 7 and 21 |
| 640 | 7 | Z48571 | 129 | Types 3, 4, 7 and 21 |
| 641 | 7 | Z48571 | 129 | All |
| 707 | 7 | Z48571 | 129 | All |
| 708 | 7 | Z48571 | 129 | All |
| 709 | 7 | Z48571 | 129 | Adenovirus B |
| 710 | 1 | AF534906 | 134 | Adenovirus C |
| 711 | 8 | AB090341 | 135 | Adenovirus D |
| 712 | 4 | X84646 | 130 | Adenovirus E |
| 714 | 7 | Z48571 | 129 | Types 3, 4, 7 and 21 |
| 715 | 7 | Z48571 | 129 | Types 3, 4, 7 and 21 |
| 716 | 7 | Z48571 | 129 | Types 3, 4, 7 and 21 |
| 717 | 7 | Z48571 | 129 | Types 3, 4, 7 and 21 |
| 718 | 7 | Z48571 | 129 | Types 3, 4, 7 and 21 |
| 719 | 7 | Z48571 | 129 | Types 3, 4, 7 and 21 |
| 720 | 7 | Z48571 | 129 | Types 3, 4, 7 and 21 |
| 721 | 7 | Z48571 | 129 | Types 3, 4, 7 and 21 |
| 739 | 4 | X84646 | 130 | Groups A, B, D and E with Type Resolution |
| 740 | 4 | X84646 | 130 | Groups A, B, D and E with Type Resolution |
| 741 | 4 | X84646 | 130 | Type 4 and others |
| 742 | 4 | X84646 | 130 | Type 4 and others |
| 743 | 4 | X84646 | 130 | Type 4 and others |
| 768 | 4 | X84646 | 130 | Groups A, B, D and E with Type Resolution |
| 769 | 4 | X84646 | 130 | Groups A, B, D and E with Type Resolution |
| 901 | 7 | Z48571 | 129 | Types 3, 4, 7 and 21 |
| 943 | 7 | Z48571 | 129 | All |
| 944 | 7 | Z48571 | 129 | All |
| 945 | 7 | Z48571 | 129 | All |
| 946 | 7 | Z48571 | 129 | All |
| 947 | 7 | Z48571 | 129 | All |
| 948 | 7 | Z48571 | 129 | All |
| 1113 | 12 | X73487 | 133 | Adenovirus A |
| 1114 | 12 | X73487 | 133 | Adenovirus A |
| 1115 | 12 | X73487 | 133 | Adenovirus A |
| 1116 | 7 | Z48571 | 129 | Adenovirus B |
| 1117 | 7 | Z48571 | 129 | Adenovirus B |
| 1118 | 1 | AF534906 | 134 | Adenovirus C |
| 1119 | 1 | AF534906 | 134 | Adenovirus C |
| 1120 | 1 | AF534906 | 134 | Adenovirus C |
| 1121 | 8 | AB090341 | 135 | Adenovirus D |
| 1122 | 8 | AB090341 | 135 | Adenovirus D |
| 1123 | 4 | X84646 | 130 | Adenovirus E |
| 1124 | 4 | X84646 | 130 | Adenovirus E |
| 1125 | 4 | X84646 | 130 | Adenovirus E |
| 1126 | 40 | L19443 | 136 | Adenovirus F |
| 1127 | 40 | L19443 | 136 | Adenovirus F |

### Example 3: Sampling Procedures

Samples were gathered from military barracks during an IRB approved study conducted by the Naval Health Research Center Respiratory Disease Laboratory, San Diego. Environmental samples were obtained from eight locations and included surface swabs and air samples collected by dry filter unit air collection and electronic air collectors. Clinical surveillance was conducted by obtaining 1,700 clinical samples from throat, serum and hand swabs using standard clinical protocols which are well known to those with ordinary skill.

### Example 4: Sample Preparation and PCR

Samples were processed to obtain viral genomic material using a Qiagen QIAamp Virus BioRobot MDx Kit. Resulting genomic material was amplified using an Eppendorf thermal cycler and the amplicons were characterized on a Bruker Daltonics MicroTOF instrument. The resulting data was analyzed using GenX software (SAIC, San Diego, CA and Ibis, Carlsbad, CA).

All PCR reactions were assembled in 50 µL reaction volumes in a 96-well microtiter plate format using a Packard MPII liquid handling robotic platform and M.J. Dyad thermocyclers (MJ research, Waltham, MA). The PCR reaction mixture consisted of 4 units of Amplitaq Gold, 1x buffer II (Applied Biosystems, Foster City, CA), 1.5 mM MgCl₂, 0.4 M betaine, 800 µM dNTP mixture and 250 nM of each primer. The following typical PCR conditions were used: 95°C for 10 min followed by 8 cycles of 95°C for 30 seconds, 48°C for 30 seconds, and 72°C 30 seconds with the 48°C annealing temperature increasing 0.9°C with each of the eight cycles. The PCR was then continued for 37 additional cycles of 95°C for 15 seconds, 56°C for 20 seconds, and 72°C 20 seconds.

### Example 5: Solution Capture Purification of PCR Products for Mass Spectrometry with Ion Exchange Resin-Magnetic Beads

For solution capture of nucleic acids with ion exchange resin linked to magnetic beads, 25 µl of a 2.5 mg/mL suspension of BioClone amine terminated superparamagnetic beads were added to 25 to 50 µl of a PCR (or RT-PCR) reaction containing approximately 10 pM of a typical PCR amplification product. The above suspension was mixed for approximately 5 minutes by vortexing or pipetting, after which the liquid was removed after using a magnetic separator. The beads containing bound PCR amplification product were then washed three times with 50mM ammonium bicarbonate/50% MeOH or 100mM ammonium bicarbonate/50% MeOH, followed by three more washes with 50% MeOH. The bound PCR amplicon was eluted with a solution of 25mM piperidine, 25mM imidazole, 35% MeOH which included peptide calibration standards.

### Example 6: Mass Spectrometry and Base Composition Analysis

The ESI-FTICR mass spectrometer is based on a Bruker Daltonics (Billerica, MA) Apex II 70e electrospray ionization Fourier transform ion cyclotron resonance mass spectrometer that employs an actively shielded 7 Tesla superconducting magnet. The active shielding constrains the majority of the fringing magnetic field from the superconducting magnet to a relatively small volume. Thus, components that might be adversely affected by stray magnetic fields, such as CRT monitors, robotic components, and other electronics, can operate in close proximity to the FTICR spectrometer. All aspects of pulse sequence control and data acquisition were performed on a 600 MHz Pentium II data station running Bruker's Xmass software under Windows NT 4.0 operating system. Sample aliquots, typically 15 µl, were extracted directly from 96-well microtiter plates using a CTC HTS PAL autosampler (LEAP Technologies, Carrboro, NC) triggered by the FTICR data station. Samples were injected directly into a 10 µl sample loop integrated with a fluidics handling system that supplies the 100 µl /hr flow rate to the ESI source. Ions were formed via electrospray ionization in a modified Analytica (Branford, CT) source employing an off axis, grounded electrospray probe positioned approximately 1.5 cm from the metalized terminus of a glass desolvation capillary. The atmospheric pressure end of the glass capillary was biased at 6000 V relative to the ESI needle during data acquisition. A counter-current flow of dry N₂ was employed to assist in the desolvation process. Ions were accumulated in an external ion reservoir comprised of an rf-only hexapole, a skimmer cone, and an auxiliary gate electrode, prior to injection into the trapped ion cell where they were mass analyzed. Ionization duty cycles greater than 99% were achieved by simultaneously accumulating ions in the external ion reservoir during ion detection. Each detection event consisted of 1M data points digitized over 2.3 s. To improve the signal-to-noise ratio (S/N), 32 scans were co-added for a total data acquisition time of 74 s.

The ESI-TOF mass spectrometer is based on a Bruker Daltonics MicroTOF™. Ions from the ESI source undergo orthogonal ion extraction and are focused in a reflectron prior to detection. The TOF and FTICR are equipped with the same automated sample handling and fluidics described above. Ions are formed in the standard MicroTOF™ ESI source that is equipped with the same off-axis sprayer and glass capillary as the FTICR ESI source. Consequently, source conditions were the same as those described above. External ion accumulation was also employed to improve ionization duty cycle during data acquisition. Each detection event on the TOF was comprised of 75,000 data points digitized over 75 µs.

The sample delivery scheme allows sample aliquots to be rapidly injected into the electrospray source at high flow rate and subsequently be electrosprayed at a much lower flow rate for improved ESI sensitivity. Prior to injecting a sample, a bolus of buffer was injected at a high flow rate to rinse the transfer line and spray needle to avoid sample contamination/carryover. Following the rinse step, the autosampler injected the next sample and the flow rate was switched to low flow. Following a brief equilibration delay, data acquisition commenced. As spectra were co-added, the autosampler continued rinsing the syringe and picking up buffer to rinse the injector and sample transfer line. In general, two syringe rinses and one injector rinse were required to minimize sample carryover. During a routine screening protocol a new sample mixture was injected every 106 seconds. More recently a fast wash station for the syringe needle has been implemented which, when combined with shorter acquisition times, facilitates the acquisition of mass spectra at a rate of just under one spectrum/minute.

Raw mass spectra were post-calibrated with an internal mass standard and deconvoluted to monoisotopic molecular masses. Unambiguous base compositions were derived from the exact mass measurements of the complementary single-stranded oligonucleotides. Quantitative results are obtained by comparing the peak heights with an internal PCR calibration standard present in every PCR well at 500 molecules per well. Calibration methods are commonly owned and disclosed in U.S. Provisional Patent application Serial No. 60/545,425.

### Example 7: De Novo Determination of Base Composition of Amplification Products using Molecular Mass Modified Deoxynucleotide Triphosphates

Because the molecular masses of the four natural nucleobases have a relatively narrow molecular mass range (A = 313.058, G = 329.052, C = 289.046, T = 304.046 - See Table 4), a persistent source of ambiguity in assignment of base composition can occur as follows: two nucleic acid strands having different base composition may have a difference of about 1 Da when the base composition difference between the two strands is G ↔ A (-15.994) combined with C ↔ T (+15.000). For example, one 99-mer nucleic acid strand having a base composition of A₂₇G₃₀C₂₁T₂₁ has a theoretical molecular mass of 30779.058 while another 99-mer nucleic acid strand having a base composition of A₂₆G₃₁C₂₂T₂₀ has a theoretical molecular mass of 30780.052. A1 Da difference in molecular mass may be within the experimental error of a molecular mass measurement and thus, the relatively narrow molecular mass range of the four natural nucleobases imposes an uncertainty factor.

The present invention provides for a means for removing this theoretical 1 Da uncertainty factor through amplification of a nucleic acid with one mass-tagged nucleobase and three natural nucleobases. The term "nucleobase" as used herein is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

Addition of significant mass to one of the 4 nucleobases (dNTPs) in an amplification reaction, or in the primers themselves, will result in a significant difference in mass of the resulting amplification product (significantly greater than 1 Da) arising from ambiguities arising from the G ↔ A combined with C ↔ T event (Table 4). Thus, the same the G ↔ A (-15.994) event combined with 5-Iodo-C ↔ T (-110.900) event would result in a molecular mass difference of 126.894. If the molecular mass of the base composition A₂₇G₃₀ **5-Iodo**-C₂₁T₂₁ (33422.958) is compared with A₂₆G₃₁**5-Iodo**-C₂₂T₂₀, (33549.852) the theoretical molecular mass difference is +126.894. The experimental error of a molecular mass measurement is not significant with regard to this molecular mass difference. Furthermore, the only base composition consistent with a measured molecular mass of the 99-mer nucleic acid is A₂₇G₃₀**5-Iodo-**C₂₁T₂₁. In contrast, the analogous amplification without the mass tag has 18 possible base compositions.

**Table 4: Molecular Masses of Natural Nucleobases and the Mass-Modified Nucleobase 5-Iodo-C and Molecular Mass Differences Resulting from Transitions**

| **Nucleobase** | **Molecular Mass** | **Transition** | **Molecular Mass** |
|---|---|---|---|
| A | 313.058 | A-->T | -9.012 |
| A | 313.058 | A-->C | -24.012 |
| A | 313.058 | A-->5-Iodo-C | 101.888 |
| A | 313.058 | A-->G | 15.994 |
| T | 304.046 | T-->A | 9.012 |
| T | 304.046 | T-->C | -15.000 |
| T | 304.046 | T-->5-Iodo-C | 110.900 |
| T | 304.046 | T-->G | 25.006 |
| C | 289.046 | C-->A | 24.012 |
| C | 289.046 | C-->T | 15.000 |
| C | 289.046 | C-->G | 40.006 |
| 5-Iodo-C | 414.946 | 5-Iodo-C-->A | -101.888 |
| 5-Iodo-C | 414.946 | 5-Iodo-C-->T | -110.900 |
| 5-Iodo-C | 414.946 | 5-Iodo-C-->G | -85.894 |
| G | 329.052 | G-->A | -15.994 |
| G | 329.052 | G-->T | -25.006 |
| G | 329.052 | G-->C | -40.006 |
| G | 329.052 | G-->5-Iodo-C | 85.894 |

Mass spectra of bioagent-identifying amplicons were analyzed independently using a maximum-likelihood processor, such as is widely used in radar signal processing. This processor, referred to as GenX, first makes maximum likelihood estimates of the input to the mass spectrometer for each primer by running matched filters for each base composition aggregate on the input data. This includes the GenX response to a calibrant for each primer.

The algorithm emphasizes performance predictions culminating in probability-of-detection versus probability-of-false-alarm plots for conditions involving complex backgrounds of naturally occurring organisms and environmental contaminants. Matched filters consist of *a priori* expectations of signal values given the set of primers used for each of the bioagents. A genomic sequence database is used to define the mass base count matched filters. The database contains the sequences of known bacterial bioagents and includes threat organisms as well as benign background organisms. The latter is used to estimate and subtract the spectral signature produced by the background organisms. A maximum likelihood detection of known background organisms is implemented using matched filters and a running-sum estimate of the noise covariance. Background signal strengths are estimated and used along with the matched filters to form signatures which are then subtracted. The maximum likelihood process is applied to this "cleaned up" data in a similar manner employing matched filters for the organisms and a running-sum estimate of the noise-covariance for the cleaned up data.

The amplitudes of all base compositions of bioagent-identifying amplicons for each primer are calibrated and a final maximum likelihood amplitude estimate per organism is made based upon the multiple single primer estimates. Models of all system noise are factored into this two-stage maximum likelihood calculation. The processor reports the number of molecules of each base composition contained in the spectra. The quantity of amplification product corresponding to the appropriate primer set is reported as well as the quantities of primers remaining upon completion of the amplification reaction.

Base count blurring can be carried out as follows. "Electronic PCR" can be conducted on nucleotide sequences of the desired bioagents to obtain the different expected base counts that could be obtained for each primer pair. See for example, ncbi.nlm.nih.gov/sutils/e-pcr/; Schuler, Genome Res. 7:541-50, 1997. In one illustrative embodiment, one or more spreadsheets, such as Microsoft Excel workbooks contain a plurality of worksheets. First in this example, there is a worksheet with a name similar to the workbook name; this worksheet contains the raw electronic PCR data. Second, there is a worksheet named "filtered bioagents base count" that contains bioagent name and base count; there is a separate record for each strain after removing sequences that are not identified with a genus and species and removing all sequences for bioagents with less than 10 strains. Third, there is a worksheet, " Sheet 1" that contains the frequency of substitutions, insertions, or deletions for this primer pair. This data is generated by first creating a pivot table from the data in the "filtered bioagents base count" worksheet and then executing an Excel VBA macro. The macro creates a table of differences in base counts for bioagents of the same species; but different strains. One of ordinary skill in the art may understand additional pathways for obtaining similar table differences without undo experimentation.

Application of an exemplary script, involves the user defining a threshold that specifies the fraction of the strains that are represented by the reference set of base counts for each bioagent. The reference set of base counts for each bioagent may contain as many different base counts as are needed to meet or exceed the threshold. The set of reference base counts is defined by taking the most abundant strain's base type composition and adding it to the reference set and then the next most abundant strain's base type composition is added until the threshold is met or exceeded. The current set of data was obtained using a threshold of 55%, which was obtained empirically.

For each base count not included in the reference base count set for that bioagent, the script then proceeds to determine the manner in which the current base count differs from each of the base counts in the reference set. This difference may be represented as a combination of substitutions, Si=Xi, and insertions, Ii=Yi, or deletions, Di=Zi. If there is more than one reference base count, then the reported difference is chosen using rules that aim to minimize the number of changes and, in instances with the same number of changes, minimize the number of insertions or deletions. Therefore, the primary rule is to identify the difference with the minimum sum (Xi+Yi) or (Xi+Zi), *e.g*., one insertion rather than two substitutions. If there are two or more differences with the minimum sum, then the one that will be reported is the one that contains the most substitutions.

Differences between a base count and a reference composition are categorized as one, two, or more substitutions, one, two, or more insertions, one, two, or more deletions, and combinations of substitutions and insertions or deletions. The different classes of nucleobase changes and their probabilities of occurrence have been delineated in U.S. Patent Application Publication No. 2004209260 (U.S. Application Serial No. 10/418,514).

### Example 8: Identification of Adenoviruses

The purpose of this series of experiments was to investigate the spread of adenovirus within a military installation by establishing a temporal relationship between the environmental presence of adenovirus and resulting illness in military personnel, as well as evaluation of asymptomatic carriage. In the military installation, adenovirus has been determined to be the cause of 72% of respiratory illness during the winter. Adenovirus is known to spread rapidly among recruits at the military installation, with outbreaks yielding 50 to 80% attack rates.

Primer pair nos. 615 (SEQ ID NOs: 45:102) and 616 (46:100) were tested in quadruplicate against representative human adenovirus species. Both primer pairs gave rise to amplification products for adenovirus types 4, 7, 8 and 40 from which high quality mass spectral signals were obtained. Adenovirus type 12 was also observed but the mass spectral signals were not as strong. Adenovirus type 1 was observed with weak mass spectral signals. Base compositions were determined from the molecular masses of the amplification products and were found to be in agreement with the base compositions calculated for the bioagent identifying amplicons of adenovirus types 4, 7, 8 and 40 defined by the primer pairs.

Primer pair number 739 (SEQ ID NOs: 30:101), a general survey primer, was found to produce primer dimers indicated by agarose gel electrophoresis. This primer pair was redesigned and tested. The best redesigned primer pair is primer pair number 769 (SEQ ID NOs: 26:121).

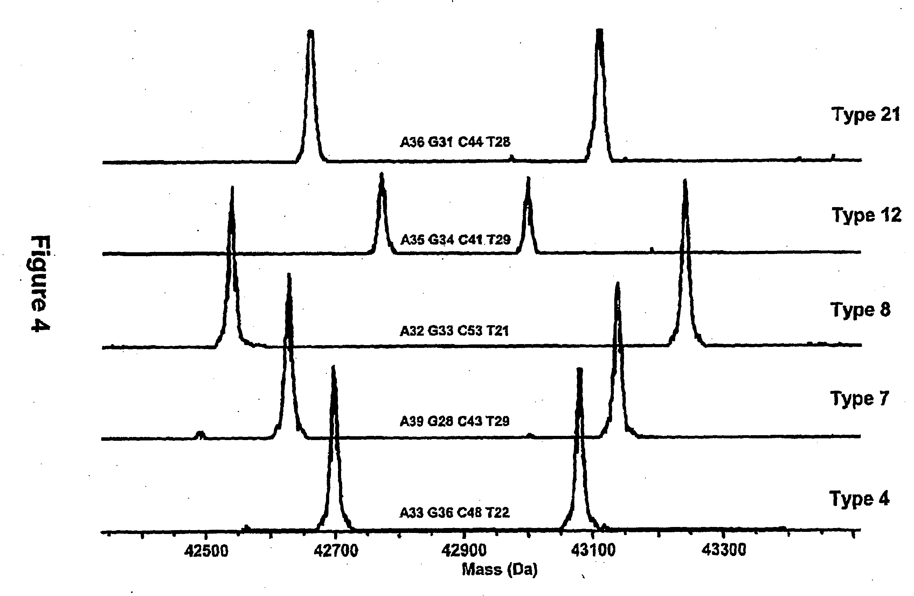

Shown in Figure 4 are mass spectra of amplification products corresponding to adenoviral bioagent identifying amplicons obtained by amplification of samples with primer pair number 943 (SEQ ID NOs: 61:122) according to procedures outlined in Example 3 followed by purification according to Example 4 and analysis of base composition according to examples 5 and 6. It is seen that the single primer pair produced adenoviral bioagent identifying amplicons whose molecular masses can be deconvolved to distinct base compositions for adenovirus types 21, 12, 8, 7, and 4. Thus, each of these adenovirus types can be efficiently distinguished from each other.

A calibration sequence based on the bioagent identifying amplicon produced by primer pair number 943 and reference sequence of adenovirus serotype 4 (GenBank accession no: X84646) was tested for the ability to quantify known amounts of adenovirus serotype 4. It was determined that adenovirus serotype 4 could be detected at levels as low as 15-30 genomes per sample using primer pair number 943 (SEQ ID NOs: 61:122). A representative mass spectrum of amplification products corresponding to adenovirus identifying amplicons and calibration amplicons obtained with primer pair number 943 (SEQ ID NOs: 61:122) is shown in Figure 5.

The limits of detection of adenoviruses in throat swabs for the two primer set comprising primer pair numbers 769 (SEQ ID NOs: 26:121) and 943 (SEQ ID NOs: 61:122) were found to be 15-30 genome copies per sample. Limits of detection in air background and in no background (clean sample) were found to be 30 genome copies per sample.

In another experiment, the ability to identify diverse adenovirus types with primer pair numbers 769 (SEQ ID NOs: 26:121) and 943 (SEQ ID NOs: 61:122) was evaluated by spiking different adenovirus types representing different adenovirus subgroups into a sample and analyzing the sample by obtaining amplification products corresponding to bioagent identifying amplicons of the adenovirus nucleic acid with the primers and analyzing the amplification products by mass spectrometry. The base compositions of the amplification products were calculated from the molecular masses and used to make the adenovirus type assignments. The results are shown in Table 5.

**Table 5: Identification of Adenoviruses From Amplification Products Obtained With Primer Pair Numbers 769 and 943**

| **Sample Number** | **Primer pair** | **Adenovirus Type Spiked Into Sample** | **Adenovirus spike Subgroup** | **Adenovirus Type Identified** | **Base composition Result** | **Adenovirus subgroup Identified** |
|---|---|---|---|---|---|---|
| 1 | 943 | 4 | E | 4 | A20G34C38T20 | E |
| 1 | 769 | 4 | E | 4 | A27G33C39T22 | E |
| 2 | 943 | 3 | B | Type 3 with G->A SNP | A23G31C37T21 | B |
| 2 | 769 | 3 | B | 3 | A27G37C24T33 | B |
| 3 | 943 | 40 | F | 40 | A21G33C39T19 | F |
| 3 | 769 | 40 | F | NA-No Product Expected | NA | NA |
| 4 | 943 | 13 | D | 17, 48 | A18G38C36T20 | D |
| 4 | 769 | 13 | D | 37 | A28G28C44T21 | D |
| 5 | 943 | Mouse | Murine A | Human type 4, Simian type 22, Simian type 25 | A20G34C38T20 | E |
| 5 | 769 | Mouse | Murine A | Murine Adeno 1 | A37G25C33T26 | A |
| 6 | 943 | 6 | C | 1, 2, 5, 4 | A20G33C39T20 | C E |
| 6 | 769 | 6 | C | NA | NA | NA |
| 7 | 943 | 10 | D | 17, 48 | A20G36C38T18 | D |
| 7 | 769 | 10 | D | 9 | A28G29C44T20 | D |
| 8 | 943 | 31 | A | Type 12 with a T->C SNP | A20G32C38T22 | A |
| 8 | 769 | 31 | A | NA-No Product Expected | NA | NA |
| 9 | 943 | 18 | A | Closest Match Type 12 | A21G32C36T23 | A |
| 9 | 769 | 18 | A | Closest Match is Bovine Type A | A31G29C31T30 | NA |
| 10 | 943 | Simian | C1 | Simian type 21, Human 21, 34 | A21G33C37T21 | B |
| 10 | 769 | Simian | C1 | NA-Not Known If Priming Expected | NA | NA |

In another experiment, testing of air samples containing spikes of adenovirus was performed. A total of 35 spiked dry filter unit air samples were provided by a military installation. The adenovirus type 4 spike concentration levels (in plate-forming units - PFU) varied between 5.62 x 10⁵ to 5.62 PFU in the presence and absence of Triton-X100 detergent on the filter surface. Sample collections from the dry filter unit were taken over a period of 12 hours and were analyzed by obtaining amplification products with primer pair numbers 769 (SEQ ID NOs: 26:121) and 943 (SEQ ID NOs: 61:122), and analyzing the products by mass spectrometry. Adenovirus was identified at concentrations as low as 5.62 PFU with no sensitivity to the presence of the detergent and no difference in identification of adenovirus over the 12 hour period.

In another experiment, environmental and clinical surveillance was undertaken within a military installation. A total of 1,600 environmental samples including surface swabs and dry filter unit air samples were taken from various locations within the barracks. A total of 1,700 clinical samples including throat, serum and hand swabs were obtained using standard protocols from symptomatic and asymptomatic military recruits occupying the barracks. All samples were tested for the presence of adenovirus by the method of the present invention. Cultures were grown for 785 of the clinical samples. The results of positive and negative identification of adenovirus in this 785 sample subgroup are shown in Table 6. These results indicate that the method of the present invention is more sensitive for identification of the presence of adenovirus than the standard culture method. In all cases, adenovirus Type 4 was identified. This provides an indication that adenovirus Type 4 is indigenous to the military barracks from which the samples were obtained and also indicates that the method of the present invention is particularly useful for epidemiological investigations of the spread of pathogens in individuals and in the environment.

**Table 6: Comparison of the Present Invention with Standard Culture Methods for Identification of Adenovirus**

| **Test Result by Present Invention** | **Test Result by Standard Culture Method** | **Sample Numbers** |
|---|---|---|
| Positive | Positive | 135 |
| Negative | Positive | 0 |
| Positive | Negative | 78 |
| Negative | Negative | 572 |

The present invention includes any combination of the various species and subgeneric groupings falling within the generic disclosure. This invention therefore includes the generic description of the invention with a proviso or negative limitation removing any subject matter from the genus, regardless of whether or not the excised material is specifically recited herein.

While in accordance with the patent statutes, description of various examples have been provided, the scope of the invention is not to be limited thereto or thereby.

Therefore, it will be appreciated that the scope of this invention is to be defined by the appended claims, rather than by the specific examples which have been presented by way of example.

## Claims

1. A composition comprising (a) an oligonucleotide primer 14 to 35 nucleobases in length having at least 70% sequence identity with SEQ ID NO:26, and (b) an oligonucleotide primer 14 to 35 nucleobases in length having at least 70% sequence identity with SEQ ID NO:121, wherein said primers form a primer pair capable of identifying subgroup and serotype members of the *Adenoviridae* family.

2. The composition of claim 1, wherein said sequence identity of each of said primers is at least 80%.

3. The composition of claim 1, wherein said sequence identity of each of said primers is at least 90%.

4. The composition of claim 1, wherein said sequence identity of each of said primers is at least 95%.

5. The composition of claim 1, wherein:
(i) the oligonucleotide primer of claim 1(a) comprises SEQ ID NO:26, and the oligonucleotide primer of claim 1(b) comprises SEQ ID NO:121; or
(ii) the oligonucleotide primer of claim 1(a) consists of SEQ ID NO:26, and the oligonucleotide primer of claim 1(b) consists of SEQ ID NO:121.

6. The composition of claim 1 wherein either or both of said primers comprises at least one modified nucleobase.

7. The composition of claim 6 wherein said modified nucleobase is 5-propynyluracil or 5- propynylcytosine.

8. The composition of claim 1 wherein either or both of said primers comprises at least one universal nucleobase.

9. The composition of claim 8 wherein said universal nucleobase is inosine.

10. The composition of claim 1 wherein either or both of said primers further comprises a non-templated T residue on the 5 '-end.

11. The composition of claim 1 wherein either or both of said primers comprises at least one non-template tag.

12. The composition of claim 1 wherein either or both of said primers comprises at least one molecular mass modifying tag.

13. A kit comprising the composition of claim 1, further comprising one or more primer pairs wherein each member of said one or more primer pairs is of a length of 14 to 35 nucleobases and has 70% to 100% sequence identity with the corresponding member from the group of primer pairs represented by SEQ ID NOs: 61:122, 38:82, 36:95, 19:93, 54:113, 36:98 and 16:106.

14. The kit of claim 13 further comprising at least one calibration polynucleotide.

15. The kit of claim 13 further comprising at least one anion exchange functional group linked to a magnetic bead.

16. A kit comprising the composition of claim 1, further comprising an oligonucleotide primer 14 to 35 nucleobases in length having at least 70% sequence identity with SEQ ID NO:61 and an oligonucleotide primer 14 to 35 nucleobases in length having at least 70% sequence identity with SEQ ID NO: 122.

17. A method for identification of an adenovirus in a sample comprising: amplifying nucleic acid from said adenovirus using the composition of claim 1 to obtain an amplification product; determining the molecular mass of said amplification product; optionally, determining the base composition of said amplification product from said molecular mass; and comparing said molecular mass or base composition with a plurality of molecular masses or base compositions of known adenovirus identifying amplicons, wherein a match between said molecular mass or base composition and a member of said plurality of molecular masses or base compositions identifies said adenovirus.

18. The method of claim 17 wherein said sample is a biological product.

19. A method of determining the presence or absence of a adenovirus in a sample comprising: amplifying nucleic acid from said sample using the composition of claim 1 to obtain an amplification product; determining the molecular mass of said amplification product; optionally, determining the base composition of said amplification product from said molecular mass; and comparing said molecular mass or base composition of said amplification product with the known molecular masses or base compositions of one or more known adenovirus identifying amplicons, wherein a match between said molecular mass or base composition of said amplification product and the molecular mass or base composition of one or more known adenovirus identifying amplicons indicates the presence of said adenovirus in said sample.

20. The method of claim 19 wherein said sample comprises a biological product.

21. A method for determination of the quantity of an unknown adenovirus in a sample comprising: contacting said sample with the composition of claim 1 and a known quantity of a calibration polynucleotide comprising a calibration sequence; concurrently amplifying nucleic acid from said unknown adenovirus and nucleic acid from said calibration polynucleotide in said sample with the composition of claim 1 to obtain a first amplification product comprising a adenovirus identifying amplicon and a second amplification product comprising a calibration amplicon; determining the molecular mass and abundance for said adenovirus identifying amplicon and said calibration amplicon; and distinguishing said adenovirus identifying amplicon from said calibration amplicon based on molecular mass, wherein comparison of adenovirus identifying amplicon abundance and calibration amplicon abundance indicates the quantity of adenovirus in said sample.

## Patentansprüche

1. Zusammensetzung, umfassend (a) einen Oligonucleotid-Primer mit einer Länge von 14 bis 35 Nucleobasen, der mindestens 70% Sequenzidentität mit SEQ ID NO:26 aufweist, und (b) einen Oligonucleotid-Primer mit einer Länge von 14 bis 35 Nucleobasen, der mindestens 70% Sequenzidentität mit SEQ ID NO:121 aufweist, wobei diese Primer ein Primerpaar bilden, das imstande ist, Untergruppen- und Serotypmitglieder der *Adenoviridae-Familie* zu identifizieren.

2. Zusammensetzung nach Anspruch 1, wobei die Sequenzidentität von jedem dieser Primer mindestens 80% beträgt.

3. Zusammensetzung nach Anspruch 1, wobei die Sequenzidentität von jedem dieser Primer mindestens 90% beträgt.

4. Zusammensetzung nach Anspruch 1, wobei die Sequenzidentität von jedem dieser Primer mindestens 95% beträgt.

5. Zusammensetzung nach Anspruch 1, wobei:
(i) der Oligonucleotid-Primer nach Anspruch 1 (a) SEQ ID NO:26 umfasst, und der Oligonucleotid-Primer nach Anspruch 1 (b) SEQ ID NO:121 umfasst; oder
(ii) der Oligonucleotid-Primer nach Anspruch 1 (a) aus SEQ ID NO:26 besteht, und der Oligonucleotid-Primer nach Anspruch 1 (b) aus SEQ ID NO:121 besteht.

6. Zusammensetzung nach Anspruch 1, wobei einer oder beide der Primer mindestens eine modifizierte Nucleobase umfasst bzw. umfassen.

7. Zusammensetzung nach Anspruch 6, wobei die modifizierte Nucleobase 5-Propinyluracil oder 5-Propinylcytosin ist.

8. Zusammensetzung nach Anspruch 1, wobei einer oder beide der Primer mindestens eine universelle Nucleobase umfasst bzw. umfassen.

9. Zusammensetzung nach Anspruch 8, wobei die universelle Nucleobase Inosin ist.

10. Zusammensetzung nach Anspruch 1, wobei einer oder beide der Primer weiter einen nicht-Templat ("non-templated") T-Rest am 5'-Ende umfasst bzw. umfassen.

11. Zusammensetzung nach Anspruch 1, wobei einer oder beide der Primer mindestens einen nicht-Templat ("non-template") Tag umfasst bzw. umfassen.

12. Zusammensetzung nach Anspruch 1, wobei einer oder beide der Primer mindestens einen Molekülmassen-modifizierenden Tag umfasst bzw. umfassen.

13. Kit, der die Zusammensetzung nach Anspruch 1 umfasst, weiter umfassend ein oder mehrere Primerpaar(e), wobei jedes Mitglied von dem einen oder mehreren Primerpaar(en) eine Länge von 14 bis 35 Nucleobasen aufweist und 70% bis 100% Sequenzidentität mit dem entsprechenden Mitglied aus der Gruppe an Primerpaaren, dargestellt durch SEQ ID Nos: 61:122, 38:82, 36:95, 19:93, 54:113, 36:98 und 16:106, aufweist.

14. Kit nach Anspruch 13, weiter umfassend mindestens ein Kalibrier-Polynucleotid.

15. Kit nach Anspruch 13, weiter umfassend mindestens eine Gruppe mit Anionenaustauschfunktionalität, die an ein Magnetkügelchen gebunden ist.

16. Kit, der die Zusammensetzung nach Anspruch 1 umfasst, weiter umfassend einen Oligonucleotid-Primer mit einer Länge von 14 bis 35 Nucleobasen, der mindestens 70% Sequenzidentität mit SEQ ID NO:61 aufweist, und einen Oligonucleotid-Primer mit einer Länge von 14 bis 35 Nucleobasen, der mindestens 70% Sequenzidentität mit SEQ ID NO:122 aufweist.

17. Verfahren zur Identifizierung eines Adenovirus in einer Probe, umfassend: das Amplifizieren von Nucleinsäure von dem Adenovirus unter Verwendung der Zusammensetzung nach Anspruch 1, um ein Amplifikationsprodukt zu erhalten; das Bestimmen der Molekülmasse des Amplifikationsprodukts; gegebenenfalls, das Bestimmen der Grundzusammensetzung des Amplifikationsprodukts aus dieser Molekülmasse; und das Vergleichen der Molekülmasse oder Grundzusammensetzung mit einer Mehrzahl an Molekülmassen oder Grundzusammensetzungen von bekannten Adenovirus-identifizierenden Ampliconen, wobei eine Übereinstimmung zwischen der Molekülmasse oder der Grundzusammensetzung und einem Mitglied der Mehrzahl an Molekülmassen oder Grundzusammensetzungen das Adenovirus identifiziert.

18. Verfahren nach Anspruch 17, wobei die Probe ein biologisches Produkt ist.

19. Verfahren des Bestimmens der Anwesenheit oder Abwesenheit eines Adenovirus in einer Probe, umfassend: das Amplifizieren von Nucleinsäure von der Probe unter Verwendung der Zusammensetzung nach Anspruch 1, um ein Amplifikationsprodukt zu erhalten; das Bestimmen der Molekülmasse des Amplifikationsprodukts; gegebenenfalls, das Bestimmen der Grundzusammensetzung des Amplifikationsprodukts aus dieser Molekülmasse; und das Vergleichen der Molekülmasse oder Grundzusammensetzung des Amplifikationsprodukts mit den bekannten Molekülmassen oder Grundzusammensetzungen von einem oder mehreren Adenovirus-identifizierenden Amplicon(en), wobei eine Übereinstimmung zwischen der Molekülmasse oder der Grundzusammensetzung des Amplifikationsprodukts und der Molekülmasse oder Grundzusammensetzung von einem oder mehreren Adenovirus-identifizierenden Amplicon(en) die Anwesenheit des Adenovirus in der Probe andeutet.

20. Verfahren nach Anspruch 19, wobei die Probe ein biologisches Produkt umfasst.

21. Verfahren zur Bestimmung der Menge eines unbekannten Adenovirus in einer Probe, umfassend: das Inkontaktbringen der Probe mit der Zusammensetzung nach Anspruch 1 und einer bekannten Menge eines Kalibrier-Polynucleotids, umfassend eine Kalibriersequenz; das gleichzeitige Amplifizieren von Nucleinsäure von dem unbekannten Adenovirus und von Nucleinsäure von dem Kalibrier-Polynucleotid in der Probe mit der Zusammensetzung nach Anspruch 1, um ein erstes Amplifikationsprodukt, umfassend ein Adenovirusidentifizierendes Amplicon, und ein zweites Amplifikationsprodukt, umfassend ein Kalibrieramplicon, zu erhalten; das Bestimmen der Molekülmasse und der Abundanz des Adenovirus-identifizierenden Amplicons und des Kalibrieramplicons; und das Unterscheiden des Adenovirus-identifizierenden Amplicons von dem Kalibrieramplicon, basierend auf der Molekülmasse, wobei ein Vergleich der Abundanz von Adenovirus-identifizierendem Amplicon und der Abundanz von Kalibrieramplicon die Menge von Adenovirus in der Probe andeutet.

## Revendications

1. Composition comprenant (a) une amorce oligonucléotidique de 14 à 35 nucléobases de longueur et présentant au moins 70 % d'identité de séquence avec l'ID SEQ. n° 26, et (b) une amorce oligonucléotidique de 14 à 35 nucléobases de longueur et présentant au moins 70 % d'identité de séquence avec l'ID SEQ. n° 121, dans laquelle lesdites amorces forment une paire d'amorces capable d'identifier des membres de sous-groupe et de sérotype de la famille des *adenoviridae.*

2. Composition de la revendication 1, dans laquelle ladite identité de séquence de chacune desdites amorces est au moins égale à 80 %.

3. Composition de la revendication 1, dans laquelle ladite identité de séquence de chacune desdites amorces est au moins égale à 90 %.

4. Composition de la revendication 1, dans laquelle ladite identité de séquence de chacune desdites amorces est au moins égale à 95 %.

5. Composition de la revendication 1, dans laquelle
(i) l'amorce oligonucléotidique de la revendication 1(a) comprend l'ID SEQ. n° 26 et l'amorce nucléotidique de la revendication 1(b) comprend l'ID SEQ. n° 121 ; ou
(ii) l'amorce oligonucléotidique de la revendication 1(a) se compose de l'ID SEQ. n° 26 et l'amorce nucléotidique de la revendication 1(b) se compose de l'ID SEQ. n° 121.

6. Composition de la revendication 1, dans laquelle l'une desdites amorces ou les deux dites amorces comporte(nt) au moins une nucléobase modifiée.

7. Composition de la revendication 6, dans laquelle ladite nucléobase modifiée est un 5-propynyluracile ou une 5-propynylcytosine.

8. Composition de la revendication 1, dans laquelle l'une desdites amorces ou les deux dites amorces comporte(nt) au moins une nucléobase universelle.

9. Composition de la revendication 8, dans laquelle ladite nucléobase universelle est l'inosine.

10. Composition de la revendication 1, dans laquelle l'une desdites amorces ou les deux dites amorces comporte(nt) en outre un résidu T non matrice sur l'extrémité 5'.

11. Composition de la revendication 1, dans laquelle l'une desdites amorces ou les deux dites amorces comporte(nt) au moins un marqueur non matrice.

12. Composition de la revendication 1, dans laquelle l'une desdites amorces ou les deux dites amorces comporte(nt) au moins un marqueur modifiant la masse moléculaire.

13. Kit comprenant la composition de la revendication 1, et comprenant en outre une ou plusieurs paires d'amorces, dans laquelle chaque membre de la ou desdites paires d'amorces a une longueur de 14 à 35 nucléobases et présente 70 % à 100 % d'identité de séquence avec le membre correspondant du groupe des paires d'amorces représenté par les ID SEQ. n° 61:122, 38:82, 36:95, 19:93, 54:113, 36:98 et 16:106.

14. Kit de la revendication 13, comprenant en outre au moins un polynucléotide d'étalonnage.

15. Kit selon la revendication 13, comprenant en outre au moins un groupe fonctionnel échangeur d'anions relié à une bille magnétique.

16. Kit comprenant la composition de la revendication 1, et comprenant en outre une amorce oligonucléotidique de 14 à 35 nucléobases de longueur et présentant au moins 70 % d'identité de séquence avec l'ID SEQ. n° 61 et une amorce oligonucléotidique de 14 à 35 nucléobases de longueur et présentant au moins 70 % d'identité de séquence avec l'ID SEQ. n° 122.

17. Procédé d'identification d'un adénovirus dans un échantillon, comprenant les étapes consistant à : amplifier l'acide nucléique dudit adénovirus en utilisant la composition de la revendication 1 pour obtenir un produit d'amplification ; déterminer la masse moléculaire dudit produit d'amplification ; en option, déterminer la composition de base dudit produit d'amplification à partir de ladite masse moléculaire ; et comparer ladite masse moléculaire ou ladite composition de base avec plusieurs masses moléculaires ou plusieurs compositions de base d'amplicons identifiant des adénovirus connus, dans lequel une adéquation entre ladite masse moléculaire ou ladite composition de base et un membre desdites plusieurs masses moléculaires ou plusieurs compositions de base identifie ledit adénovirus.

18. Procédé de la revendication 17, dans lequel ledit échantillon est un produit biologique.

19. Procédé de détermination de la présence ou de l'absence d'un adénovirus dans un échantillon, comprenant les étapes consistant à : amplifier l'acide nucléique dudit échantillon en utilisant la composition de la revendication 1 pour obtenir un produit d'amplification ; déterminer la masse moléculaire dudit produit d'amplification ; en option, déterminer la composition de base dudit produit d'amplification à partir de ladite masse moléculaire ; et comparer ladite masse moléculaire ou ladite composition de base dudit produit d'amplification avec les masses moléculaires ou les compositions de base connues d'un ou de plusieurs amplicons identifiant des adénovirus connus, dans lequel une adéquation centre ladite masse moléculaire ou ladite composition de base dudit produit d'amplification et la masse moléculaire ou la composition de base d'un ou de plusieurs amplicons identifiant des adénovirus connus indique la présence dudit adénovirus dans ledit échantillon.

20. Procédé de la revendication 19, dans lequel ledit échantillon comprend un produit biologique.

21. Procédé de détermination de la quantité d'un adénovirus inconnu dans un échantillon, comprenant les étapes consistant à : mettre en contact ledit échantillon avec la composition de la revendication 1 et une quantité connue d'un polynucléotide d'étalonnage comprenant une séquence d'étalonnage ; amplifier concurremment l'acide nucléique dudit adénovirus inconnu et l'acide nucléique dudit polynucléotide d'étalonnage dans ledit échantillon avec la composition de la revendication 1 pour obtenir un premier produit d'amplification comprenant un amplicon identifiant l'adénovirus et un second produit d'amplification comprenant un amplicon d'étalonnage ; déterminer la masse moléculaire et l'abondance dudit amplicon identifiant l'adénovirus et dudit amplicon d'étalonnage ; et effectuer une distinction entre ledit amplicon identifiant l'adénovirus et ledit amplicon d'étalonnage sur la base de la masse moléculaire, où une comparaison de l'abondance dudit amplicon identifiant l'adénovirus et de l'abondance de l'amplicon d'étalonnage indique la quantité d'adénovirus dans ledit échantillon.


### SEQUENCE LISTING

<110> Hall, Thomas A.
   Sampath, Rangarajan
   Blyn, Lawrence
<120> COMPOSITIONS FOR USE IN IDENTIFICATION OF ADENOVIRUSES
<130> DIBIS-0073WO
<150> 60/671,003
   <151> 2005-04-13
<160> 136
<170> FastSEQ for Windows version 4.0
<210> 1
   <211> 19
   <212> DNA
   <213> Artificial Sequence
<220>
   <223> Primer
<400> 1
   acagacactt accagggtg 19
<210> 2
   <211> 20
   <212> DNA
   <213> Artificial Sequence
<220>
   <223> Primer
<400> 2
   agacccaatt acattggctt 20
<210> 3
   <211> 18
   <212> DNA
   <213> Artificial Sequence
<220>
   <223> Primer
<400> 3
   agtccgggtc tggtgcag 18
<210> 4
   <211> 18
   <212> DNA
   <213> Artificial Sequence
<220>
   <223> Primer
<400> 4
   atggccaccc catcgatg 18
<210> 5
   <211> 20
   <212> DNA
   <213> Artificial Sequence
<220>
   <223> Primer
<400> 5
   atgtactaca acagtactgg 20
<210> 6
   <211> 24
   <212> DNA
   <213> Artificial Sequence
<220>
   <223> Primer
<400> 6
   caatccgttc tggttccgga tgaa 24
<210> 7
   <211> 21
   <212> DNA
   <213> Artificial Sequence
<220>
   <223> Primer
<400> 7
   cggatccaag ctaatctttg g 21
<210> 8
   <211> 20
   <212> DNA
   <213> Artificial Sequence
<220>
   <223> Primer
<400> 8
   gatatggcca ccccatcgat 20
<210> 9
   <211> 21
   <212> DNA
   <213> Artificial Sequence
<220>
   <223> Primer
<400> 9
   ggaaagacat tactgcagac a 21
<210> 10
   <211> 21
   <212> DNA
   <213> Artificial Sequence
<220>
   <223> Primer
<400> 10
   ggaatttttt gatggtagag a 21
<210> 11
   <211> 20
   <212> DNA
   <213> Artificial Sequence
<220>
   <223> Primer
<400> 11
   gggcttatgt actacaacag 20
<210> 12
   <211> 23
   <212> DNA
   <213> Artificial Sequence
<220>
   <223> Primer
<400> 12
   ggtcgttatg tgcctttcca cat 23
<210> 13
   <211> 30
   <212> DNA
   <213> Artificial Sequence
<220>
   <223> Primer
<400> 13
   taaaaaggtg tcaatcatgt ttgactcctc 30
<210> 14
   <211> 24
   <212> DNA
   <213> Artificial Sequence
<220>
   <223> Primer
<400> 14
   taacacctac gagtacatga acgg 24
<210> 15
   <211> 24
   <212> DNA
   <213> Artificial Sequence
<220>
   <223> Primer
<400> 15
   taacagaccc aattacattg gctt 24
<210> 16
   <211> 17
   <212> DNA
   <213> Artificial Sequence
<220>
   <223> Primer
<400> 16
   taagcgcccg ataccca 17
<210> 17
   <211> 20
   <212> DNA
   <213> Artificial Sequence
<220>
   <223> Primer
<400> 17
   tacagacact taccagggtg 20
<210> 18
   <211> 21
   <212> DNA
   <213> Artificial Sequence
<220>
   <223> Primer
<400> 18
   tacctttcaa cctgaacctc a 21
<210> 19
   <211> 26
   <212> DNA
   <213> Artificial Sequence
<220>
   <223> Primer
<400> 19
   tacgactaca tgaacaagcg agtggt 26
<210> 20
   <211> 25
   <212> DNA
   <213> Artificial Sequence
<220>
   <223> Primer
<400> 20
   tactacaaca gcactggcaa tatgg 25
<210> 21
   <211> 27
   <212> DNA
   <213> Artificial Sequence
<220>
   <223> Primer
<400> 21
   tagaggaaaa atatggaggc agagctc 27
<210> 22
   <211> 20
   <212> DNA
   <213> Artificial Sequence
<220>
   <223> Primer
<400> 22
   tagatctggc tttctttgac 20
<210> 23
   <211> 19
   <212> DNA
   <213> Artificial Sequence
<220>
   <223> Primer
<400> 23
   tcaacatggg tgtgctggc 19
<210> 24
   <211> 22
   <212> DNA
   <213> Artificial Sequence
<220>
   <223> Primer
<400> 24
   tcaatgggca tacatgcaca tc 22
<210> 25
   <211> 24
   <212> DNA
   <213> Artificial Sequence
<220>
   <223> Primer
<400> 25
   tcaccaacac ctacgagtac atga 24
<210> 26
   <211> 25
   <212> DNA
   <213> Artificial Sequence
<220>
   <223> Primer
<400> 26
   tcaccaacac ctacgagtac atgaa 25
<210> 27
   <211> 22
   <212> DNA
   <213> Artificial Sequence
<220>
   <223> Primer
<400> 27
   tcactaaaga caaaggtctt cc 22
<210> 28
   <211> 20
   <212> DNA
   <213> Artificial Sequence
<220>
   <223> Primer
<400> 28
   tcagccagag ccgcaagtag 20
<210> 29
   <211> 20
   <212> DNA
   <213> Artificial Sequence
<220>
   <223> Primer
<400> 29
   tcatgctacg ggtcttttgc 20
<210> 30
   <211> 29
   <212> DNA
   <213> Artificial Sequence
<220>
   <223> Primer
<400> 30
   tccaacacca acacctacga gtacatgaa 29
<210> 31
   <211> 24
   <212> DNA
   <213> Artificial Sequence
<220>
   <223> Primer
<400> 31
   tcccaatggg catacatgca catc 24
<210> 32
   <211> 30
   <212> DNA
   <213> Artificial Sequence
<220>
   <223> Primer
<400> 32
   tccctacttt gtatactctg gaaccattcc 30
<210> 33
   <211> 27
   <212> DNA
   <213> Artificial Sequence
<220>
   <223> Primer
<400> 33
   tcctaaatac tgttttcctc tggatgg 27
<210> 34
   <211> 27
   <212> DNA
   <213> Artificial Sequence
<220>
   <223> Primer
<400> 34
   tcctaaatac tgttttcctc tggatgg 27
<210> 35
   <211> 28
   <212> DNA
   <213> Artificial Sequence
<220>
   <223> Primer
<400> 35
   tcctgtggag aaatttcctg tactccaa 28
<210> 36
   <211> 25
   <212> DNA
   <213> Artificial Sequence
<220>
   <223> Primer
<400> 36
   tcgccaagcc taccaacaaa gaagg 25
<210> 37
   <211> 23
   <212> DNA
   <213> Artificial Sequence
<220>
   <223> Primer
<400> 37
   tctacctctg cgctgcaaac atg 23
<210> 38
   <211> 25
   <212> DNA
   <213> Artificial Sequence
<220>
   <223> Primer
<400> 38
   tcttatcagc cagagccgca agtag 25
<210> 39
   <211> 25
   <212> DNA
   <213> Artificial Sequence
<220>
   <223> Primer
<400> 39
   tcttatgtac tacaacagca ctgga 25
<210> 40
   <211> 25
   <212> DNA
   <213> Artificial Sequence
<220>
   <223> Primer
<400> 40
   tgaatgctgt ggttgacttg caaga 25
<210> 41
   <211> 29
   <212> DNA
   <213> Artificial Sequence
<220>
   <223> Primer
<400> 41
   tgaatgctgt ggttgacttg caagacaga 29
<210> 42
   <211> 19
   <212> DNA
   <213> Artificial Sequence
<220>
   <223> Primer
<400> 42
   tgacagacac ttaccaggg 19
<210> 43
   <211> 25
   <212> DNA
   <213> Artificial Sequence
<220>
   <223> Primer
<400> 43
   tgatatagat ctggctttct ttgac 25
<210> 44
   <211> 27
   <212> DNA
   <213> Artificial Sequence
<220>
   <223> Primer
<400> 44
   tgatatcact aaagacaaag gtcttcc 27
<210> 45
   <211> 21
   <212> DNA
   <213> Artificial Sequence
<220>
   <223> Primer
<400> 45
   tgatatggcc accccatcga t 21
<210> 46
   <211> 22
   <212> DNA
   <213> Artificial Sequence
<220>
   <223> Primer
<400> 46
   tgatatggcc accccatcga tg 22
<210> 47
   <211> 24
   <212> DNA
   <213> Artificial Sequence
<220>
   <223> Primer
<400> 47
   tgcaagatgg ccaccccatc gatg 24
<210> 48
   <211> 22
   <212> DNA
   <213> Artificial Sequence
<220>
   <223> Primer
<400> 48
   tgccatgcta cgggtctttt gc 22
<210> 49
   <211> 26
   <212> DNA
   <213> Artificial Sequence
<220>
   <223> Primer
<400> 49
   tgccccaatg ggcatacatg cacatc 26
<210> 50
   <211> 25
   <212> DNA
   <213> Artificial Sequence
<220>
   <223> Primer
<400> 50
   tgcttatgta ctacaacagc actgg 25
<210> 51
   <211> 25
   <212> DNA
   <213> Artificial Sequence
<220>
   <223> Primer
<400> 51
   tgcttatgta ctacaacagc actgg 25
<210> 52
   <211> 28
   <212> DNA
   <213> Artificial Sequence
<220>
   <223> Primer
<400> 52
   tggaacttca ggaaggatgt taacatgg 28
<210> 53
   <211> 22
   <212> DNA
   <213> Artificial Sequence
<220>
   <223> Primer
<400> 53
   tggaattttt cgatggtaga ga 22
<210> 54
   <211> 21
   <212> DNA
   <213> Artificial Sequence
<220>
   <223> Primer
<400> 54
   tggcaacatg ggtgtgctgg c 21
<210> 55
   <211> 25
   <212> DNA
   <213> Artificial Sequence
<220>
   <223> Primer
<400> 55
   tgggattgac agatacttac caggg 25
<210> 56
   <211> 28
   <212> DNA
   <213> Artificial Sequence
<220>
   <223> Primer
<400> 56
   tggtgaaatc ctgttatggt tcattcgc 28
<210> 57
   <211> 24
   <212> DNA
   <213> Artificial Sequence
<220>
   <223> Primer
<400> 57
   tgtcgggctt atgtactaca acag 24
<210> 58
   <211> 24
   <212> DNA
   <213> Artificial Sequence
<220>
   <223> Primer
<400> 58
   tgtgcctttc cacatacagg tgcc 24
<210> 59
   <211> 29
   <212> DNA
   <213> Artificial Sequence
<220>
   <223> Primer
<400> 59
   ttatgatata gagctggctt tctttgaca 29
<210> 60
   <211> 26
   <212> DNA
   <213> Artificial Sequence
<220>
   <223> Primer
<400> 60
   ttccatgccc aacagaccca actaca 26
<210> 61
   <211> 24
   <212> DNA
   <213> Artificial Sequence
<220>
   <223> Primer
<400> 61
   ttgcaagatg gccaccccat cgat 24
<210> 62
   <211> 23
   <212> DNA
   <213> Artificial Sequence
<220>
   <223> Primer
<400> 62
   ttggaaagac attactgcag aca 23
<210> 63
   <211> 28
   <212> DNA
   <213> Artificial Sequence
<220>
   <223> Primer
<400> 63
   tttcaagtgc ctcagaaatt ctttgctg 28
<210> 64
   <211> 20
   <212> DNA
   <213> Artificial Sequence
<220>
   <223> Primer
<400> 64
   actgtggtgt catctttgtc 20
<210> 65
   <211> 21
   <212> DNA
   <213> Artificial Sequence
<220>
   <223> Primer
<400> 65
   atatgagtat ctggagtctg c 21
<210> 66
   <211> 20
   <212> DNA
   <213> Artificial Sequence
<220>
   <223> Primer
<400> 66
   caagtcaacc acagcattca 20
<210> 67
   <211> 20
   <212> DNA
   <213> Artificial Sequence
<220>
   <223> Primer
<400> 67
   ccaacttgag gctctggctg 20
<210> 68
   <211> 20
   <212> DNA
   <213> Artificial Sequence
<220>
   <223> Primer
<400> 68
   ccagtgctgt tgtagtacat 20
<210> 69
   <211> 17
   <212> DNA
   <213> Artificial Sequence
<220>
   <223> Primer
<400> 69
   cggtcggtgg tcacatc 17
<210> 70
   <211> 20
   <212> DNA
   <213> Artificial Sequence
<220>
   <223> Primer
<400> 70
   ccagtgctgt tgtagtacat 20
<210> 71
   <211> 19
   <212> DNA
   <213> Artificial Sequence
<220>
   <223> Primer
<400> 71
   ctgtccggcg atgtgcatg 19
<210> 72
   <211> 24
   <212> DNA
   <213> Artificial Sequence
<220>
   <223> Primer
<400> 72
   cttgccggtc gttcaaagag gtag 24
<210> 73
   <211> 21
   <212> DNA
   <213> Artificial Sequence
<220>
   <223> Primer
<400> 73
   ggcttcgccg tctgtaattt c 21
<210> 74
   <211> 18
   <212> DNA
   <213> Artificial Sequence
<220>
   <223> Primer
<400> 74
   gggcgaactg caccagac 18
<210> 75
   <211> 24
   <212> DNA
   <213> Artificial Sequence
<220>
   <223> Primer
<400> 75
   ggtatgtact cataggtgtt ggtg 24
<210> 76
   <211> 19
   <212> DNA
   <213> Artificial sequence
<220>
   <223> Primer
<400> 76
   taaagcacaa tttcaggcg 19
<210> 77
   <211> 30
   <212> DNA
   <213> Artificial Sequence
<220>
   <223> Primer
<400> 77
   taagtgaaca ttttctgcgt acattacaat 30
<210> 78
   <211> 29
   <212> DNA
   <213> Artificial Sequence
<220>
   <223> Primer
<400> 78
   taagtgaaca ttttctgcta cattacaat 29
<210> 79
   <211> 28
   <212> DNA
   <213> Artificial Sequence
<220>
   <223> Primer
<400> 79
   tactatcaaa gaaagccagg tctatatc 28
<210> 80
   <211> 21
   <212> DNA
   <213> Artificial Sequence
<220>
   <223> Primer
<400> 80
   tactgtggtg tcatctttgt c 21
<210> 81
   <211> 24
   <212> DNA
   <213> Artificial Sequence
<220>
   <223> Primer
<400> 81
   tagacccgga ctcaggtact ccga 24
<210> 82
   <211> 21
   <212> DNA
   <213> Artificial Sequence
<220>
   <223> Primer
<400> 82
   tagcgtagga gccatagcac g 21
<210> 83
   <211> 25
   <212> DNA
   <213> Artificial sequence
<220>
   <223> Primer
<400> 83
   tagctgtcca cagcctgatt ccaca 25
<210> 84
   <211> 25
   <212> DNA
   <213> Artificial Sequence
<220>
   <223> Primer
<400> 84
   tagctgtcca ccgcctgatt ccaca 25
<210> 85
   <211> 33
   <212> DNA
   <213> Artificial Sequence
<220>
   <223> Primer
<400> 85
   taggaccatg ttcacatcct tgctgaagtt cca 33
<210> 86
   <211> 20
   <212> DNA
   <213> Artificial Sequence
<220>
   <223> Primer
<400> 86
   taggcggtgt tgtgggccat 20
<210> 87
   <211> 22
   <212> DNA
   <213> Artificial Sequence
<220>
   <223> Primer
<400> 87
   tatatgagta tctggagtct gc 22
<210> 88
   <211> 20
   <212> DNA
   <213> Artificial Sequence
<220>
   <223> Primer
<400> 88
   tcaacttgag gctctggctg 20
<210> 89
   <211> 30
   <212> DNA
   <213> Artificial Sequence
<220>
   <223> Primer
<400> 89
   tcaacttgtt gcctatggct atttcattag 30
<210> 90
   <211> 26
   <212> DNA
   <213> Artificial Sequence
<220>
   <223> Primer
<400> 90
   tcaccttctt tgttggtagg cttggc 26
<210> 91
   <211> 27
   <212> DNA
   <213> Artificial Sequence
<220>
   <223> Primer
<400> 91
   tcagcccaat ttcgcgaagg aatagaa 27
<210> 92
   <211> 31
   <212> DNA
   <213> Artificial Sequence
<220>
   <223> Primer
<400> 92
   tccacccatc aaaaaattcc atgtcaatat c 31
<210> 93
   <211> 21
   <212> DNA
   <213> Artificial Sequence
<220>
   <223> Primer
<400> 93
   tccagcattg cggtggtggt t 21
<210> 94
   <211> 27
   <212> DNA
   <213> Artificial Sequence
<220>
   <223> Primer
<400> 94
   tccatcgaaa aattccatgt caatatc 27
<210> 95
   <211> 29
   <212> DNA
   <213> Artificial Sequence
<220>
   <223> Primer
<400> 95
   tccatgttca catcctttct gaagttcca 29
<210> 96
   <211> 27
   <212> DNA
   <213> Artificial Sequence
<220>
   <223> Primer
<400> 96
   tccatcgaaa aattccatgt caatatc 27
<210> 97
   <211> 28
   <212> DNA
   <213> Artificial Sequence
<220>
   <223> Primer
<400> 97
   tccccatcca cagaacgctt tatttcaa 28
<210> 98
   <211> 28
   <212> DNA
   <213> Artificial Sequence
<220>
   <223> Primer
<400> 98
   tccgtagttt tgctgtcaaa gaaagcca 28
<210> 99
   <211> 25
   <212> DNA
   <213> Artificial Sequence
<220>
   <223> Primer
<400> 99
   tcctttctga agttccactc atagg 25
<210> 100
   <211> 16
   <212> DNA
   <213> Artificial Sequence
<220>
   <223> Primer
<400> 100
   tcgcgggcga actgca 16
<210> 101
   <211> 19
   <212> DNA
   <213> Artificial Sequence
<220>
   <223> Primer
<400> 101
   tcgcgttgcg gtggtggtt 19
<210> 102
   <211> 18
   <212> DNA
   <213> Artificial Sequence
<220>
   <223> Primer
<400> 102
   tcgggcgaac tgcaccag 18
<210> 103
   <211> 23
   <212> DNA
   <213> Artificial Sequence
<220>
   <223> Primer
<400> 103
   tcggtatttc tgtcttgcaa gtc 23
<210> 104
   <211> 21
   <212> DNA
   <213> Artificial Sequence
<220>
   <223> Primer
<400> 104
   tctgtcttgc aagtcaacca c 21
<210> 105
   <211> 21
   <212> DNA
   <213> Artificial Sequence
<220>
   <223> Primer
<400> 105
   tgaaccgtag catggtttca t 21
<210> 106
   <211> 25
   <212> DNA
   <213> Artificial Sequence
<220>
   <223> Primer
<400> 106
   tgaagttgtc cctaaaacca atgta 25
<210> 107
   <211> 23
   <212> DNA
   <213> Artificial Sequence
<220>
   <223> Primer
<400> 107
   tgaccagcca gcacacccat gtt 23
<210> 108
   <211> 22
   <212> DNA
   <213> Artificial Sequence
<220>
   <223> Primer
<400> 108
   tgatttccat ggcaaaagga tt 22
<210> 109
   <211> 22
   <212> DNA
   <213> Artificial Sequence
<220>
   <223> Primer
<400> 109
   tgatttccat ggcaaaagga tt 22
<210> 110
   <211> 22
   <212> DNA
   <213> Artificial Sequence
<220>
   <223> Primer
<400> 110
   tgcaagtcaa ccacagcatt ca 22
<210> 111
   <211> 22
   <212> DNA
   <213> Artificial Sequence
<220>
   <223> Primer
<400> 111
   tgccagtgct gttgtagtac at 22
<210> 112
   <211> 20
   <212> DNA
   <213> Artificial Sequence
<220>
   <223> Primer
<400> 112
   tgcgtaggag ccatagcacg 20
<210> 113
   <211> 26
   <212> DNA
   <213> Artificial Sequence
<220>
   <223> Primer
<400> 113
   tgctcggtat ttctgtcttg caagtc 26
<210> 114
   <211> 21
   <212> DNA
   <213> Artificial Sequence
<220>
   <223> Primer
<400> 114
   tgcttcgccg tctgtaattt c 21
<210> 115
   <211> 27
   <212> DNA
   <213> Artificial Sequence
<220>
   <223> Primer
<400> 115
   tggaggagtc aaacatgatt gacacct 27
<210> 116
   <211> 18
   <212> DNA
   <213> Artificial Sequence
<220>
   <223> Primer
<400> 116
   tggcgcgggc gaactgca 18
<210> 117
   <211> 19
   <212> DNA
   <213> Artificial Sequence
<220>
   <223> Primer
<400> 117
   tgggagccac cactcgctt 19
<210> 118
   <211> 17
   <212> DNA
   <213> Artificial Sequence
<220>
   <223> Primer
<400> 118
   tgggcgaact gcaccag 17
<210> 119
   <211> 19
   <212> DNA
   <213> Artificial Sequence
<220>
   <223> Primer
<400> 119
   tgggcgaact gcaccagac 19
<210> 120
   <211> 25
   <212> DNA
   <213> Artificial Sequence
<220>
   <223> Primer
<400> 120
   tggttgaagg gatttacgtt gtcca 25
<210> 121
   <211> 26
   <212> DNA
   <213> Artificial Sequence
<220>
   <223> Primer
<400> 121
   tggttgaagg gatttacgtt gtccat 26
<210> 122
   <211> 20
   <212> DNA
   <213> Artificial Sequence
<220>
   <223> Primer
<400> 122
   tgtggcgcgg gcgaactgca 20
<210> 123
   <211> 20
   <212> DNA
   <213> Artificial sequence
<220>
   <223> Primer
<400> 123
   tgttgcgcgg gcgaactgca 20
<210> 124
   <211> 25
   <212> DNA
   <213> Artificial sequence
<220>
   <223> Primer
<400> 124
   tgtttctgtc ttgcaagtca accac 25
<210> 125
   <211> 22
   <212> DNA
   <213> Artificial Sequence
<220>
   <223> Primer
<400> 125
   tgcaagtcaa ccacagcatt ca 22
<210> 126
   <211> 20
   <212> DNA
   <213> Artificial Sequence
<220>
   <223> Primer
<400> 126
   ttaaagcaca atttcaggcg 20
<210> 127
   <211> 20
   <212> DNA
   <213> Artificial Sequence
<220>
   <223> Primer
<400> 127
   ttgcggtggt ggttgaaggg 20
<210> 128
   <211> 26
   <212> DNA
   <213> Artificial Sequence
<220>
   <223> Primer
<400> 128
   ttgttcacat ccttgctgaa gttcca 26
<210> 129
   <211> 2918
   <212> DNA
   <213> Adenovirus
<220>
<400> 129
   tcatcaccac gccgccgcag cagaggagaa aggaagaggt cgcgcgccga ggctgagttg 60
   ctttcaagat ggccacccca tcgatgatgc cccaatgggc atacatgcac atcgccggac 120
   aggatgcttc ggagtacctg agtccgggtc tggtgcagtt cgcccgtgca acagacacct 180
   acttcagtat ggggaacaaa tttagaaacc ccacagtggc gcccacccac gatgtgacca 240
   ccgaccgtag ccagcgactg atgctgcgct tcgtacccgt tgaccgggaa gacaatacct 300
   actcttacaa agttcgctac acgctggctg taggcgacaa cagagtgctt gacatggcca 360
   gcacattctt tgacattcgg ggggtgcttg atagaggtcc tagctttaag ccatattctg 420
   gcacagctta caattcactg gctcctaagg gcgcgcccaa tacatctcag tggatagtta 480
   cagcaggaga agaaagagca gtaactacca ccacaaacac atttggcatt gcttccatga 540
   agggagacaa tattactaaa gaaggtttag aaattgggaa agacattact gcagacaaca 600
   agcccattta tgccgataaa acatatcagc cagagcctca agttggagaa gaatcatgga 660
   ctgatactga tggaacaaat gaaaagtttg gaggtagagc tcttaaacca gctaccaaaa 720
   tgaagccatg ctacgggtct tttgcaagac ctacaaatat aaaagggggc caagctaaaa 780
   acagaaaagt aaaaccaacc gaaggagatg ttgagactga ggagccagat attgatatgg 840
   aattttttga tggtagagaa gctgctgacg ctttttcgcc tgaaattgtg ctttacacgg 900
   aaaatgtcaa tttagaaact ccagacagcc atgtagttta caagccagga acttctgatg 960
   ataactctca tgcaaatttg ggtcaacaag ccatgcctaa cagacccaat tacattggct 1020
   tcagggataa ctttgtaggc ctaatgtact acaacagtac tggaaatatg ggagttttgg 1080
   ctggccaagc atcacaactg aatgcagtgg ttgacttgca ggacagaaac actgaactgt 1140
   catatcagct tttgcttgat tctctgggag acagaaccag atacttcagt atgtggaacc 1200
   aggctgtgga cagttatgat cccgatgttc gcattattga aaatcacggc atcgaggatg 1260
   aactgcctaa ttactgtttt cctctggatg gcataggacc agcgaaaaca tatcaaggca 1320
   ttaaatctaa agacaatgga tgggaaaaag atgataatgt ttctaaatct aatgaaatag 1380
   ccataggcaa caaccaggct atggaaatta atatccaagc taacctttgg agaagttttc 1440
   tgtactccaa tgtggctttg taccttccag atgtttacaa gtacacgcca actaacatta 1500
   ctctgcccgc caacaccaac acctatgagt acatgaacgg gcgagtggta tccccatctc 1560
   tggttgattc atacatcaac attggcgcca ggtggtctct tgacccaatg gacaatgtga 1620
   atccattcaa ccaccaccgc aatgctggtc tgcgctaccg gtccatgctt ctgggcaatg 1680
   gtcgttatgt gcctttccac atacaagtgc ctcaaaaatt ctttgctgtc aagaacctac 1740
   ttcttctacc tggctcctac acctatgagt ggaacttcag aaaggatgtg aacatggtcc 1800
   tgcaaagttc ccttggaaat gacctcagaa cagatggtgc taccataagt ttcaccagca 1860
   tcaatctgta tgccaccttc ttccccatgg ctcacaacac agcttccacc cttgaagcca 1920
   tgctgcgcaa cgataccaat gatcagtcat tcaacgacta cctctctgca gctaacatgc 1980
   tttaccccat ccctgccaat gcaaccaaca ttccaatttc catcccatct cgcaactggg 2040
   cagccttcag gggctggtcc ttcactaggc tcaaaaccaa ggagactcca tctcttggat 2100
   cagggttcga tccctacttc gtatattctg gatctattcc ctacctggat ggcacctttt 2160
   accttaacca cactttcaag aaggtctcca tcatgtttga ctcctcagtc agctggcctg 2220
   gcaatgacag gctgttgagc ccaaatgagt ttgaaatcaa gcgcactgtg gacggggaag 2280
   ggtacaatgt ggcccaatgt aacatgacca aagactggtt cctggttcag atgcttgcca 2340
   actacaacat tggctaccag ggcttttaca tccctgaggg atacaaggat cgcatgtact 2400
   cctttttcag aaacttccag cctatgagca ggcaggtggt tgatgaggtt aattacactg 2460
   actacaaagc cgtcacctta ccataccaac acaacaactc tggctttgta gggtatcttg 2520
   cacctactat gagacaaggg gaaccttacc cagccaatta tccatacccg ctcatcggaa 2580
   ctactgccgt taagagtgtc acccagaaaa agttcctgtg tgacaggacc atgtggcgca 2640
   ttcccttctc cagcaacttc atgtccatgg gggcccttac cgacctggga cagaacatgc 2700
   tctatgccaa ctcagcccat gcgctggaca tgacttttga ggtggatccc atggatgagc 2760
   ccaccctgct ttatcttctt ttcgaagtct tcgacgtggt cagagtgcac cagccacacc 2820
   gcggcgtcat cgaggccgtc tacctgcgca caccgttctc ggccggcaac gccaccacat 2880
   aagaagcctc ttgcttcttg caagcagcag ctgcagcc                         2918
<210> 130
   <211> 2811
   <212> DNA
   <213>Adenovirus
<220>
<400> 130
   atggccaccc catcgatgct gccccagtgg gcgtacatgc acatcgccgg acaggacgct 60
   tcggagtacc tgagtccggg tctggtgcag ttcgcccggg ccacagacac ctacttcagt 120
   ctggggaaca agtttaggaa ccccacggtg gcgcctaccc acgatgtgac caccgaccgc 180
   agccagcggc tgagcctgcg ctttgtgccc gtggaccggg aggacaacac ctactcgtac 240
   aaagtgcgct acacgctggc cgtgggcgac aaccgcgtgc tggacatggc cagcacctac 300
   tttgacatcc gcggcgtgct ggatcggggc cctagcttca aaccctactc cggcactgcc 360
   tacaacagcc tggctcccaa gggagcgccc aacacctgcc agtggaagga ttctgacagc 420
   aaaatgcata cctttggggt agctgccatg cccggtgtta ctgggaaaaa gatagaagct 480
   gatgggctgc ctattggaat agattcaact tctggaactg acacagtaat ttatgctgat 540
   aaaactttcc aaccagaacc acaagttgga aatgacagtt gggttgacac caatggtgca 600
   gaggaaaaat atggaggcag agctctaaag gacactacaa aaatgaaacc ctgttatggt 660
   tcattcgcca agcctaccaa caaagaaggt ggtcaggcta acttaaaaga ttcagaaccc 720
   gccgccacca ctcctaacta tgatatagac ctggctttct ttgacagcaa aactattgtt 780
   gctaactacg atccagatat tgtaatgtac acagaaaatg ttgacttgca gactccagat 840
   actcatattg tatacaaacc tggaacagag gacaccagct ctgaatccaa tttgggtcag 900
   caggccatgc ctaacagacc caactacatt ggcttcagag acaattttat cgggctcatg 960
   tactacaaca gcactggcaa tatgggggtg ctggccggtc aggcctctca gctgaatgct 1020
   gtggttgact tgcaagacag aaacactgaa ctgtcctacc agctcttgct tgactctctg 1080
   ggtgacagaa cccggtattt cagtatgtgg aatcaggcgg tggacagcta tgatcctgat 1140
   gtgcgcatta ttgaaaacca tggtgtggag gatgaattgc caaactattg ctttccgttg 1200
   aatggtgtgg gattgacaga cacttaccag ggtgttaaag ttaaaacaga tgcaggttct 1260
   gaaaagtggg acaaagatga caccacagtt agtaatgcta atgaaatcca tgtaggcaat 1320
   ccttttgcca tggaaatcaa catccaagcc aacctgtgga ggaacttcct ctatgccaat 1380
   gttggcctct atttgcctga taaatacaaa tacacaccgg ccaacatcac cctgcccacc 1440
   aacaccaaca cctacgagta catgaacggc cgggtggtgg cgccctcgct ggtggacgcc 1500
   tacattaaca ttggggcgcg ctggtcgctg gaccccatgg acaacgtaaa tcccttcaac 1560
   caccaccgca atgcgggctt gccgtaccgc tccatgctcc tgggcaacgg gcgctacgtg 1620
   ccattccaca tccaggttcc tcagaaattt tttcccatta agagcctcct gctcctgccc 1680
   gggtcctaca cctacgagtg gaacttccgc aaggacgtca acatgatcct gcagagttcc 1740
   cttggcaacg acctgcgcac agacggggcc tccatcacct tcaccagcat taacctctac 1800
   gccaccttct tccccatggc gcacaacacc gcctccacgc ttgaggccat gctgcgcaac 1860
   gacaccaatg accaatcctt caacgactac ctctcggcgg ccaacatgct ctatcccatc 1920
   ccggccaacg ccaccaacgt gcccatctcc atcccctcgc gcaactgggc ggcctttcgc 1980
   ggctggtcct tcacgcgact caagaccaaa gagacgccct cgctgggctc cgggttcgac 2040
   ccctacttcg tctactcggg ctccatcccc tacctcgacg gcaccttcta cctcaaccac 2100
   accttcaaga aggtctccat caccttcgac tcttccgtca gctggcccgt caacgaccgg 2160
   ctcctgacgc ccaacgagtt cgaaatcaag cgcaccgtcg acggcgaggg atacaacgtg 2220
   gcccagtgca acatgaccaa ggactggttc ctggtccaga tgctggccca ctacaacatc 2280
   ggctaccagg gcttctacgt gcccgagggc tacaaggacc gcatgtactc cttcttccgc 2340
   aacttccagc ccatgagccg ccaggtggtg gacgaggtta actacaagga ctaccaggcc 2400
   gtcaccctgg cctaccaaca caacaactcg ggcttcgttg gatacctcgc gcccactatg 2460
   cgccagggcc agccctaccc cgccaactac ccctacccgc tcatcggcaa gagcgccgtt 2520
   accagcgtca cccagaaaaa gttcatctgc gacagggtca tgtggcgcat ccccttctcc 2580
   agcaacttca tgtccatggg cgcgctcacc gacctcggcc agaacatgct ctatgctaac 2640
   tccgcccacg cgctagacat gaatttcgaa gtcgacccca tggatgagcc caccctcctc 2700
   tatgttgtct tcgaagtctt cgacgtcgtc cgagtgcacc agccccaccg cggcgtcatt 2760
   gaggccgtct acctgcgcac ccccttctca gccggtaacg ccaccacata a 2811
<210> 131
   <211> 2850
   <212> DNA
   <213> Adenovirus
<220>
<400> 131
   atggccaccc catcgatgct gccccagtgg gcatacatgc acatcgccgg acaggatgct 60
   tcggagtacc tgagtccggg tctggtgcag ttcgcccgcg ccacagacac ctacttcaat 120
   ctggggaaca agtttaggaa ccccaccgtg gcgcccaccc atgatgtgac caccgaccgc 180
   agtcagcggc tgatgctgcg ctttgtgccc gttgaccggg aagacaatac ctacgcatac 240
   aaagttcgat acaccttggc tgtgggcgac aacagagtgc tggatatggc cagcactttc 300
   tttgacattc ggggtgtgtt ggatagaggc cctagcttca agccatactc tggcactgct 360
   tacaactcgt tggcccctaa gggcgctccc aatacatctc agtggattgc tgaaggcgta 420
   aaaaaagaag atgggggatc tgacgaagag gaagagaaaa atctcaccac ttacactttt 480
   ggaaatgccc cagtgaaagc agaaggtggt gatatcacta aagacaaagg tcttccaatt 540
   ggttcagaaa ttacagacgg cgaagccaaa ccaatttatg cagataaact ataccaacca 600
   gaacctcagg tgggagatga aacttggact gacacagatg gaacaactga gaagtatggt 660
   ggtagagctc taaagccaga aactaaaatg aaaccctgct atgggtcttt tgctaaaccc 720
   actaacgtca aaggcggaca ggcaaaacaa aaaactactg aacaaccgca aaaccagcag 780
   gttgaatatg atattgacat gaactttttt gatgaagcgt cacagaaagc aaacttcagt 840
   ccaaaaattg tgatgtatgc agaaaatgta gacttggaaa ccccagacac tcatgtggtg 900
   tacaaacctg gtacttcaga agaaagttct catgctaatc tgggtcaaca atctatgccc 960
   aacagaccca actacattgg ctttagagat aactttattg gacttatgta ctacaacagt 1020
   actggcaaca tgggagtgct ggcaggtcaa gcatcccaat tgaatgcggt ggttgacttg 1080
   caggacagaa acacagaact atcatatcaa ctactgcttg actctctggg tgacagaacc 1140
   agatacttca gcatgtggaa tcaagcagtc gatagctatg atcctgatgt gcgcattatt 1200
   gaaaatcatg gggtggaaga tgagcttccc aactactgct ttccattgga tggagtaggg 1260
   gtaccaataa gtagttacaa aataattgaa ccaaacggac agggtgcaga ttggaaagag 1320
   cctgacataa atggaacaag tgaaattgga caaggaaatc tctttgccat ggaaattaac 1380
   ctccaagcta atctctggag aagttttctt tattccaatg tggctctgta tctcccagac 1440
   tcctacaaat acaccccagc caatgtcact cttccaacta acaccaacac ttatgactac 1500
   atgaatgggc gggtggttcc cccatccctg gtggatacct acgtaaacat tggcgccaga 1560
   tggtctttgg atgccatgga caatgtcaac ccctttaacc atcaccgcaa cgctggcctg 1620
   cgataccggt ccatgctttt gggcaatggt cgttacgtgc ctttccacat tcaagtgcct 1680
   cagaaattct ttgctgtgaa gaacctgctg cttctacccg gttcttacac ctacgagtgg 1740
   aacttcagaa aggatgtgaa catggtcctg cagagttccc ttggtaatga tctccgggtc 1800
   gatggtgcca gcataagttt taccagcatc aatctctatg ccaccttctt ccccatggcc 1860
   cacaacactg cctccaccct tgaagccatg ctgcgcaatg acaccaatga tcaatcattc 1920
   aatgactacc tttctgctgc caacatgctc taccccatcc cggccaacgc taccaacgtt 1980
   cccatctcca ttccctctcg caactgggcc gccttcagag gctggtcctt caccagactc 2040
   aaaaccaagg agactccctc tttgggatca gggttcgatc cctactttgt ttactctggt 2100
   tctataccct acctggatgg taccttctac cttaaccaca ctttcaagaa agtctccatc 2160
   atgtttgact cttcagtgag ctggcctggt aatgacagat tgctaagtcc aaatgagttc 2220
   gaaatcaagc gcacagttga tggggaaggc tacaatgtgg cccaatgtaa catgaccaaa 2280
   gactggttcc tggtccagat gcttgccaac tacaacattg gataccaggg cttctacgtt 2340
   cctgagggtt acaaggatcg catgtactcc ttcttcagaa acttccagcc catgagtaga 2400
   caggtggttg atgagattaa ctacaaagac tataaagctg tcgccgtacc ctaccagcat 2460
   aataactctg gctttgtggg ttacatggct cctaccatgc gtcagggtca agcgtaccct 2520
   gctaactacc cataccccct aattggaacc actgcagtaa ccagtgtcac ccagaaaaaa 2580
   ttcctgtgcg acaggaccat gtggcgcatc ccattctcta gcaacttcat gtccatgggt 2640
   gcccttacag acctgggaca gaacttgctg tatgccaact cggcccatgc gctggacatg 2700
   acttttgagg tggatcccat ggatgagccc accctgcttt atcttctttt cgaagtcttc 2760
   gacgtggtca gagtgcacca gccacaccgc ggcgtcatcg aggccgtcta cctgcgcaca 2820
   ccgttctccg ccggcaacgc caccacataa 2850
<210> 132
   <211> 2907
   <212> DNA
   <213> Adenovirus
<220>
<400> 132
   atggctaccc cttcgatgat gccgcagtgg tcttacatgc acatctcggg ccaggacgcc 60
   tcggagtacc tgagccccgg gctggtgcag tttgcccgcg ccaccgagac gtacttcagc 120
   ctgaataaca agtttagaaa ccccacggtg gcacctacgc acgacgtaac cacagaccgg 180
   tcccagcgtt tgacgctgcg gttcatccct gtggaccgcg aggataccgc gtactcgtac 240
   aaagcgcggt tcaccctggc tgtgggtgac aaccgtgtgc ttgatatggc ttccacgtac 300
   tttgacatcc gcggcgtgct ggacaggggg cctactttta agccctactc cggcactgcc 360
   tacaacgctc tagctcccaa gggcgctcct aactcctgtg agtgggaaca aaccgaagat 420
   agcggccggg cagttgccga ggatgaagaa gaggaagatg aagatgaaga agaggaagaa 480
   gaagagcaaa acgctcgaga tcaggctact aagaaaacac atgtctatgc ccaggctcct 540
   ttgtctggag aaacaattac aaaaagcggg ctacaaatag gatcagacaa tgcagaaaca 600
   caagctaaac ctgtatacgc agatccttcc tatcaaccag aacctcaaat tggcgaatct 660
   cagtggaacg aagctgatgc taatgcggca ggagggagag tgcttaaaaa aacaactccc 720
   atgaaaccat gctatggatc ttatgccagg cctacaaatc cttttggtgg tcaatccgtt 780
   ctggttccgg atgaaaaagg ggtgcctctt ccaaaggttg acttgcaatt cttctcaaat 840
   actacctctt tgaacgaccg gcaaggcaat gctactaaac caaaagtggt tttgtacagt 900
   gaagatgtaa atatggaaac cccagacaca catctgtctt acaaacctgg aaaaggtgat 960
   gaaaattcta aagctatgtt gggtcaacaa tctatgccaa acagacccaa ttacattgct 1020
   ttcagggaca attttattgg cctaatgtat tataacagca ctggcaacat gggtgttctt 1080
   gctggtcagg catcgcagct aaatgccgtg gtagatttgc aagacagaaa cacagagctg 1140
   tcctatcaac tcttgcttga ttccataggt gatagaacca gatatttttc tatgtggaat 1200
   caggctgtag acagctatga tccagatgtt agaatcattg aaaaccatgg aactgaggat 1260
   gaattgccaa attattgttt tcctcttggg ggtattgggg taactgacac ctatcaagct 1320
   attaaggcta atggcaatgg ctcaggcgat aatggagata ctacatggac aaaagatgaa 1380
   acttttgcaa cacgtaatga aataggagtg ggtaacaact ttgccatgga aattaaccta 1440
   aatgccaacc tatggagaaa tttcctttac tccaatattg cgttgtacct gccagacaag 1500
   ctaaaataca accccaccaa tgtggaaata tctgacaacc ccaacaccta cgactacatg 1560
   aacaagcgag tggtggctcc cgggcttgta gactgctaca ttaaccttgg ggcgcgctgg 1620
   tctctggact acatggacaa cgttaatccc tttaaccacc accgcaatgc gggcctccgt 1680
   tatcgctcca tgttgttggg aaacggccgc tacgtgccct ttcacattca ggtgccccaa 1740
   aagttttttg ccattaaaaa cctcctcctc ctgccaggct catatacata tgaatggaac 1800
   ttcaggaagg atgttaacat ggttctgcag agctctctgg gaaacgatct tagagttgac 1860
   ggggctagca ttaagtttga cagcatttgt ctttacgcca ccttcttccc catggcccac 1920
   aacacggcct ccacgctgga agccatgctc agaaatgaca ccaacgacca gtcctttaat 1980
   gactaccttt ccgccgccaa catgctatac cccatacccg ccaacgccac caacgtgccc 2040
   atctccatcc catcgcgcaa ctgggcagca tttcgcggtt gggccttcac acgcttgaag 2100
   acaaaggaaa ccccttccct gggatcaggc tacgaccctt actacaccta ctctggctcc 2160
   ataccatacc ttgacggaac cttctatctt aatcacacct ttaagaaggt ggccattacc 2220
   tttgactctt ctgttagctg gccgggcaac gaccgcctgc ttactcccaa tgagtttgag 2280
   attaaacgct cagttgacgg ggagggctac aacgtagctc agtgcaacat gaccaaggac 2340
   tggttcctgg tgcagatgtt ggccaactac aatattggct accagggctt ctacattcca 2400
   gaaagctaca aggaccgcat gtactcgttc ttcagaaact tccagcccat gagccggcaa 2460
   gtggttgacg atactaaata caaggagtat cagcaggttg gaattcttca ccagcataac 2520
   aactcaggat tcgtaggcta cctcgctccc accatgcgcg agggacaggc ttaccccgcc 2580
   aacgtgccct acccactaat aggcaaaacc gcggttgaca gtattaccca gaaaaagttt 2640
   ctttgcgatc gcaccctttg gcgcatccca ttctccagta actttatgtc catgggcgca 2700
   ctcacagacc tgggccaaaa ccttctctac gccaactccg cccacgcgct agacatgact 2760
   tttgaggtgg atcccatgga cgagcccacc cttctttatg ttttgtttga agtctttgac 2820
   gtggtccgtg tgcaccagcc gcaccgcggc gtcatcgaga ccgtgtacct gcgcacgccc 2880
   ttctcggccg gcaacgccac aacataa                                     2907
<210> 133
   <211> 2757
   <212> DNA
   <213> Adenovirus
<220>
<400> 133
   atggccactc cctcgatgat gccgcagtgg tcttacatgc acatcgccgg tcaggatgcc 60
   tcggagtacc tgagtcccgg tctggtgcaa ttcgcccgcg ccacggacac ctacttcacc 120
   ctgggaaaca agtttagaaa ccccaccgtg gctcccaccc atgatgttac caccgatcgc 180
   tcgcagcgtc tgacgctgcg ttttgtgccc gtggatcggg aagatactac ctactcctac 240
   aaggctcgct ttacgctggc tgtgggtgac aaccgcgtgt tagacatggc tagttcttac 300
   tttgacattc gaggggtact ggatcgtggt cccagtttta agccctattc cggaaccgcc 360
   tacaattctt tggcaccaaa aggcgctcct aatgcttcac aatggtcaga taacgctaag 420
   cttaatacct ttgctcaggc gccgtatctt agcgacacta tcaccgccgc cgatggtatt 480
   aaagttggaa cagacaccgc ccaggcaggc gcggcggtgt atgccaacaa aacttatcag 540
   ccagagccgc aagtaggacc aagtgaatgg aacaccagca ttgaaaacgt taaagctggc 600
   gggagggcat taaagcaaac cactgcaatg cagccgtgct atggctccta cgctcgtcca 660
   accaacgaac acggaggaca atccaaggat gacaacattg aacttaagtt ctttgattca 720
   gctaacaatg cagcaaacac tgctcaagtt gtgttctata ccgaagacgt aaaccttgaa 780
   atgccagaca cgcatcttgt gtttaagcct actgttacca atggaacaat tgcttctgag 840
   tcgctgttgg gacagcaagc agcgccaaat agagcaaact acattgcatt cagagataat 900
   tttattggcc tgatgtatta caacagtaca ggcaacatgg gtgtattggc cgggcaagct 960
   tcccaactta acgcagtagt agacctgcaa gacagaaata cagagctgtc ataccagtta 1020
   atgctggatg ctttgggaga cagaacacgg tacttttcct tgtggaattc cgcagtggac 1080
   agttacgacc ctgacgttcg cgttattgag aatcacgggg tagaggatga actaccaaat 1140
   tattgctttc ctcttagcgc agtaggtgaa ataaaaaatt acaaaggcat taagccagat 1200
   aacggaggag gaggtggctg gactgccgac aacactgtca gtgaagcaaa ccacataggc 1260
   attgggaata tagccgccat ggaaattaat ttgcaggcta atttgtggag aagcttcttg 1320
   tactcaaatg tgggcttata cctaccagac gacttaaaat acactccagg aaacataaaa 1380
   ctacctgata acaagaacac ctacgagtac atgaacgggc gtgtgactgc cccggggttg 1440
   gtggatacct atgtcaatat cggcgctcgc tggtccccag atgtgatgga taatgtaaac 1500
   ccttttaacc accaccgaaa cgcagggttg cgctacagat ccatgttgct aggcaatggg 1560
   agatttgttc cttttcacat tcaggtgccg caaaaatttt ttgccatcag aaatttgttg 1620
   ctgttgcccg gttcctacac ttacgaatgg aactttagaa aggatgtaaa catgattctt 1680
   cagagcacac tgggaaatga tcttcgggtg gacggagcca gcgttcgctt tgacaacatt 1740
   gccctgtatg ctaacttttt tcccatggca cataacacag cttctacttt agaagccatg 1800
   ttaagaaatg acaccaacga ccagtctttt aacgattatt tgtgtgctgc aaacatgctg 1860
   tatcccatcc cagctaacgc caccagcgtg cccatttcaa taccttcgcg aaattgggcg 1920
   gcatttagag gctggagctt tactcgccta aaaactaaag aaactccttc cctgggttca 1980
   gggtttgacc cctactttgt atactctgga accattccct atttagacgg caccttttac 2040
   ctaaaccaca cttttaagaa ggtgtcaatc atgtttgact cctccgtgag ttggcctgga 2100
   aatgaccgtt tgctaacccc aaatgaattt gaaataaagc gttctgtgga tggggaggga 2160
   tacaatgtgg cccaatgcaa tatgactaag gattggttcc taatacaaat gcttagtcat 2220
   tacaacattg gataccaagg tttttacatt ccagagagct acaaggaccg catgtattct 2280
   ttctttagaa actttcagcc catgagtagg caagttgtgg ataccacaga atataagaac 2340
   tacaaaaaag taaccgtaga gtttcaacat aacaactcag gattcgtggg atacctgggc 2400
   cccactatgc gggagggaca agcttacccc gccaactatc cctaccctct tataggccaa 2460
   acagctgtgg aaagcatcac acagaaaaag tttctatgcg atcgtgttat gtggcgcatc 2520
   ccattttcta gtaacttcat gtctatgggg gcgctaacgg atcttgggca aaatatgctg 2580
   tacgcaaact cagcccatgc tctagacatg acatttgagg tggatccaat ggatgagcct 2640
   acccttcttt atgttttatt tgaagttttc gacgtggtac gcattcacca gccacaccgc 2700
   ggcgtcattg aagcggtcta cctgcgcacg cccttctcgg cgggtaacgc taccacc    2757
<210> 134
   <211> 2895
   <212> DNA
   <213> Adenovirus
<220>
<400> 134
   atggctaccc cttcgatgat gccgcagtgg tcttacatgc acatctcggg ccaggacgcc 60
   tcggagtacc tgagccccgg gctggtgcag ttcgcccgcg ccaccgagac gtacttcagc 120
   ctgaataaca agtttagaaa ccccacggtg gcgcctacgc acgacgtgac cacagaccgg 180
   tctcagcgtt tgacgctgcg gtttatcccc gtggaccgcg aggataccgc atactcgtac 240
   aaggcgcggt ttaccctggc tgtgggtgac aaccgtgtgc ttgacatggc ttccacatac 300
   tttgacattc gcggcgtgct ggaccggggc cccactttta agccctactc cggcactgcc 360
   tacaacgctc tagcccccaa aggcgctccc aattcctgcg agtgggaaca agaagaacca 420
   actcaggaaa tggctgaaga acttgaagat gaggaggagg cagaggagga ggaggcagag 480
   gaggaggcag aagcaccaca agctgatcag aaggttaaga agactcatgt atatgctcag 540
   gctcctttgg caggggaaaa aattaccgcc aatggcttac aaatagtttc tgatacccaa 600
   actgaaggca atccagtttt tgccgatccc acttatcaac ctgaacctca ggttggagaa 660
   tctcagtgga atgaagctga agcaactgca tctggaggca gagtactaaa aaagactact 720
   cccatgaaac catgctacgg atcgtatgcc agacctacaa ataaaaatgg gggtcaaggt 780
   atactagtag caaacaacca aggtgctcta gagtctaaag ttgaaatgca gttttttgcc 840
   ccctctggca ctgccatgaa tgaaagaaat gctgttcagc caagtattgt tttgtacagt 900
   gaggatgtta atatggaaac tcctgatact cacatttcat acaaaccaag caaaactgat 960
   gaaaactcta aggctatgtt gggtcaacaa gcaatgccaa acagacccaa ttacatcgct 1020
   tttagggaca attttattgg ccttatgtat tacaacagca ctggtaacat gggtgtcctt 1080
   gctggacaag catcacagct aaatgccgtg gtagacttgc aggacagaaa cacagagctg 1140
   tcatatcaac ttttgcttga ttctattggc gatagaacca gatacttttc catgtggaat 1200
   caggctgtag acagctatga tccagatgtc agaatcattg aaaatcatgg aactgaggat 1260
   gagttgccaa attactgctt cccacttggc ggtatagggg taactgacac ctaccaaggt 1320
   ataaaatcaa acggaaacgg taatcctcaa aactggacca aaaatgacga ttttgcggca 1380
   cgtaatgaaa taggtgtggg aaacaacttt gccctggaga ttaaccttaa tgccaaccta 1440
   tggagaaatt tcctctactc caacattgca ctgtacctgc ctgacaagct aaaatacact 1500
   cctacaaatg tggaaatatc tcccaaccct aattcatacg attatatgaa caagcgagtg 1560
   gtggctcccg ggttggtgga ttgctacatt aaccttggag cgcgttggtc attggactac 1620
   atggacaacg tcaacccctt taaccatcac cgcaatgcgg gcctacgcta ccgctccatg 1680
   ttgctgggca acggtcgcta cgtgcccttt cacatccagg ttcctcagaa gttttttgcc 1740
   attaagaacc tcctactctt gccgggctca tacacctacg agtggaactt caggaaagat 1800
   gttaacatgg tcctgcaaag ctccctagga aacgacctaa gagttgacgg agccagcatt 1860
   aagtttgaca gcatttgcct ctacgccacc ttttttccga tggcccacaa caccgcctca 1920
   acgcttgaag ccatgcttag aaacgacacc aacgaccagt cctttaacga ctacctatcc 1980
   gccgccaaca tgctttaccc catacccgcc aacgccacca acgtgcccat ctctatcccc 2040
   tcgcgcaact gggcggcttt ccgaggctgg gcctttacgc gccttaagac taaggaaacc 2100
   ccatccctgg gttccggcta cgacccttac tatacctact ctggctccat accctaccta 2160
   gacggaacct tttaccttaa tcacaccttc aaaaaggtag ccatcacctt tgactcttct 2220
   gttagctggc ctggcaatga ccgtctgctt acccccaacg agtttgagat caagcgttca 2280
   gttgacggag agggctacaa cgttgcccaa tgcaacatga ccaaagactg gttcttggta 2340
   cagatgctag ccaactacaa cataggctac cagggctttt atatcccaga aagctataag 2400
   gaccgcatgt actccttctt tagaaacttc cagcccatga gccgtcaggt ggtggacgat 2460
   accaaataca aggactacca acaggtgggc atcctccacc agcacaataa ctctggcttt 2520
   gttggttacc tcgctcccac catgcgagag ggacaggcct accccgccaa cttcccctac 2580
   ccgcttatag gcaagaccgc ggttgacagt attacccaga aaaagtttct ttgcgaccgc 2640
   accctttggc gcattccatt ctccagtaac tttatgtcca tgggtgcact cacagacctg 2700
   ggccaaaacc ttctctatgc aaactccgcc cacgcgctag atatgacttt tgaggtggat 2760
   cccatggacg agcccaccct tctttatgtt ttgtttgaag tctttgacgt ggtccgtgtg 2820
   caccagccgc accgcggcgt catcgagacc gtgtacctgc gcacgccctt ctcggctggc 2880
   aacgccacaa cataa 2895
<210> 135
   <211> 1110
   <212> DNA
   <213> Adenovirus
<220>
<400> 135
   ttttgacatc cgcggcgtcc tggaccgcgg tcccagcttc aaaccctact cgggcacggc 60
   ttacaacagc ctggctccca agggcgcccc caatcccagt cagtgggaac aggcaaaaac 120
   aggagctggt gtagaccaaa atcaaaaaga aactcgcact tacggcgtag ccgccactgg 180
   cggatataac attacaaaag aaggacttca gattggaatt gatgaaacta aagaagatcc 240
   aaacaacaaa atttatgcag ataaaacatt ccagccggaa ccacaaatag gggaaaataa 300
   ttggcaggac actaatgttt tctatggcgg cagagctctt aaaaaggaaa ccaagatgaa 360
   accatgctat ggctcatttg ccagaccaac taataaaaaa ggagggcagg ctaaagtttt 420
   aaccactgaa gatggtcaac ctacagaaaa ctttgacata gacttggctt tctttgatat 480
   tccacaagct ggtggaaatg acaatcttga tccagatatg attctgtatg ccgaaaatgt 540
   aaatctagaa acacctgaca cacatgtagt ttataaacca ggcaaggatg atgcaagttc 600
   tgccgctaac ctcacacaac agtccatgcc aaacagaccc aactacatcg gtttcaggga 660
   caactttgtg ggacttatgt attacaacag cactggcaac atgggtgtgc tggctggtca 720
   ggcctctcag ttaaatgctg tggtcgactt gcaagacaga aataccgagc tgtcttatca 780
   gcttttatta gattctctgg gtgacaggac cagatacttt agtatgtgga actctgcggt 840
   ggacagctat gatccagatg tcaggatcat tgagaatcac ggtgtggagg acgaacttcc 900
   aaattattgc ttcccattgg atggtaccgg taccaatgcc acataccaag gtgtggaacc 960
   agataatgct caaggtcaaa atgataaatg gaaaaaggac gagaaagtgg ctgctcaaaa 1020
   ccaaatttgc aagggcaata tttatgccat ggagattaac ctccaggcca acctgtggaa 1080
   gagttttctg tactcgaacg tggccttgga 1110
<210> 136
   <211> 2769
   <212> DNA
   <213> Adenovirus
<220>
<400> 136
   atggccaccc cctcgatgat gccgcaatgg tcttacatgc acatcgccgg gcaggacgcc 60
   tcggagtacc tgagcccggg cctggtgcag ttcgcccgtg ccaccgatac ctacttcagc 120
   ctggggaaca agttcagaaa ccccaccgtg gctcccaccc acgatgtaac cacagacagg 180
   tcgcagcgac tgacgctgcg cttcgtgccc gtcgaccgcg aggaaaccgc ctactcttac 240
   aaagtgcgct ttacgctggc cgtgggcgac aaccgggttt tggacatggc cagcacctac 300
   tttgacatcc gcggcgtgct ggatcgtggt cccagcttta aaccctattc gggcactgca 360
   tacaactccc tggcccccaa aggtgctccc aatcctagcc agtggacaaa ccaaaacaaa 420
   acaaactcct ttggacaagc tccctatata ggacaaaaaa tcaccaatca gggcgtgcaa 480
   gtgggctcag actccaacaa tcgcgatgtg tttgccgata aaacgtacca accggagcct 540
   caagtggggc agacgcaatg gaacattaat ccaatgcaaa acgctgcggg aagaatacta 600
   aaacaaacca cgcccatgca gccatgttat gggtcatacg ctagaccaac aaacgaaaaa 660
   ggaggtcaag ccaagctggt aaaaaatgac gacaatcaga ccacaacaac aaacgtaggt 720
   ttaaactttt ttaccactgc cactgaaacc gctaattttt caccaaaggt ggttctgtac 780
   agcgaagatg ttaacttaga agcgcccgat acccaccttg tgtttaagcc agatgtcaac 840
   ggcacaagtg ccgagctttt actgggacag caggccgctc ccaatcgacc taattacatt 900
   ggttttaggg acaacttcat tggtttgatg tactacaatt ccactggcaa catgggagtg 960
   ctggccgggc aagcttctca gctcaacgca gtggtggact tacaagatag aaacacggag 1020
   ctgtcttacc agttaatgct tgacgcttta ggggatcgga gtcgatactt ctccatgtgg 1080
   aaccaggcag tggacagcta tgacccagac gtgagaatta ttgaaaatca tggcgtggaa 1140
   gacgagctcc ccaactattg ctttcctctt aatgggcaag gaatatctaa cagttaccaa 1200
   ggcgtaaaga ctgacaatgg aactaactgg tctcagaata atacagacgt ctcaagcaac 1260
   aacgaaattt ccattggcaa tgtgtttgcc atggagatta atctggcggc taacttgtgg 1320
   agaagcttct tgtactcaaa tgtagccctg tacttgcctg actcttacaa aataaccccc 1380
   gataacatta ctttacccga caacaaaaat acatatgcct acatgaacgg tcgggttgcc 1440
   gtccccagcg ccctggatac atacgtgaac attggggcgc ggtggtctcc agaccccatg 1500
   gacaacgtta atccctttaa ccaccaccgc aatgctggtc tgcgctaccg ttctatgctc 1560
   ctgggtaacg gccgctacgt gccttttcac atccaagtgc cccagaaatt tttcgccatt 1620
   aaaaatctcc tgctcctgcc cgggtcctac acctatgagt ggaacttccg gaaggatgtt 1680
   aacatgattc tccaaagcag tctcggtaac gacctcaggg tcgatggagc cagcgtcagg 1740
   tttgacagca ttaacctgta tgccaacttt ttccccatgg ctcacaacac cgcttccacc 1800
   ttggaagcaa tgcttcgtaa tgataccaac gatcagtctt tcaacgacta cctctgcgct 1860
   gcaaacatgc tttaccccat acccgccaac gctactagcg tgcccatttc tattccttcg 1920
   cgaaattggg ctgcttttcg ggggtggagt tttactagac taaaaactaa agaaaccccc 1980
   tctttggggt ccgggtttga tccatatttc acctactctg gctccgtccc atacttggat 2040
   ggcacctttt acctgaacca cacttttaaa aaggtgtccg ttatgttcga ctcctctgtg 2100
   agctggcctg gtaacgaccg actacttact cccaacgagt ttgaaatcaa acgaaccgtg 2160
   gatggggaag gatacaacgt ggctcaatgt aacatgacca aggactggtt cctcatacaa 2220
   atgctcagtc actacaatat tggctaccag ggtttccacg taccagaaag ctacaaggac 2280
   aggatgtact cctttttccg aaacttccaa cccatgagcc gccaggtggt agacactacc 2340
   acctacacgg agtatcagaa tgtaactctc cctttccagc ataataactc tggctttgta 2400
   ggatacatgg gacctgccat acgggaggga caagcttacc ccgccaacta tccatacccc 2460
   cttattggtc agacggccgt accaagcctg actcagaaaa aatttctttg cgatcgtacc 2520
   atgtggcgca ttcccttttc cagcaacttt atgtctatgg gggccctgac cgacctgggg 2580
   caaaacatgc tgtacgccaa ctccgcccac gcgctcgaca tgacttttga ggtggacccc 2640
   atggatgagc ccacacttct ctatgttctg ttcgaagttt tcgacgttgt gcgcatccac 2700
   cagccgcacc gcggcgtcat cgaggccgtc tacctgcgta cgccgttctc ggccggtaac 2760
   gccaccaca                                                        2769